# EUROPEAN PATENT SPECIFICATION

(11) **EP 4 225 435 B1**
(45) Date of publication and mention of the grant of the patent: **11.12.2024**
(21) Application number: 21802105.3
(22) Date of filing: 08.10.2021
(51) Int. Cl.: A61P 9/00, A61P 9/04, A61P 9/10, C07D 233/88, C07D 401/04, C07D 401/06, C07D 401/12, C07D 403/04, C07D 403/06, C07D 403/12, C07D 405/04, C07D 409/04, C07D 413/04, C07D 413/06, C07D 417/04, C07D 471/04, A61K 31/4168, A61K 31/4178

(54) **AMINOIMIDAZOLE FPR2 AGONISTS**
AMINOIMIDAZOL-FPR2-AGONISTEN
AGONISTES DU FPR2 AMINOIMIDAZOLE

(30) Priority: 09.10.2020 US 202063089730 P
(43) Date of publication of application: 16.08.2023
(73) Proprietor: Bristol-Myers Squibb Company, Princeton, NJ 08543 (US)
(72) Inventor: PEREZ, Heidi L., Princeton, New Jersey 08543 (US); JOHNSON, James A., Pennington, New Jersey 08534 (US); DEWNANI, Sunita V., Princeton, New Jersey 08543 (US)
(74) Representative: Dehns
(86) International application number: PCT/US2021/054077
(87) International publication number: WO 2022/076764

(56) References cited:
- WO-A1-2009/099177
- WO-A1-2018/217684
- WO-A1-2020/112583
- ASAHINA, Y. ET AL.: "Discovery of BMS-986235/LAR-1219: A Potent Formyl Peptide Receptor 2 (FPR2) Selective Agonist for the Prevention of Heart Failure", JOURNAL OF MEDICINAL CHEMISTRY, vol. 63, no. 17, 14 May 2020 (2020-05-14), pages 9003 - 9019, XP055849143, ISSN: 0022-2623, DOI: 10.1021/acs.jmedchem.9b02101

## Description

### BACKGROUND OF THE INVENTION

The present invention relates to novel aminoimidazole compounds of Formula (I) which are formyl peptide 2 (FPR2) receptor agonists, and also relates to compositions containing them. The compounds may be used, for example, for the treatment of atherosclerosis, heart failure, chronic obstructive pulmonary disease (COPD), and related diseases.

Formyl peptide receptor 2 (FPR2) belongs to a small group of seven-transmembrane domain, G protein-coupled receptors that are expressed in multiple human tissues including immune cells and are known to be important in host defense and inflammation. FPR2 shares significant sequence homology with FPR1 and FPR3 (Chen K, et. al., Journal of Autoimmunity 85, 2017, 64-77). Collectively, these receptors bind a number of structurally diverse agonists, including N-formyl and non-formyl peptides which act as chemo attractants and activate phagocytes. The endogenous peptide Annexin A1 and its N-terminal fragments are examples of ligands that bind human FPR1 and FPR2. Fatty acids such as the eicosanoid lipoxin A4, which belongs to a class of small pro-resolution mediators (SPMs), has also been reported as an agonist for FPR2 (Ye RD., et al., Pharmacol. Rev., 2009, 61, 119-61).

Endogenous FPR2 pro-resolution ligands, such as lipoxin A₄ and Annexin A1, have been reported to trigger a wide array of cytoplasmatic cascades such as Gi coupling, Ca²⁺ mobilization and β-arrestin recruitment. (Cattaneo, F, et. al., Int J Mol Sci. 2013 April; 14(4): 7193-7230). FPR2 regulates both innate and adaptive immune systems including neutrophils, macrophages, T-, and B-cells. In neutrophils, FPR2 ligands modulate movement, cytotoxicity and life span. In macrophages, agonism of FPR2 prevents apoptosis and enhances efferocytosis. (Chandrasekharan JA, Sharma-Walia N,. J. Inflamm. Res., 2015, 8, 181-92). The initiation of resolution of inflammation by FPR2 agonism is responsible for enhancing anti-fibrotic wound healing and returning of the injured tissue to homeostasis (Romano M., et al., Eur. J. Pharmacol., 2015, 5, 49-63).

Chronic inflammation is part of the pathway of pathogenesis of many human diseases and stimulation of resolution pathways with FPR2 agonists may have both protective and reparative effects. Ischemia-reperfusion (I/R) injury is a common feature of several diseases associated with high morbidity and mortality, such as myocardial infarction and stroke. Non-productive wound healing associated with cardiomyocyte death and pathological remodeling resulting from ischemia-reperfusion injury leads to scar formation, fibrosis, and progressive loss of heart function. FPR2 modulation is proposed to enhance myocardial wound healing post injury and diminish adverse myocardial remodeling (Kain V., et al., J. Mol. Cell. Cardiol., 2015, 84, 24-35). In addition, FPR2 pro-resolution agonists, in the central nervous system, may be useful therapeutics for the treatment of a variety of clinical I/R conditions, including stroke in brain (Gavins FN., Trends Pharmacol. Sci., 2010, 31, 266-76) and I/R induced spinal cord injury (Liu ZQ ., et al., Int. J. Clin. Exp. Med., 2015, 8, 12826-33).

In addition to beneficial effects of targeting the FPR2 receptor with novel pro-resolution agonists for treatment of I/R induced injury, utility of these ligands can also be applied to other diseases. In the cardiovascular system both the FPR2 receptor and its pro-resolution agonists were found to be responsible for atherogenic-plaque stabilization and healing (Petri MH., et al., Cardiovasc. Res., 2015, 105, 65-74; and Fredman G., et al., Sci. Trans. Med., 2015, 7(275);275ra20). FPR2 agonists also have been shown to be beneficial in preclinical models of chronic inflammatory human diseases, including: infectious diseases, psoriasis, dermatitis, inflammatory bowel syndrome, Crohn's disease, ocular inflammation, sepsis, pain, metabolic/diabetes diseases, cancer, COPD, asthma and allergic diseases, cystic fibrosis, acute lung injury and fibrosis, rheumatoid arthritis and other joint diseases, Alzheimer's disease, kidney fibrosis, and organ transplantation (Romano M., et al., Eur. J. Pharmacol., 2015, 5, 49-63, Perrett, M., et al., Trends in Pharm. Sci., 2015, 36, 737-755).

Asahina Y., et al., J. Med. Chem., 2020, 63, 9003-9019, discloses the discovery of BMS-986235/LAR-1219, a potent FPR2 selective agonist for the prevention of heart failure. WO 2018/217684 discloses the use of a formyl peptide receptor 2/lipoxin A4 receptor (FPR2/ALX) agonist for the treatment of heart failure. WO 2020/112583 discloses compounds which are FPR2 receptor agonists and/or formyl peptide I (FPRI) receptor agonists, as well as compositions and methods of using the compounds, for example, for the treatment of atherosclerosis, heart failure, and related diseases.

### DESCRIPTION OF THE INVENTION

The invention encompasses compounds of Formula (I), which are formyl peptide 2 (FPR2) receptor agonists, compositions containing them, and the compounds or compositions for use in the treatment of atherosclerosis, heart failure, chronic obstructive pulmonary disease (COPD), and related diseases, for example.

One aspect of the invention is a compound of Formula (I): 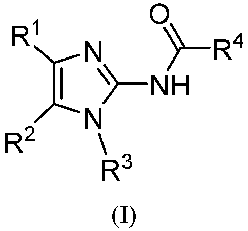 wherein
R¹ is alkyl, haloalkyl, hydroxyalkyl, alkoxyalkyl, (alkoxycarbonyl)alkyl, alkoxycarbonyl, (NR⁶R⁷)carbonyl, Ar¹, or (Ar¹)alkyl;
Ar¹ is cycloalkyl, aryl, heteroaryl comprising carbon atoms and 1-5 heteroatoms selected from N, NR^{5a}, O, and S, heterocyclyl comprising carbon atoms and 1-5 heteroatoms selected from N, NR^{5a}, O, and S, or spiroheterocyclyl comprising carbon atoms and 1-5 heteroatoms selected from N, NR^{5a}, O, and S, each substituted with 1-5 R⁵;
R² is hydrogen, alkyl, or haloalkyl;
R³ is phenyl or pyridinyl substituted with 1 R^{3a} and 1-2 R^{3b};
R^{3a} is halo, haloalkyl, alkoxy, or haloalkoxy;
R^{3b} is hydrogen, halo, or haloalkyl;
R⁴ is phenyl or pyridinyl substituted with 1-2 R^{4a};
R^{4a} is halo, alkoxy, or haloalkoxy;
R⁵ is hydrogen, hydroxyl, cyano, halo, alkyl, haloalkyl, amino, haloalkylamino, alkoxyalkyl, hydroxyalkyl, alkoxy, haloalkoxy, carboxamide, alkoxycarbonyl, alkylsulfonylamino, or hydroxyalkylcarbonyl;
R^{5a} is hydrogen, alkyl, haloalkyl, alkoxyalkyl, hydroxyalkyl, hydroalkylcarbonyl, carboxamide, alkylaminocarbonyl, aminocarbonylalkylcarbonyl, alkylsulfonyl, or alkoxycarbonyl;
R⁶ and R⁷ are independently hydrogen, alkyl, haloalkyl, hydroxyalkyl, cycloalkyl, aryl, heteroaryl comprising carbon atoms and 1-4 heteroatoms selected from N, NR^{8a}, O, and S, heterocyclyl comprising carbon atoms and 1-4 heteroatoms selected from N, NR^{8a}, O, and S, arylalkyl, or heteroarylalkyl comprising carbon atoms and 1-4 heteroatoms selected from N, NR^{8a}, O, and S; wherein said cycloalkyl, aryl, heteroaryl, or heterocyclyl is substituted with 1-5 R⁸;
or R⁶ and R⁷, together with the nitrogen to which they are attached, form a heterocyclyl or heteroaryl comprising carbon atoms and 0-3 additional heteroatoms selected from N, NR^{8a}, O, S, wherein said heteroaryl or heterocyclyl is substituted with 1-5 R⁸;
R⁸ is hydrogen, halo, hydroxy, hydroxyalkyl, alkyl, alkoxy, or oxo;
R^{8a} is hydrogen, hydroxyalkyl, or alkyl;
or a pharmaceutically acceptable salt thereof.

Another aspect of the invention is a compound of Formula (I) wherein
R³ is phenyl substituted with 1 R^{3a} and 1-2 R^{3b};
R^{3a} is halo, haloalkyl, or alkoxy substituent in the para-position with respect to the imidazole moiety;
R^{3b} is hydrogen, halo, or haloalkyl; and
other variables are as defined in Formula (I) above.

Another aspect of the invention is a compound of Formula (I) wherein
R⁴ is phenyl substituted with 1 R^{4a} in the para-position with respect to the amide moiety; and
other variables are as defined in Formula (I) above.

Another aspect of the invention is a compound of Formula (II): 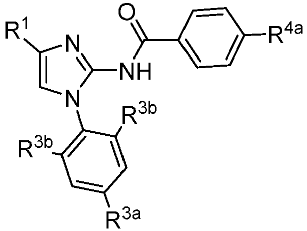 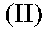 wherein
R¹ is alkyl, haloalkyl, hydroxyalkyl, alkoxyalkyl, (alkoxycarbonyl)alkyl, alkoxycarbonyl, (NR⁶R⁷)carbonyl, Ar¹, or (Ar¹)alkyl;
Ar¹ is cycloalkyl, aryl, heteroaryl comprising carbon atoms and 1-4 heteroatoms selected from N, NR^{5a}, O, and S, heterocyclyl comprising carbon atoms and 1-4 heteroatoms selected from N, NR^{5a}, O, and S, spiroheterocyclyl comprising carbon atoms and 1-4 heteroatoms selected from N, NR^{5a}, O, and S, each substituted with 1-4 R⁵;
R^{3a} is alkoxy;
R^{3b} is hydrogen, halo or haloalkyl;
R^{4a} is halo or haloalkoxy;
R⁵ is hydrogen, hydroxyl, cyano, halo, alkyl, haloalkyl, amino, haloalkylamino, alkoxyalkyl, hydroxyalkyl, alkoxy, haloalkoxy, alkoxycarbonyl, or alkylsulfonylamino;
R^{5a} is hydrogen, alkyl, haloalkyl, alkoxyalkyl, hydroxyalkyl, hydroalkylcarbonyl, carboxamide, alkylaminocarbonyl, aminocarbonylalkylcarbonyl, alkylsulfonyl, or alkoxycarbonyl;
R⁶ and R⁷ are independently hydrogen, alkyl, haloalkyl, hydroxyalkyl, cycloalkyl, heteroaryl comprising carbon atoms and 1-4 heteroatoms selected from N, NR^{8a}, O, and S, arylalkyl, or heteroarylalkyl comprising carbon atoms and 1-4 heteroatoms selected from N, NR^{8a}, O, and S, wherein said cycloalkyl, heteroaryl, or heteroarylalkyl is substituted with 1-4 R⁸;
or R⁶ and R⁷, together with the nitrogen to which they are attached, form a heterocyclyl or heteroaryl with 0-3 additional heteroatoms selected from N, NR^{8a}, O, and S, wherein said heterocyclyl or heteroaryl is substituted with 1-4 R⁸;
R⁸ is hydrogen, halo, hydroxy, hydroxyalkyl, alkyl, alkoxy, or oxo;
R^{8a} is hydrogen, hydroxyalkyl, or alkyl;
or a pharmaceutically acceptable salt thereof.

Another aspect of the invention is a compound of Formula (II), wherein
R¹ is Ar¹ substituted with 1-3 R⁵;
Ar¹ is cycloalkyl, aryl, heteroaryl comprising carbon atoms and 1-3 heteroatoms selected from N, NR^{5a}, O, and S, heterocyclyl comprising carbon atoms and 1-3 heteroatoms selected from N, NR^{5a}, O, and S, spiroheterocyclyl comprising carbon atoms and 1-3 heteroatoms selected from N, NR^{5a}, O, and S, each substituted with 1-3 R⁵;
R^{3a} is alkoxy;
R^{3b} is hydrogen or halo;
R^{4a} is haloalkoxy;
R⁵ is hydrogen, hydroxyl, cyano, halo, alkyl, haloalkyl, amino, haloalkylamino, alkoxyalkyl, hydroxyalkyl, hydroalkylcarbonyl, alkoxy, haloalkoxy, alkoxycarbonyl, or alkylsulfonylamino; and
R^{5a} is hydrogen, alkyl, haloalkyl, alkoxyalkyl, hydroxyalkyl, alkylaminocarbonyl, aminocarbonylalkylcarbonyl, alkylsulfonyl, or alkoxycarbonyl.

Another aspect of the invention is a compound of Formula (II), wherein
Ar¹ is 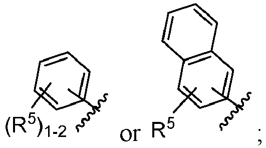
R^{3a} is alkoxy;
R^{3b} is hydrogen or halo;
R^{4a} is haloalkoxy; and
R⁵ is hydrogen, cyano, halo, alkyl, haloalkyl, alkoxyalkyl, hydroxyalkyl, alkoxy, or haloalkoxy.

Another aspect of the invention is a compound of Formula (II), wherein
Ar¹ is 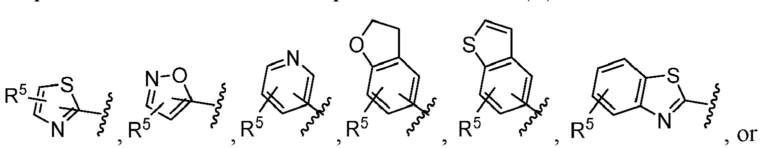 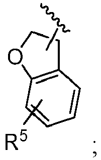
R^{3a} is alkoxy;
R^{3b} is hydrogen or halo;
R^{4a} is haloalkoxy;
R⁵ is hydrogen, halo, alkyl, haloalkyl, alkoxyalkyl, hydroxyalkyl, alkoxy, alkoxycarbonyl, or haloalkoxy.

Another aspect of the invention is a compound of Formula (II), wherein
Ar¹ is 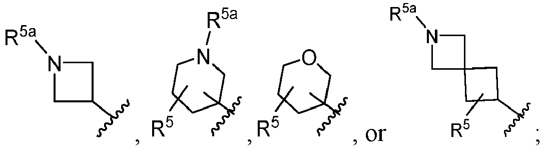
R^{3a} is alkoxy;
R^{3b} is hydrogen or halo;
R^{4a} is haloalkoxy;
R⁵ is hydrogen, alkyl, or hydroxyalkyl; and
R^{5a} is hydrogen, alkyl, hydroalkylcarbonyl, alkylaminocarbonyl, aminocarbonylalkylcarbonyl, alkylsulfonyl, or alkoxycarbonyl.

Another aspect of the invention is a compound of Formula (II), wherein
Ar¹ is 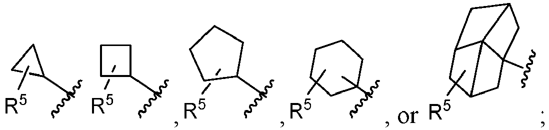
R^{3a} is alkoxy;
R^{3b} is hydrogen or halo;
R^{4a} is haloalkoxy;
R⁵ is hydrogen, hydroxyl, hydroxyalkyl, amino, haloalkylamino, or alkylsulfonylamino.

Another aspect of the invention is a compound of Formula (II), wherein
R¹ is (Ar¹)alkyl;
R^{3a} is alkoxy;
R^{3b} is hydrogen or halo; and
R^{4a} is haloalkoxy.

Another aspect of the invention is a compound of Formula (II), wherein
Ar¹ in (Ar¹)alkyl is 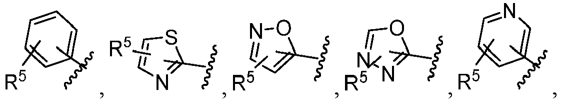 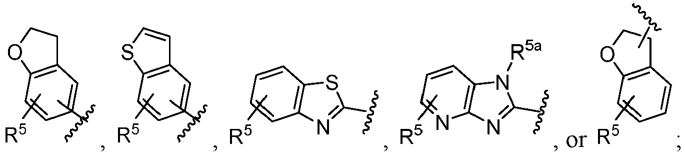
R^{3a} is alkoxy;
R^{3b} is hydrogen or halo;
R^{4a} is haloalkoxy;
R⁵ is hydrogen, cyano, halo, alkyl, haloalkyl, alkoxyalkyl, hydroxyalkyl, alkoxy, or haloalkoxy; and
R^{5a} is hydrogen or alkyl.

Another aspect of the invention is a compound of Formula (II), wherein
R¹ is alkyl or haloalkyl;
R^{3a} is alkoxy;
R^{3b} is hydrogen or halo; and
R^{4a} is haloalkoxy.

Another aspect of the invention is a compound of Formula (II), wherein
R¹ is alkoxycarbonyl or (alkoxycarbonyl)alkyl;
R^{3a} is alkoxy;
R^{3b} is hydrogen or halo; and
R^{4a} is haloalkoxy.

Another aspect of the invention is a compound of Formula (II), wherein
R¹ is (NR⁶R⁷)carbonyl;
R^{3a} is alkoxy;
R^{3b} is hydrogen or halo;
R^{4a} is haloalkoxy;
R⁶ and R⁷ are independently hydrogen, alkyl, haloalkyl, hydroxyalkyl, cycloalkyl, heteroaryl comprising carbon atoms and 1-3 heteroatoms selected from N, NR^{8a}, O, and S, or heteroarylalkyl comprising carbon atoms and 1-3 heteroatoms selected from N, NR^{8a}, O, and S, wherein said cycloalkyl, heteroaryl, or heteroarylalkyl is substituted with 1-3 R⁸;
or R⁶ and R⁷, together with the nitrogen to which they are attached, form 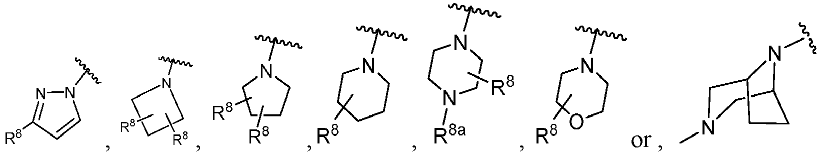
R⁸ is hydrogen, halo, hydroxy, hydroxyalkyl, alkyl, alkoxy, or oxo;
R^{8a} is hydrogen, hydroxyalkyl, or alkyl.

Another aspect of the invention is a compound of Formula (II), wherein
R¹ is (NR⁶R⁷)carbonyl;
R^{3a} is alkoxy;
R^{3b} is hydrogen or halo;
R^{4a} is haloalkoxy;
R⁶ is hydrogen;
R⁷is 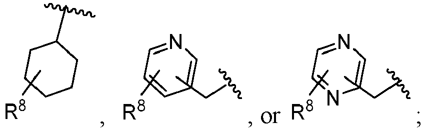
R⁸ is hydrogen, halo, hydroxy, hydroxyalkyl, alkyl, or alkoxy.

For a compound of Formula (I) or (II), the scope of any instance of a variable substituent, including R¹, R², R³, R⁴, and Ar¹, can be used independently with the scope of any other instance of a variable substituent. As such, the invention includes combinations of the different aspects.

Unless specified otherwise, these terms have the following meanings.
"Halo" refers to fluoro, chloro, bromo, and iodo.
"Hydroxyl" refers to -OH.
"Oxo" refers to =O.
"Carbonyl" refers to a group -C=O.
"Alkyl" refers to a straight or branched alkyl group composed of 1 to 7 carbons such as methyl, ethyl, n-propyl, isopropyl, butyl, isobutyl, pentyl, hexyl, heptyl, and the like.
"Haloalkyl" and "haloalkoxy" refer to halo substituted alkyl or alkoxy groups. Haloalkyl or haloalkoxy include mono-substituted as well as multiple halo substituted alkyl or alkoxy groups, up to perhalo substituted alkyl or alkoxy.
"Hydroxyalkyl" refers to an alkyl group that has at least one hydrogen atom substituted with a hydroxyl group.
"Alkoxyalkyl" refers to an alkyl group that has at least one hydrogen atom substituted with an alkoxy group described above.
"Alkylsulfonyl" refers to a group -SO₂-alkyl wherein alkyl is as herein defined.
"Alkylsulfonylamino" refers to a group -NHSO₂-alkyl wherein alkyl is as herein defined.
"Amine" refers to a group of the formula -NRR', where R and R' can be, independently, hydrogen or an alkyl, aryl, aralkyl, cycloalkyl, or haloalkyl described above.
"Cycloalkyl" refers to a non-aromatic mono- or multicyclic ring system comprising from about 3 to about 10 ring carbon atoms. Non-limiting examples of monocyclic cycloalkyls include cyclopropyl, cyclobutyl, cyclopentyl, cyclohexyl, cycloheptyl and cyclooctyl. Non-limiting examples of multicyclic cycloalkyls include 1-decalinyl, norbornyl and adamantyl.
"Aryl" refers to a monocyclic or bicyclic aromatic hydrocarbon groups having 6 to 12 carbon atoms, or a bicyclic fused ring system wherein one or both of the rings is aromatic.
"Heteroaryl" refers to a 5 to 7 membered monocyclic or 8 to 11 membered bicyclic aromatic ring system with 1-5 heteroatoms independently selected from nitrogen, oxygen, and sulfur. Where a bonding attachment location is not specified, the bonding may be attached at any appropriate location as understood by practitioners in the art.
"Heterocyclyl," "heterocycle" or "heterocyclic" refers to nonaromatic monocyclic ring structures in which one or more atoms in the ring, the heteroatom(s), is an element other than carbon. Heteroatoms are typically O, S or N atoms. Examples of heterocyclyl groups include: piperidine, piperazine, morpholine, pyrrolidine, tetrahydrofuran, azetidine, oxirane, or aziridine, and the like.
"Spiroheterocyclyl" refers to a spirocycle wherein at least one of the rings is a heterocycle (e.g., at least one of the rings is aziridinyl, azetidinyl, furanyl, morpholinyl, or piperadinyl).

Combinations of substituents and bonding patterns are only those that result in stable compounds as understood by practitioners in the art. Parenthetic and multiparenthetic terms are intended to clarify bonding relationships to those skilled in the art. For example, a term such as ((R)alkyl) means an alkyl substituent further substituted with the substituent R.

Some examples of compounds where R^{3a} is substituted in the para-position with respect to the imidazole are illustrated below.

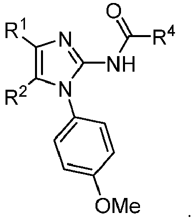

Some examples of compounds where R^{4a} is substituted in the para-position with respect to the amide moiety are illustrated below.

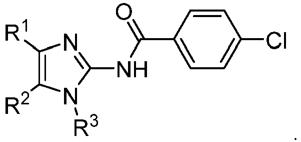

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention exist in stereoisomeric forms including the structure below with the indicated carbon. The invention includes all stereoisomeric forms of the compounds including enantiomers and diastereomers. Methods of making and separating stereoisomers are known in the art. The invention includes all tautomeric forms of the compounds. The invention includes atropisomers and rotational isomers.

The invention is intended to include all isotopes of atoms occurring in the compounds. Isotopes include those atoms having the same atomic number but different mass numbers. By way of general example and without limitation, isotopes of hydrogen include deuterium and tritium. Isotopes of carbon include ¹¹C, ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties.

### BIOLOGICAL METHODS

N-formyl peptide receptors (FPRs) are a family of chemo attractant receptors that facilitate leukocyte response during inflammation. FPRs belong to the seven-transmembrane G protein-coupled receptor superfamily and are linked to inhibitory G-proteins (Gi). Three family members (FPR1, FPR2 and FPR3) have been identified in humans and are predominantly found in myeloid cells with varied distribution and have also been reported in multiple organs and tissues. After agonist binding, the FPRs activate a multitude of physiological pathways, such as intra cellular signaling transduction, Ca²⁺ mobilization and transcription. The family interacts with a diverse set of ligands that includes proteins, polypeptides and fatty acid metabolites which activate both pro-inflammatory and pro-resolution downstream responses. FPR2 and FPR1 Cyclic Adenosine Monophosphate (cAMP) Assays were used to measure the activity of the compounds in this patent.

FPR2 and FPR1 Cyclic Adenosine Monophosphate (cAMP) Assays. A mixture of forskolin (5 µM final for FPR2 or 10 µM final for FPR1) and IBMX (200 µM final) were added to 384-well Proxiplates (Perkin-Elmer) pre-dotted with test compounds in DMSO (1% final) at final concentrations in the range of 0.020 nM to 100 µM. Chinese Hamster Ovary cells (CHO) overexpressing human FPR1 or human FPR2 receptors were cultured in F-12 (Ham's) medium supplemented with 10% qualified FBS, 250 µg/ml zeocin and 300 µg/ml hygromycin (Life Technologies). Reactions were initiated by adding 2,000 human FPR2 cells per well or 4,000 human FPR1 cells per well in Dulbecco's PBS (with calcium and magnesium) (Life Technologies) supplemented with 0.1% BSA (Perkin-Elmer). The reaction mixtures were incubated for 30 min at room temperature. The level of intracellular cAMP was determined using the HTRF HiRange cAMP assay reagent kit (Cisbio) according to manufacturer's instruction. Solutions of cryptate conjugated anti-cAMP and d2 flurorophore-labelled cAMP were made in a supplied lysis buffer separately. Upon completion of the reaction, the cells were lysed with equal volume of the d2-cAMP solution and anti-cAMP solution. After a 1-h room temperature incubation, time-resolved fluorescence intensity was measured using the Envision (Perkin-Elmer) at 400 nm excitation and dual emission at 590 nm and 665 nm. A calibration curve was constructed with an external cAMP standard at concentrations ranging from 1 µM to 0.1 pM by plotting the fluorescent intensity ratio from 665 nm emission to the intensity from the 590 nm emission against cAMP concentrations. The potency and activity of a compound to inhibit cAMP production was then determined by fitting to a 4-parametric logistic equation from a plot of cAMP level versus compound concentrations.

The examples disclosed below were tested in the FPR2 and FPR1 cAMP assay described above and found having FPR2 and/or FPR1 agonist activity. Table 1 below lists EC₅₀ values in the FPR2 and FPR1 cAMP assays measured for the following examples.

**Table 1**

| Example | hFPR2 cAMP2 EC₅₀ (µM) | hFPR1 cAMP2 EC₅₀ (µM) |
|---|---|---|
| 1 | 0.004 | 0.20 |
| 2 | 0.003 | 0.038 |
| 3 | 0.008 | 0.088 |
| 4 | 0.011 | 0.40 |
| 5 | 0.047 | 0.14 |
| 6 | 0.076 | 0.12 |
| 7 | 0.033 | 0.19 |
| 8 | 0.008 | 0.23 |
| 9 | 0.22 | 0.46 |
| 10 | 0.007 | 0.20 |
| 11 | 0.024 | 0.14 |
| 12 | 0.015 | 0.45 |
| 13 | 0.053 | 0.55 |
| 14 | 0.039 | 0.16 |
| 15 | 0.023 | 0.13 |
| 16 | 0.027 | 0.23 |
| 17 | 0.005 | 0.046 |
| 18 | 0.051 | 0.40 |
| 19 | 0.028 | 0.093 |
| 20 | 0.005 | 0.019 |
| 21 | 0.029 | 0.076 |
| 22 | 0.086 | 0.14 |
| 23 | 0.028 | 0.044 |
| 24 | 0.009 | 0.11 |
| 25 | 0.006 | 0.047 |
| 26 | 0.072 | 0.40 |
| 27 | 0.055 | 0.66 |
| 28 | 0.70 | 1.03 |
| 29 | 0.015 | 0.032 |
| 30 | 0.009 | 0.10 |
| 31 | 0.32 | >10 |
| 32 | 0.018 | 0.11 |
| 33 | 0.039 | 0.94 |
| 34 | 0.016 | 0.12 |
| 35 | 0.099 | 1.4 |
| 36 | 0.031 | 0.32 |
| 37 | 0.016 | 0.80 |
| 38 | 0.015 | 0.62 |
| 39 | 0.021 | 1.0 |
| 40 | 0.011 | 1.0 |
| 41 | 0.026 | 0.23 |
| 42 | 0.048 | 0.057 |
| 43 | 0.010 | 0.040 |
| 44 | 0.048 | 0.37 |
| 45 | 0.014 | 0.71 |
| 46 | 0.027 | 0.21 |
| 47 | 0.035 | 0.11 |
| 48 | 0.11 | 0.60 |
| 49 | 0.090 | 0.75 |
| 50 | 0.077 | >10 |
| 51 | 0.083 | 2.9 |
| 52 | 0.063 | >10 |
| 53 | 0.13 | 4.1 |
| 54 | 0.32 | >10 |
| 55 | 0.28 | >10 |
| 56 | 0.089 | 0.11 |
| 57 | 0.12 | 1.9 |
| 58 | 0.17 | 3.4 |
| 59 | 0.082 | 2.2 |
| 60 | 0.056 | 1.4 |
| 61 | 0.13 | >10 |
| 62 | 0.032 | 0.83 |
| 63 | 0.010 | 0.71 |
| 64 | 0.011 | 0.21 |
| 65 | 0.081 | 0.32 |
| 66 | 0.028 | 0.54 |
| 67 | 0.054 | 0.083 |
| 68 | 0.045 | 0.11 |
| 69 | 0.086 | 2.988 |
| 70 | 0.031 | 0.97 |
| 71 | 0.046 | 1.3 |
| 72 | 0.022 | 0.47 |
| 73 | 0.023 | 0.30 |
| 74 | 0.007 | 0.25 |
| 75 | 0.023 | >10 |
| 76 | 0.010 | 0.24 |
| 77 | 0.013 | 0.12 |
| 78 | 0.056 | 0.87 |
| 79 | 0.081 | 4.2 |
| 80 | 0.059 | 0.50 |
| 81 | 0.004 | 5.4 |
| 82 | 0.010 | 0.085 |
| 83 | 0.005 | 0.40 |

### PHARMACEUTICAL COMPOSITIONS AND METHODS OF USE

The compounds of the present invention may be administered to mammals, preferably humans, for the treatment of a variety of conditions and disorders associated with the FPR2 receptor such as Behcet's disease, Sweet disease, systemic lupus erythematosus (SLE), Wegener's granulomatosis, virus infection, diabetes, amputations, cancers, bacterial infection, physical external injuries, physical disorders including exposure to radiation, vasoconstriction, anaphylactic reactions, allergic reactions, rhinitis, shocks (endotoxic, hemorrhagic, traumatic, splanchnic ischemia, and circulatory shocks), rheumatoid arthritis, gout, psoriasis, benign prostatic hyperplasia, myocardial ischemia, myocardial infarction, heart failure, brain injuries, pulmonary diseases, COPD, COAD, COLD, acute lung injury, acute respiratory distress syndrome, chronic bronchitis, pulmonary emphysema, asthma (allergic asthma and non-allergic asthma), cystic fibrosis, kidney fibrosis, nephropathy, renal glomerular diseases, ulcerative colitis, IBD, Crohn's disease, periodontitis, pains, Alzheimer's disease, AIDS, uveitic glaucoma, conjunctivitis, Sjoegren's syndrome, rhinitis, atherosclerosis, neuroinflammatory diseases including multiple sclerosis, stroke, sepsis, and the like.

Unless otherwise specified, the following terms have the stated meanings. The term "subject" refers to any human or other mammalian species that could potentially benefit from treatment with a FPR2 and/or FPR1 agonist as understood by practioners in this field. Some subjects include human beings of any age with risk factors for cardiovascular disease. Common risk factors include age, sex, weight, family history, sleep apnea, alcohol or tobacco use, physical inactivity arrthymia or signs of insulin resistance such as acanthosis nigricans, hypertension, dyslipidemia, or polycystic ovary syndrome (PCOS). The term "patient" means a person suitable for therapy as determined by practitioners in the field. "Treating" or "treatment" cover the treatment of a patient or subject as understood by practitioners in this field. "Preventing" or "prevention" cover the preventive treatment (*i.e.*, prophylaxis and/or risk reduction) of a subclinical disease-state in a patient or subject aimed at reducing the probability of the occurrence of a clinical disease-state as understood by practitioners in this field. Patients are selected for preventative therapy based on factors that are known to increase risk of suffering a clinical disease state compared to the general population. "Therapeutically effective amount" means an amount of a compound that is effective as understood by practitioners in this field.

Another aspect of the invention are pharmaceutical compositions comprising a therapeutically effective amount of a compound of Formulae (I)-(II) in combination with a pharmaceutical carrier.

Another aspect of the invention are pharmaceutical compositions comprising a therapeutically effective amount of a compound of Formulae (I)-(II) in combination with at least one other therapeutic agent and a pharmaceutical carrier.

"Pharmaceutical composition" means a composition comprising a compound of the invention in combination with at least one additional pharmaceutically acceptable carrier. A "pharmaceutically acceptable carrier" refers to media generally accepted in the art for the delivery of biologically active agents to animals, in particular, mammals, including, i.e., adjuvant, excipient or vehicle, such as diluents, preserving agents, fillers, flow regulating agents, disintegrating agents, wetting agents, emulsifying agents, suspending agents, sweetening agents, flavoring agents, perfuming agents, anti-bacterial agents, anti-fungal agents, lubricating agents and dispensing agents, depending on the nature of the mode of administration and dosage forms.

Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, *e.g.*, stabilization of the active agent, binders, etc., well known to those of ordinary skill in the art. Descriptions of suitable pharmaceutically acceptable carriers, and factors involved in their selection, are found in a variety of readily available sources such as, for example, Allen, L.V., Jr. et al., Remington: The Science and Practice of Pharmacy (2 Volumes), 22nd Edition, Pharmaceutical Press (2012).

Particularly when provided as a single dosage unit, the potential exists for a chemical interaction between the combined active ingredients. For this reason, when the compound of the present invention and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material that affects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

Compounds of the present invention may be used in a method for treating heart disease comprising administering a therapeutically effective amount of a compound of Formulae (I)-(II) to a patient.

Compounds of the present invention may be used in a method for treating heart disease wherein the heart disease is selected from the group consisting of angina pectoris, unstable angina, myocardial infarction, heart failure, acute coronary disease, acute heart failure, chronic heart failure, and cardiac iatrogenic damage.

It will be understood that treatment or prophylaxis of heart failure may involve treatment or prophylaxis of a cardiovascular event as well. Treatment or prophylaxis as referred to herein may refer to treatment or prophylaxis of certain negative symptoms or conditions associated with or arising as a result of a cardiovascular event. By way of example, treatment or prophylaxis may involve reducing or preventing negative changes in fractional shortening, heart weight, lung weight, myocyte cross sectional area, pressure overload induced cardiac fibrosis, stress induced cellular senescence, and/or cardiac hypertrophy properties, or any combination thereof, associated with or arising as a result of a cardiovascular event. Treatment may be administered in preparation for or in response to a cardiovascular event to alleviate negative effects. Prevention may involve a pro-active or prophylactic type of treatment to prevent the cardiovascular event or to reduce the onset of negative effects of a cardiovascular event.

Compounds of Formulae (I)-(II) or a pharmaceutically acceptable salt thereof may be used for the preparation of a pharmaceutical composition for the treatment or prophylaxis of heart failure, for example, heart failure results from hypertension, an ischemic heart disease, a non-ischemic heart disease, exposure to a cardiotoxic compound, myocarditis, Kawasaki's disease, Type I and Type II diabetes, thyroid disease, viral infection, gingivitis, drug abuse, alcohol abuse, pericarditis, atherosclerosis, vascular disease, hypertrophic cardiomyopathy, dilated cardiomyopathy, myocardial infarction, atrial fibrosis, left ventricular systolic dysfunction, left ventricular diastolic dysfunction, coronary bypass surgery, pacemaker implantation surgery, starvation, an eating disorder, muscular dystrophies, and a genetic defect. The heart failure to be treated may be diastolic heart failure, heart failure with reduced ejection fraction (HF_{R}EF), heart failure with preserved ejection fraction (HF_{P}EF), acute heart failure, and chronic heart failure of ischemic and non-ischemic origin.

Compounds of Formulae (I)-(II) may be used in a method to treat systolic and/or diastolic dysfunction, wherein the compound is administered in a therapeutically effective amount to increase the ability of the cardiac muscle cells to contract and relax thereby increasing the filling and emptying of both the right and left ventricles, preferably, the left ventricle.

Compounds of Formulae (I)-(II) may be used in a method to treat heart failure wherein the compound is administered in a therapeutically effective amount to increase ejection fraction in the left ventricle.

Compounds of Formulae (I)-(II) may be used in a method to treat heart failure wherein the compound is administered in a therapeutically effective amount to reduce fibrosis in heart tissue.

Compounds of the present invention may be used in a method for treating heart disease wherein the treatment is post myocardial infarction.

Compounds of the present invention may be used in a method for treating heart disease comprising administering a therapeutically effective amount of a compound of Formulae (I)-(II) to a patient in conjuction with other therapeutic agents.

The compounds of this invention can be administered by any suitable means, for example, orally, such as tablets, capsules (each of which includes sustained release or timed release formulations), pills, powders, granules, elixirs, tinctures, suspensions (including nanosuspensions, microsuspensions, spray-dried dispersions), syrups, and emulsions; sublingually; bucally; parenterally, such as by subcutaneous, intravenous, intramuscular, or intrasternal injection, or infusion techniques (e.g., as sterile injectable aqueous or non-aqueous solutions or suspensions); nasally, including administration to the nasal membranes, such as by inhalation spray; topically, such as in the form of a cream or ointment; or rectally such as in the form of suppositories. They can be administered alone, but generally will be administered with a pharmaceutical carrier selected on the basis of the chosen route of administration and standard pharmaceutical practice.

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired.

By way of general guidance, the daily oral dosage of each active ingredient, when used for the indicated effects, will range between about 0.01 to about 5000 mg per day, preferably between about 0.1 to about 1000 mg per day, and most preferably between about 0.1 to about 250 mg per day. Intravenously, the most preferred doses will range from about 0.01 to about 10 mg/kg/minute during a constant rate infusion. Compounds of this invention may be administered in a single daily dose, or the total daily dosage may be administered in divided doses of two, three, or four times daily.

Dosage forms (pharmaceutical compositions) suitable for administration may contain from about 1 milligram to about 2000 milligrams of active ingredient per dosage unit. In these pharmaceutical compositions the active ingredient will ordinarily be present in an amount of about 0.1-95% by weight based on the total weight of the composition. A typical capsule for oral administration contains at least one of the compounds of the present invention (250 mg), lactose (75 mg), and magnesium stearate (15 mg). The mixture is passed through a 60 mesh sieve and packed into a No. 1 gelatin capsule. A typical injectable preparation is produced by aseptically placing at least one of the compounds of the present invention (250 mg) into a vial, aseptically freeze-drying and sealing. For use, the contents of the vial are mixed with 2 mL of physiological saline, to produce an injectable preparation.

The compounds of the present invention may be employed in combination with other suitable therapeutic agents useful in the treatment of the aforementioned diseases or disorders including: anti-atherosclerotic agents, anti-dyslipidemic agents, anti-diabetic agents, anti-hyperglycemic agents, anti-hyperinsulinemic agents, anti-thrombotic agents, anti-retinopathic agents, anti-neuropathic agents, anti-nephropathic agents, anti-ischemic agents, anti-hypertensive agents, anti-obesity agents, anti-hyperlipidemic agents, anti-hypertriglyceridemic agents, anti-hypercholesterolemic agents, anti-restenotic agents, anti-pancreatic agents, lipid lowering agents, anorectic agents, memory enhancing agents, anti-dementia agents, cognition promoting agents, appetite suppressants, agents for treating heart failure, agents for treating peripheral arterial disease, agents for treating malignant tumors, and anti-inflammatory agents.

The compounds of the invention may be used with at least one of the following heart failure agents selected from loop diuretics, Angiotensin converting enzyme (ACE) inhibitors, Angiotensin II receptor blockers (ARBs), angiotensin receptor-neprilysin inhibitors (ARNI), beta blockers, mineralocorticoid receptor antagonists, nitroxyl donors, RXFP1 agonists, APJ agonists and cardiotonic agents. These agents include, but are not limited to furosemide, bumetanide, torsemide, sacubitrial-valsartan, thiazide diruetics, captopril, enalapril, lisinopril, carvedilol, metopolol, bisoprolol, serelaxin, spironolactone, eplerenone, ivabradine, candesartan, eprosartan, irbestarain, losartan, olmesartan, telmisartan, and valsartan.

The compounds of the present invention may be employed in combination with at least one of the following therapeutic agents in treating atherosclerosis: anti-hyperlipidemic agents, plasma HDL-raising agents, anti-hypercholesterolemic agents, cholesterol biosynthesis inhibitors (such as HMG CoA reductase inhibitors), LXR agonist, probucol, raloxifene, nicotinic acid, niacinamide, cholesterol absorption inhibitors, bile acid sequestrants (such as anion exchange resins, or quaternary amines (e.g., cholestyramine or colestipol)), low density lipoprotein receptor inducers, clofibrate, fenofibrate, benzofibrate, cipofibrate, gemfibrizol, vitamin B₆, vitamin B₁₂, anti-oxidant vitamins, β-blockers, anti-diabetes agents, angiotensin II antagonists, angiotensin converting enzyme inhibitors, platelet aggregation inhibitors, fibrinogen receptor antagonists, aspirin and fibric acid derivatives.

The compounds of the present invention may be employed in combination at least one of the following therapeutic agents in treating cholesterol biosynthesis inhibitor, particularly an HMG-CoA reductase inhibitor. Examples of suitable HMG-CoA reductase inhibitors include, but are not limited to, lovastatin, simvastatin, pravastatin, fluvastatin, atorvastatin, and rosuvastatin.

The compounds of the invention may be used in combination with at least one of the following anti-diabetic agents depending on the desired target therapy. Studies indicate that diabetes and hyperlipidemia modulation can be further improved by the addition of a second agent to the therapeutic regimen. Examples of anti-diabetic agents include, but are not limited to, sulfonylureas (such as chlorpropamide, tolbutamide, acetohexamide, tolazamide, glyburide, gliclazide, glynase, glimepiride, and glipizide), biguanides (such as metformin), thiazolidinediones (such as ciglitazone, pioglitazone, troglitazone, and rosiglitazone), and related insulin sensitizers, such as selective and non-selective activators of PPARα, PPARβ and PPARγ; dehydroepiandrosterone (also referred to as DHEA or its conjugated sulphate ester, DHEA-SO₄); anti-glucocorticoids; TNFα inhibitors; dipeptidyl peptidase IV (DPP4) inhibitor (such as sitagliptin, saxagliptin), GLP-1 agonists or analogs (such as exenatide), α-glucosidase inhibitors (such as acarbose, miglitol, and voglibose), pramlintide (a synthetic analog of the human hormone amylin), other insulin secretagogues (such as repaglinide, gliquidone, and nateglinide), insulin, as well as the therapeutic agents discussed above for treating atherosclerosis.

The compounds of the invention may be used in combination with at least one of the following anti-obesity agents selected from phenylpropanolamine, phentermine, diethylpropion, mazindol, fenfluramine, dexfenfluramine, phentiramine, β₃-adrenoreceptor agonist agents; sibutramine, gastrointestinal lipase inhibitors (such as orlistat), and leptins. Other agents used in treating obesity or obesity-related disorders include neuropeptide Y, enterostatin, cholecytokinin, bombesin, amylin, histamine H₃ receptors, dopamine D₂ receptor modulators, melanocyte stimulating hormone, corticotrophin releasing factor, galanin and gamma amino butyric acid (GABA).

The compounds of the present invention are also useful as standard or reference compounds, for example as a quality standard or control, in tests or assays involving the FPR2. Such compounds may be provided in a commercial kit, for example, for use in pharmaceutical research involving FPR2 activity. For example, a compound of the present invention could be used as a reference in an assay to compare its known activity to a compound with an unknown activity. This would ensure the experimenter that the assay was being performed properly and provide a basis for comparison, especially if the test compound was a derivative of the reference compound. When developing new assays or protocols, compounds according to the present invention could be used to test their effectiveness. The compounds of the present invention may also be used in diagnoswtic assays involving FPR2.

The compounds of the present invention may be used in an article of manufacture. As used herein, article of manufacture is intended to include, but not be limited to, kits and packages. The article of manufacture may comprise: (a) a first container; (b) a pharmaceutical composition located within the first container, wherein the composition, comprises a first therapeutic agent, comprising a compound of the present invention or a pharmaceutically acceptable salt form thereof; and, (c) a package insert stating that the pharmaceutical composition can be used for the treatment of dyslipidemias and the sequelae thereof. The package insert may state that the pharmaceutical composition can be used in combination (as defined previously) with a second therapeutic agent for the treatment of dyslipidemias and the sequelae thereof. The article of manufacture can further comprise: (d) a second container, wherein components (a) and (b) are located within the second container and component (c) is located within or outside of the second container. Located within the first and second containers means that the respective container holds the item within its boundaries. The first container is a receptacle used to hold a pharmaceutical composition. This container can be for manufacturing, storing, shipping, and/or individual/bulk selling. First container is intended to cover a bottle, jar, vial, flask, syringe, tube (*e.g.*, for a cream preparation), or any other container used to manufacture, hold, store, or distribute a pharmaceutical product. The second container is one used to hold the first container and, optionally, the package insert. Examples of the second container include, but are not limited to, boxes (*e.g.*, cardboard or plastic), crates, cartons, bags (*e.g.*, paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached. The package insert is a label, tag, marker, etc. that recites information relating to the pharmaceutical composition located within the first container. The information recited will usually be determined by the regulatory agency governing the area in which the article of manufacture is to be sold (*e.g.*, the United States Food and Drug Administration). Preferably, the package insert specifically recites the indications for which the pharmaceutical composition has been approved. The package insert may be made of any material on which a person can read information contained therein or thereon. Preferably, the package insert is a printable material (*e.g.*, paper, plastic, cardboard, foil, adhesive-backed paper or plastic, etc.) on which the desired information has been formed (*e.g.*, printed or applied).

### CHEMISTRY METHODS

Abbreviations as used herein, are defined as follows: "1x" for once, "2x" for twice, "3x" for thrice, "°C" for degrees Celsius, "aq" for aqueous, "Col" for column, "eq" for equivalent or equivalents, "g" for gram or grams, "mg" for milligram or milligrams, "L" for liter or liters, "mL" for milliliter or milliliters, "µL" for microliter or microliters, "N" for normal, "M" for molar, "nM" for nanomolar, "mol" for mole or moles, "mmol" for millimole or millimoles, "min" for minute or minutes, "h" for hour or hours, "rt" for room temperature, "ON" for overnight, "atm" for atmosphere, "psi" for pounds per square inch, "conc." for concentrate, "aq" for "aqueous", "sat" or "sat'd " for saturated, "MW" for molecular weight, "mw" or "µwave" for microwave, "mp" for melting point, "Wt" for weight, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "nOe" for nuclear Overhauser effect spectroscopy, "¹H" for proton, "δ " for delta, "s" for singlet, "d" for doublet, "t" for triplet, "q" for quartet, "m" for multiplet, "br" for broad, "Hz" for hertz, and "α", "β", "R", "S", "E", and "Z" are stereochemical designations familiar to one skilled in the art.

### Abbreviations:

- AcOH or HOAc: acetic acid
- ACN: acetonitrile
- ADDP: 1,1'-(azodicarbonyl) dipiperidine
- BOP: (Benzotriazol-1-yloxy)tris(dimethylamino)phosphonium hexafluorophosphate
- CDCl₃: deutero-chloroform
- CD₃OD: deutero-methanol
- CDI: 1,1'-carbonyldiimidazole
- conc: concentrated
- DCM: dichloromethane
- DIEA or DIPEA: diisopropylethylamine
- DMF: dimethyl formamide
- DMSO: dimethyl sulfoxide
- DMSO-d₆: deutero-dimethyl sulfoxide
- Et₃N or TEA: triethylamine
- EtOAc: ethyl acetate
- EtOH: ethanol
- HATU: 1-[bis(dimethylamino)methylene]-1H-1,2,3-triazolo[4,5-b]pyridinium 3-oxide hexafluorophosphate
- HCl: hydrochloric acid
- HPLC: high-performance liquid chromatography
- K₂HPO₄: potassium hydrogenphosphate
- LCMS: liquid chromatography mass spectrometry
- MeOH: methanol
- MgSO₄: magnesium sulfate
- NMP: N-methyl-2-pyrrolidone
- NaCl: sodium chloride
- Na₂CO₃: sodium carbonate
- NaHCO₃: sodium bicarbonate
- NaOH: sodium hydroxide
- Na₂SO₄: sodium sulfate
- NH₄Cl: ammonium chloride
- NH₄OAc: ammonium acetate
- Pd(OAc)₂: palladium(II) acetate
- Pd(PPh₃)₄: tetrakis(triphenylphosphine)palladium(0)
- Rt: retention time
- SiO₂: silica oxide
- SOCl₂: thionyl chloride
- TEA: triethylamine
- TFA: trifluoroacetic acid
- THF: tetrahydrofuran
- T3P^{®}: 1-propanephosphonic acid cyclic anhydride

The compounds of the present invention can be prepared in a number of ways known to one skilled in the art of organic synthesis. The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention.

The novel compounds of this invention may be prepared using the reactions and techniques described in this section. Also, in the description of the synthetic methods described below, it is to be understood that all proposed reaction conditions, including choice of solvent, reaction atmosphere, reaction temperature, duration of the experiment and workup procedures, are chosen to be the conditions standard for that reaction, which should be readily recognized by one skilled in the art. Restrictions to the substituents that are compatible with the reaction conditions will be readily apparent to one skilled in the art and alternate methods must then be used.

### SYNTHESIS

The compounds of Formula (I), wherein R¹, R², R³, and R⁴ are defined below, may be prepared by the exemplary processes described in the following schemes and working examples, as well as relevant published literature procedures that are used by one skilled in the art. Exemplary reagents and procedures for these reactions appear hereinafter and in the working examples. Protection and de-protection in the processes below may be carried out by procedures generally known in the art (see, for example, Wuts, P.G.M. et al., Protecting Groups in Organic Synthesis, 4th Edition, Wiley (2007)). General methods of organic synthesis and functional group transformations are found in: Trost, B.M. et al., eds., Comprehensive Organic Synthesis: Selectivity, Strategy & Efficiency in Modern Organic Chemistry, Pergamon Press, New York, NY (1991); Smith, M.B. et al., March's Advanced Organic Chemistry: Reactions, Mechanisms, and Structure. 6th Edition, Wiley & Sons, New York, NY (2007); Katritzky, A.R. et al, eds., Comprehensive Organic Functional Groups Transformations II, 2nd Edition, Elsevier Science Inc., Tarrytown, NY (2004); Larock, R.C., Comprehensive Organic Transformations, VCH Publishers, Inc., New York, NY (1999), and references therein.

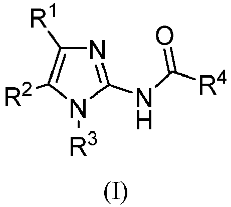

Aminoimidazole compounds of this invention in which R³ is a substituted phenyl can be prepared using the general route as described in Scheme 1.

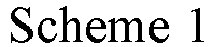

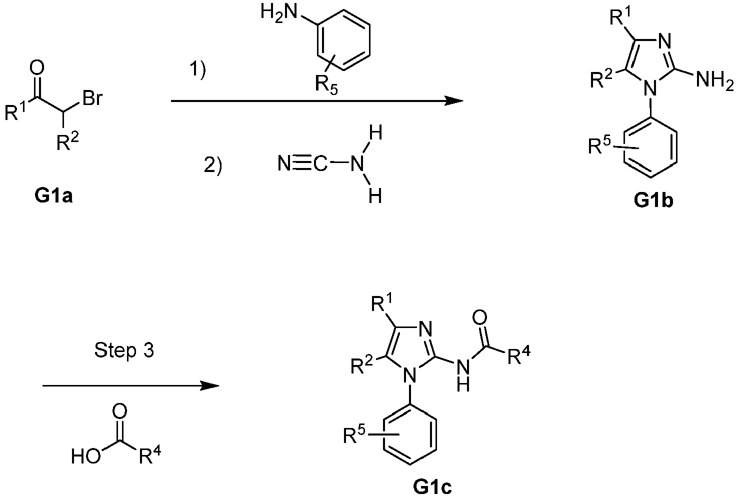

Displacement of a substituted alpha-bromo ketone **G1a**, with an aniline in the presence of an inorganic base such as sodium bicarbonate in a suitable solvent such as DMF and subsequent condensation with excess cyanamide in a solvent such as MeOH or NMP can result in **G1b**. Coupling with an aryl carboxylic acid activated by, for example, HATU or BOP in presence of a tertiary amine base (e.g., TEA, DIEA) can provide substituted aminoimidazole **G1c**.

### EXPERIMENTALS

The following methods were used in the exemplified Examples, except where noted otherwise. Purification of intermediates and final products was carried out via either normal or reverse phase chromatography. Normal phase chromatography was carried out using prepacked SiO₂ cartridges eluting with either gradients of hexanes and EtOAc or DCM and MeOH unless otherwise indicated.

Reverse phase preparative HPLC of Examples was carried out using Waters XBridge C18 column (19 x 200 mm, 5-µm particles) with UV and LCMS detection using variable gradients of mobile phase A (95% water, 5% ACN) and mobile phase B (5% water, 95% ACN) containing 0.1% TFA or 10 mM NH₄OAc.

Reverse phase analytical HPLC/MS was performed on a Waters Acquity system coupled with a Waters MICROMASS^{®} ZQ Mass Spectrometer.
Method A: Linear gradient of 0 to 100% B over 3 min, with 0.75 min hold time at 100% B;
   UV visualization at 220 nm
   Column: Waters BEH C18 2.1 × 50 mm
   Flow rate: 1.0 mL/min
   Solvent A: 10 mM NH₄OAc, 95% water, 5% ACN
   Solvent B: 10 mM NH₄OAc, 5% water, 95% ACN
Method B: Linear gradient of 0 to 100% B over 3 min, with 0.75 min hold time at 100% B;
   UV visualization at 220 nm
   Column: Waters BEH C18 2.1 × 50 mm
   Flow rate: 1.0 mL/min
   Solvent A: 0.1% TFA, 95% water, 5% ACN
   Solvent B: 0.1% TFA, 5% water, 95% ACN
Method C: Linear gradient of 2 to 98% B over 1 min, with 0.50 min hold time at 100% B;
   UV visualization at 220 nm
   Column: Waters BEH C18 2.1 × 50 mm
   Flow rate: 0.8 mL/min
   Solvent A: water containing 0.05% TFA
   Solvent B: ACN containing 0.05% TFA
¹H NMR spectra were obtained with Bruker spectrometers operating at frequencies 300 MHz, 400 MHz or 500 MHz. Spectra data are reported in the format: chemical shift (multiplicity, coupling constants, and number of hydrogens). Chemical shifts are specified in ppm downfield of a tetramethylsilane internal standard (δ units, tetramethylsilane = 0 ppm) and/or referenced to solvent peaks, which in ¹H NMR spectra appear at 2.50 ppm for (CD₃)₂SO, 3.31 ppm for CD₃OD, 1.94 ppm for CD₃CN, and 7.26 ppm for CDCl₃.

### Example 1: N-[1-(2,6-Difluoro-4-methoxyphenyl)-4-phenyl-1H-imidazol-2-yl]-4-(difluoromethoxy)benzamide

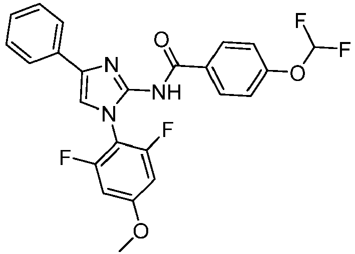

### Intermediate 1: 1-(2,6-Difluoro-4-methoxyphenyl)-4-phenyl-1H-imidazol-2-amine

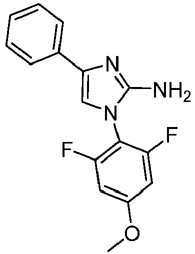

To a stirred solution of 2,6-difluoro-4-methoxyaniline in DMF (3.8 mL) at room temperature was added sodium bicarbonate (71 mg, 0.85 mmol) followed by 2-bromo-1-phenylethanone (180 mg, 0.88 mmol). The resulting solution was stirred at room temperature for 16 h. To the resulting intermediate was added cyanamide (264 mg, 6.28 mmol). The resulting solution was heated at 95 °C for 16 h. The crude material was purified by reverse phase HPLC to afford Intermediae 1 (25 mg, 0.084 mmol, 13% yield). MS(ESI) m/z: 302.3 (M+H)⁺. ¹H NMR (500 MHz, DMSO-d₆) *δ* 7.65 (br d, *J* = 7.6 Hz, 2H), 7.31 (t, *J* = 7.6 Hz, 2H), 7.23 - 7.10 (m, 2H), 6.96 (br d, *J* = 9.8 Hz, 2H), 5.60 (s, 2H), 3.84 (s, 3H).

### Example 1: N-[1-(2,6-Difluoro-4-methoxyphenyl)-4-phenyl-1H-imidazol-2-yl]-4-(difluoromethoxy)benzamide

To a stirred solution of 4-(difluoromethoxy) benzoic acid (79 mg, 0.42 mmol) in DMF (0.25 ml) was added BOP (190 mg, 0.42 mmol) followed by DIEA (0.15 mL, 0.84 mmol). The resulting solution was stirred at room temperature for 15 min. To the resulting mixture was added a solution of Intermediate 1 (25 mg, 0.084 mmol) in DMF (0.25 mL) and the reaction was heated at 50 °C for 16 h. The crude material was purified by reverse phase HPLC to afford Example 1 (18 mg, 0.038 mmol, 45% yield). MS(ESI) m/z: 472.2 (M+H)⁺.

The following Examples in Table 2 were prepared as described for Example 1.

**Table 2**

| Ex. No. | Name | Structure | Obs Mass | Method Rt Purity |
|---|---|---|---|---|
| 2 | 4-(Difluoromethoxy)-*N-*[1-(4-methoxyphenyl)-4-phenyl-1*H*-imidazol-2-yl]benzamide | 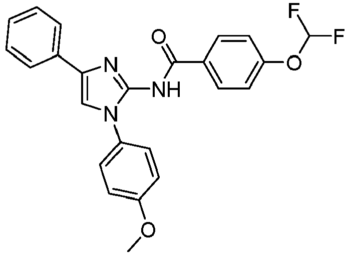 | 436.3 | Method A, Rt = 1.75 min, 96.5% |
| 3 | *N*-[1-(2,6-Difluoro-4-methoxyphenyl)-4-(3- methoxyphenyl)-1*H-*imidazol-2-yl]-4-(difluoromethoxy)benz amide | 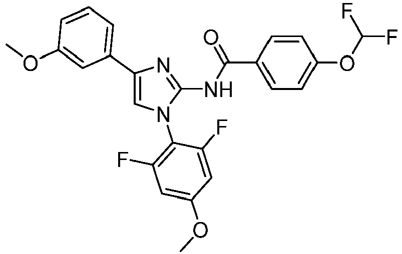 | 502.4 | Method B, Rt = 2.01 min, 97% |
| 4 | *N*-[1-(2,6-Difluoro-4-methoxyphenyl)-4-(4-fluorophenyl)-1*H-*imidazol-2-yl]-4-(difluoromethoxy)benz amide | 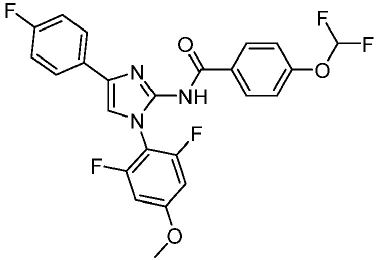 | 490.3 | Method A, Rt = 1.98 min, 96.2% |
| 5 | *N*-[4-(4-Chloro-3-methylphenyl)-1-(2,6-difluoro-4-methoxyphenyl)-1*H-*imidazol-2-yl]-4-(difluoromethoxy)benz amide | 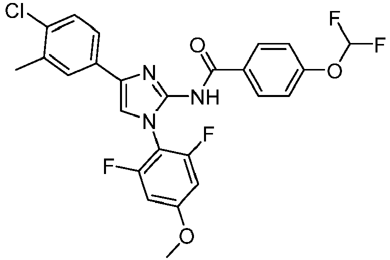 | 520.3 | Method B, Rt = 2.27 min, 100% |
| 6 | *N*-[1-(2,6-Difluoro-4-methoxyphenyl)-4-[4-(trifluoromethyl)phenyl ]-1*H*-imidazol-2-yl]-4-(difluoromethoxy)benz amide | 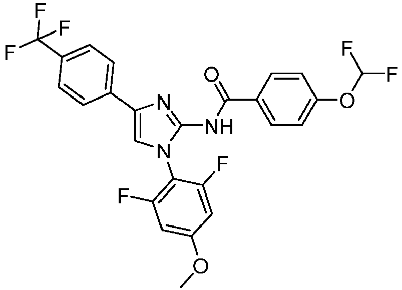 | 540.3 | Method B, Rt = 2.18 min, 97.6% |
| 7 | *N-*[1-(2,6-Difluoro-4-methoxyphenyl)-4-[4-(difluoromethoxy)phen yl]-1*H*-imidazol-2-yl]-4-(difluoromethoxy)benz amide | 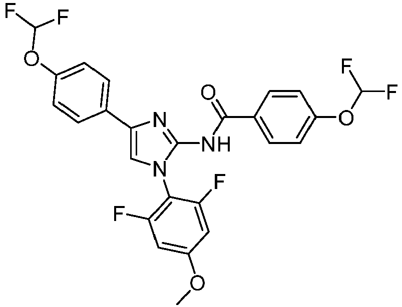 | 538.1 | Method B, Rt = 2.13 min, 100% |
| 8 | *N-*[1-(2,6-Difluoro-4-methoxyphenyl)-5-methyl-4-phenyl-1*H-*imidazol-2-yl]-4-(difluoromethoxy)benz amide | 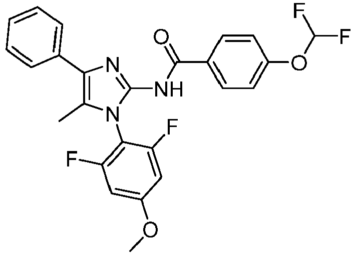 | 486.4 | Method A, Rt = 1.95 min, 98% |
| 9 | *N*-[1-(2,6-Difluoro-4-methoxyphenyl)-4-(naphthalen-2-yl)-1*H-*imidazol-2-yl]-4-(difluoromethoxy)benz amide | 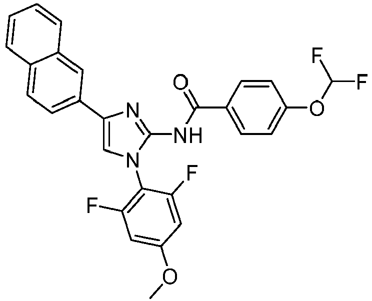 | 522 | Method B, Rt = 2.31 min, 100% |
| 10 | *N*-[1-(2,6-Difluoro-4-methoxyphenyl)-4-(3 - fluorophenyl)-1*H-*imidazol-2-yl]-4-(difluoromethoxy)benz amide | 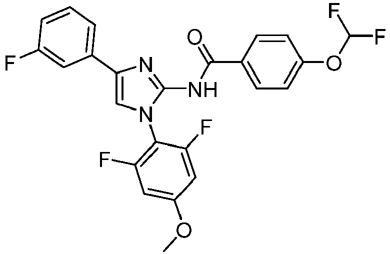 | 490.3 | Method B, Rt = 2.02 min, 96.7% |
| 11 | *N*-[4-(4-Chlorophenyl)-1-(2,6-difluoro-4-methoxyphenyl)-1*H-*imidazol-2-yl]-4-(difluoromethoxy)benz amide | 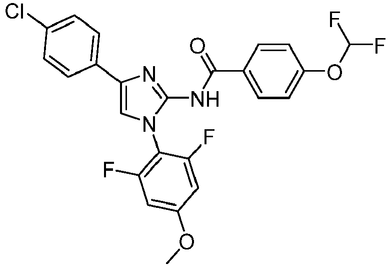 | 506.3 | Method A, Rt = 2.11 min, 98.2% |
| 12 | *N*-[4-(3-Chlorophenyl)-1-(2,6-difluoro-4-methoxyphenyl)-1*H-*imidazol-2-yl]-4-(difluoromethoxy)benz amide | 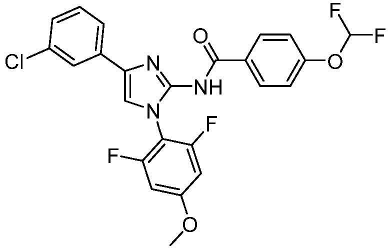 | 506.3 | Method B, Rt = 2.13 min, 96.8% |
| 13 | *N*-[4-(4-Chlorophenyl)-1-(2,6-difluoro-4-methoxyphenyl)-5-methyl-1*H*-imidazol-2-yl]-4-(difluoromethoxy)benz amide | 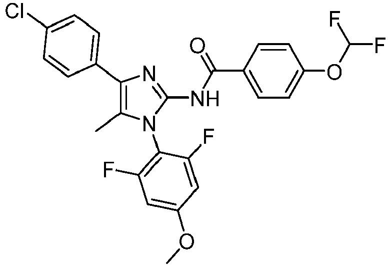 | 520.3 | Method B, Rt = 2.25 min, 96.7% |
| 14 | *N*-[1-(2,6-Difluoro-4-methoxyphenyl)-4-(4-methoxyphenyl)-1*H-*imidazol-2-yl]-4-(difluoromethoxy)benz amide | 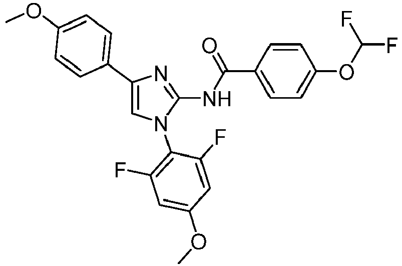 | 501.9 | Method B, Rt = 2.09 min, 100% |
| 15 | *N-*[1-(2,6-Difluoro-4-methoxyphenyl)-4-(4-methylphenyl)-1*H-*imidazol-2-yl]-4-(difluoromethoxy)benz amide | 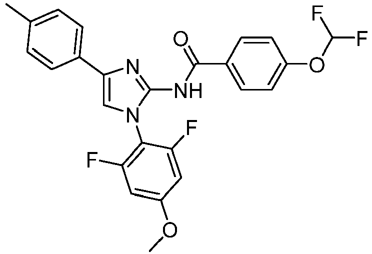 | 486.4 | Method A, Rt = 2.08 min, 97.1% |
| 16 | *N*-[4-(4-Cyanophenyl)-1-(2,6-difluoro-4-methoxyphenyl)-1*H-*imidazol-2-yl]-4-(difluoromethoxy)benz amide | 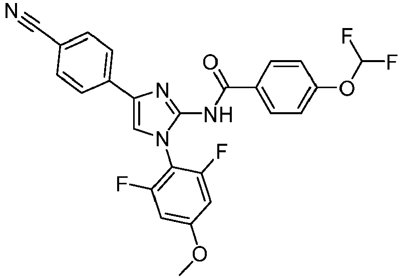 | 497.3 | Method B, Rt = 1.89 min, 93.8% |
| 17 | *N*-[4-(3-Cyanophenyl)-1-(2,6-difluoro-4-methoxyphenyl)-1*H-*imidazol-2-yl]-4-(difluoromethoxy)benz amide | 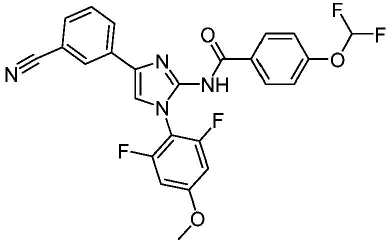 | 497.2 | Method B, Rt = 1.97 min, 100% |
| 18 | *N*-[4-(3,4-Dichlorophenyl)-1-(2,6-difluoro-4-methoxyphenyl)-1*H-*imidazol-2-yl]-4-(difluoromethoxy)benz amide | 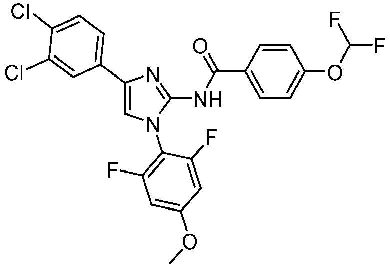 | 540 | Method A, Rt = 2.28 min, 96.7% |
| 19 | 4-(Difluoromethoxy)-*N*-[4-(2,3-dihydro-1-benzofuran-5-yl)-1-(4-methoxyphenyl)-1*H-*imidazol-2-yl]benzamide | 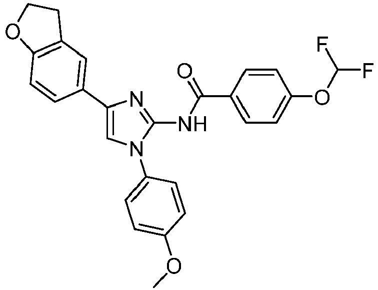 | 478.1 | Method A, Rt = 1.75 min, 93.7% |
| 20 | *N*-[4-(2-Chloropyridin-4-yl)-1-(4-methoxyphenyl)-1*H-*imidazol-2-yl]-4-(difluoromethoxy)benz amide | 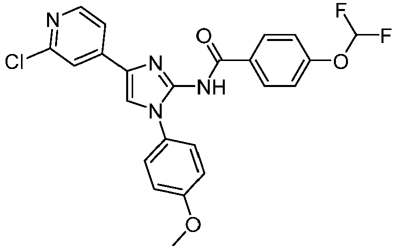 | 471.1 | Method A, Rt = 1.76 min, 96.1% |
| 21 | 4-(Difluoromethoxy)-N [4-(2,4-difluorophenyl)-1-(4-methoxyphenyl)-1*H-*imidazol-2-yl]benzamide | 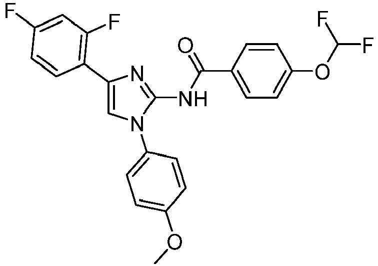 | 472.1 | Method A, Rt = 1.97 min, 97.8% |
| 22 | *N*-[4-(1-Benzothiophen-5-yl)-1-(4-methoxyphenyl)-1*H*-imidazol-2-yl]-4-(difluoromethoxy)benz amide | 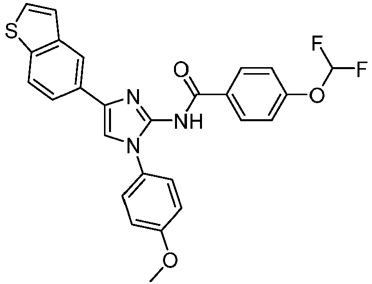 | 492.1 | Method A, Rt = 1.95 min, 95.7% |
| 23 | *N*-[4-(1,3-Benzothiazol-2-yl)-1-(4-methoxyphenyl)-1*H*-imidazol-2-yl]-4-(difluoromethoxy)benz amide | 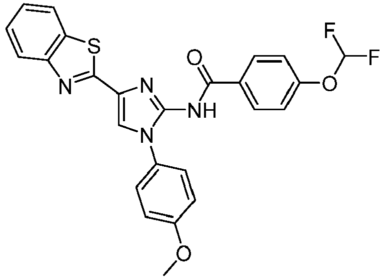 | 493.1 | Method B, Rt = 2.0 min, 95.5% |
| 24 | 4-(Difluoromethoxy)-*N*-[1-(4-methoxyphenyl)-4-(1,3-thiazol-2-yl)-1*H-*imidazol-2-yl]benzamide | 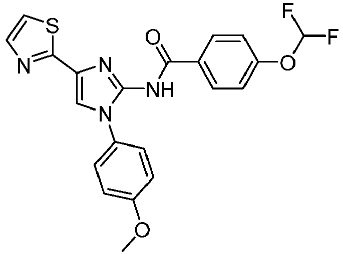 | 442.9 | Method A, Rt = 1.59 min, 96.6% |
| 25 | 4-(Difluoromethoxy)-*N*-[4-(2-fluorophenyl)-1-(4-methoxyphenyl)-1*H*-imidazol-2-yl]benzamide | 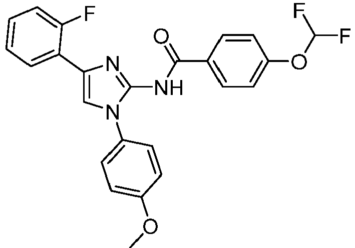 | 453.9 | Method A, Rt = 2.0 min, 95.4% |
| 26 | *N*-[4-(5-Chloro-1-benzofuran-2-yl)-1-(4-methoxyphenyl)-1*H-*imidazol-2-yl]-4-(difluoromethoxy)benz amide | 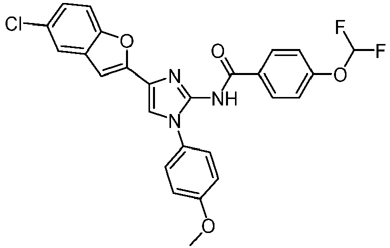 | 510.1 | Method A, Rt = 2.2 min, 98.1% |
| 27 | *N*-[4-Cyclohexyl-1-(4-methoxyphenyl)-1*H-*imidazol-2-yl]-4-(difluoromethoxy)benz amide | 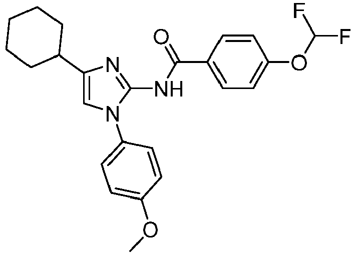 | 442.1 | Method A, Rt = 1.78 min, 95.9% |
| 28 | 4-(Difluoromethoxy)-*N-*[1-(4-methoxyphenyl)-4-(pyridin-2-yl)-1*H-*imidazol-2-yl]benzamide | 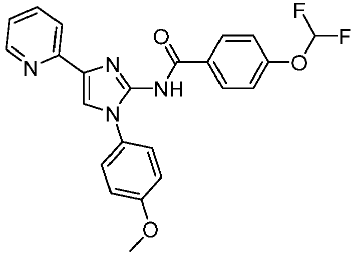 | 437 | Method B, Rt = 1.71 min, 96.8% |
| 29 | Ethyl 5-{2-[4-(difluoromethoxy)benz amido]-1-(4-methoxyphenyl)-1*H-*imidazol-4-yl}-1,2-oxazole-3-carboxylate | 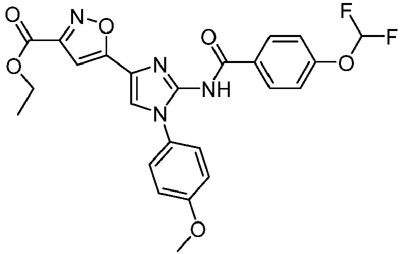 | 499.1 | Method A, Rt = 1.82 min, 93.5% |
| 30 | Ethyl 2-[4-(difluoromethoxy)benz amido]-1-(4-methoxyphenyl)-1*H-*imidazole-4-carboxylate | 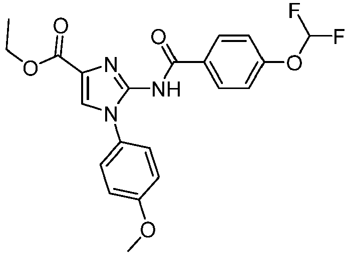 | 432 | Method A, Rt = 1.65 min, 98.4% |
| 31 | *N*-[4-(Adamantan-1-yl)-1-(4-methoxyphenyl)-1*H-*imidazol-2-yl]-4-(difluoromethoxy)benz amide | 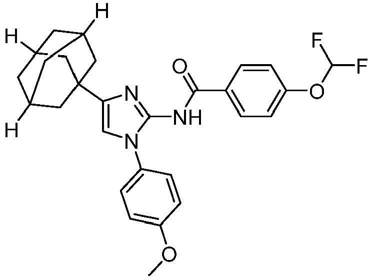 | 494.2 | Method A, Rt = 2.0 min, 99.3% |
| 32 | *N*-[4-Tert-butyl-1-(4-methoxyphenyl)-1*H-*imidazol-2-yl]-4-(difluoromethoxy)benz amide | 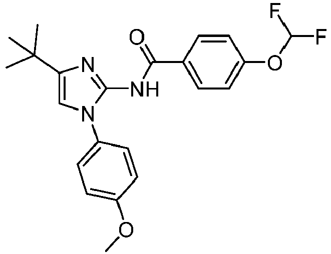 | 416.1 | Method A, Rt = 1.59 min, 96.2% |
| 33 | *N*-[1-(2,6-Difluoro-4-methoxyphenyl)-4-(2-fluorophenyl)-1*H-*imidazol-2-yl]-4-(difluoromethoxy)benz amide | 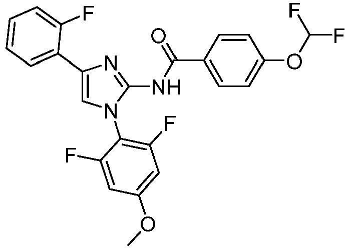 | 490.2 | Method A, Rt = 2.09 min, 97.5% |
| 34 | *N*-[4-(2-Chloropyridin-4-yl)-1-(2,6-difluoro-4-methoxyphenyl)-1*H-*imidazol-2-yl]-4-(difluoromethoxy)benz amide | 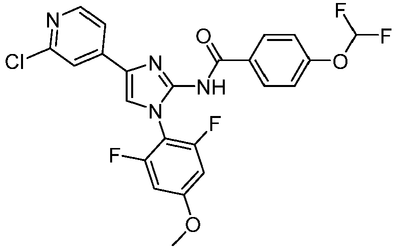 | 507.2 | Method A, Rt = 1.8 min, 100% |
| 35 | *N-*[1-(2,6-Difluoro-4-methoxyphenyl)-4-(2-methylpropyl)-1*H-*imidazol-2-yl]-4-(difluoromethoxy)benz amide | 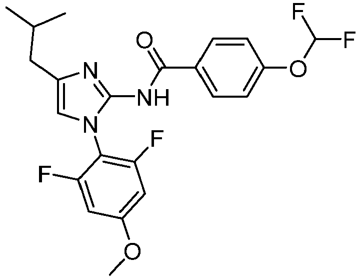 | 452.2 | Method A, Rt = 1.87 min, 96.4% |
| 36 | *N-*[1-(2,6-Difluoro-4-methoxyphenyl)-4-(3,4-difluorophenyl)-1*H*-imidazol-2-yl]-4-(difluoromethoxy)benz amide | 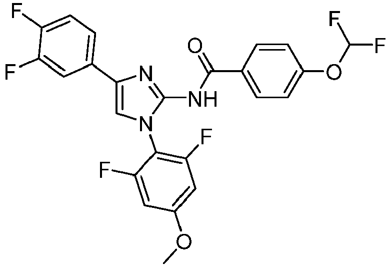 | 508.2 | Method B, Rt = 2.09 min, 100% |
| 37 | *N*-[4-Cyclopropyl-1-(2,6-difluoro-4-methoxyphenyl)-1*H-*imidazol-2-yl]-4-(difluoromethoxy)benz amide | 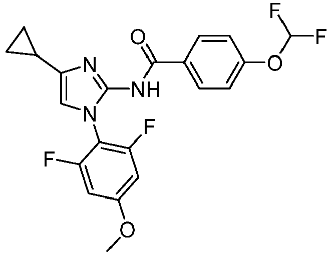 | 436.2 | Method A, Rt = 1.63 min, 98.2% |
| 38 | Ethyl 1-(2,6-difluoro-4-methoxyphenyl)-2-[4-(difluoromethoxy)benz amido]-1*H*-imidazole-4-carboxylate | 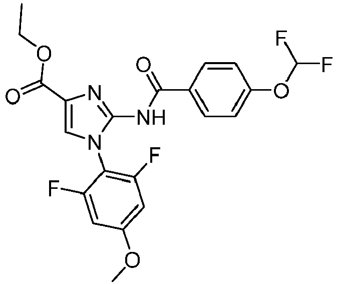 | 468.3 | Method A, Rt = 1.8 min, 94.7% |
| 39 | *N-*[1-(2,6-Difluoro-4-methoxyphenyl)-4-ethyl-1*H*-imidazol-2-yl]-4-(difluoromethoxy)benz amide | 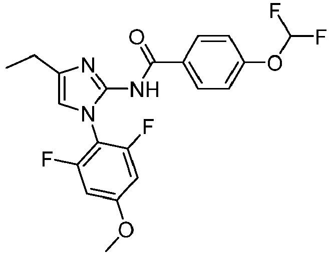 | 424 | Method A, Rt = 1.54 min, 99.3% |
| 40 | *N-*[1-(2,6-Difluoro-4-methoxyphenyl)-4-(1,3-thiazol-2-yl)-1*H-*imidazol-2-yl]-4-(difluoromethoxy)benz amide | 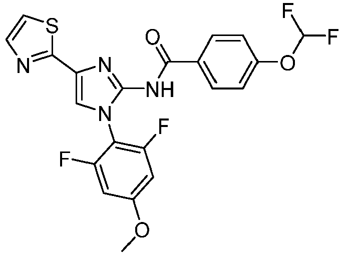 | 478.9 | Method A, Rt = 1.62 min, 98.4% |
| 41 | *N*-[1-(2,6-Difluoro-4-methoxyphenyl)-4-(oxan-4-yl)-1*H-*imidazol-2-yl]-4-(difluoromethoxy)benz amide | 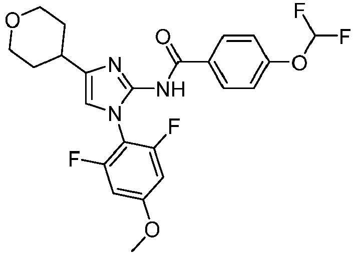 | 480.2 | Method B, Rt = 1.81 min, 96.4% |
| 42 | 4-(Difluoromethoxy)-*N-*[1-(4-methoxyphenyl)-4-(1,1,2,2,2-pentafluoroethyl)-1*H-*imidazol-2-yl]benzamide | 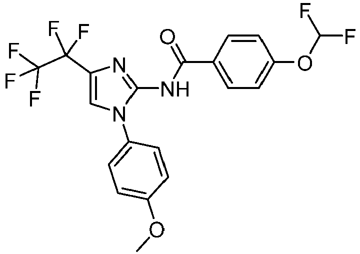 | 477.9 | Method B, Rt = 1.94 min, 100% |
| 43 | 4-(Difluoromethoxy)-*N-*[1-(4-methoxyphenyl)-4-(trifluoromethyl)-1*H-*imidazol-2-yl]benzamide | 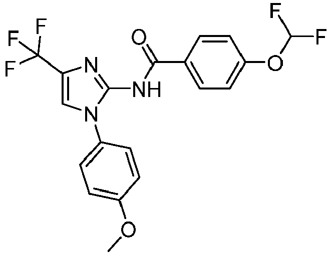 | 427.9 | Method B, Rt = 1.77 min, 94.5% |

### Example 44: 1-(2,6-Difluoro-4-methoxyphenyl)-2-[4-(difluoromethoxy)benzamido]-N-[(1H-pyrazol-3-yl)methyl]-1H-imidazole-4-carboxamide

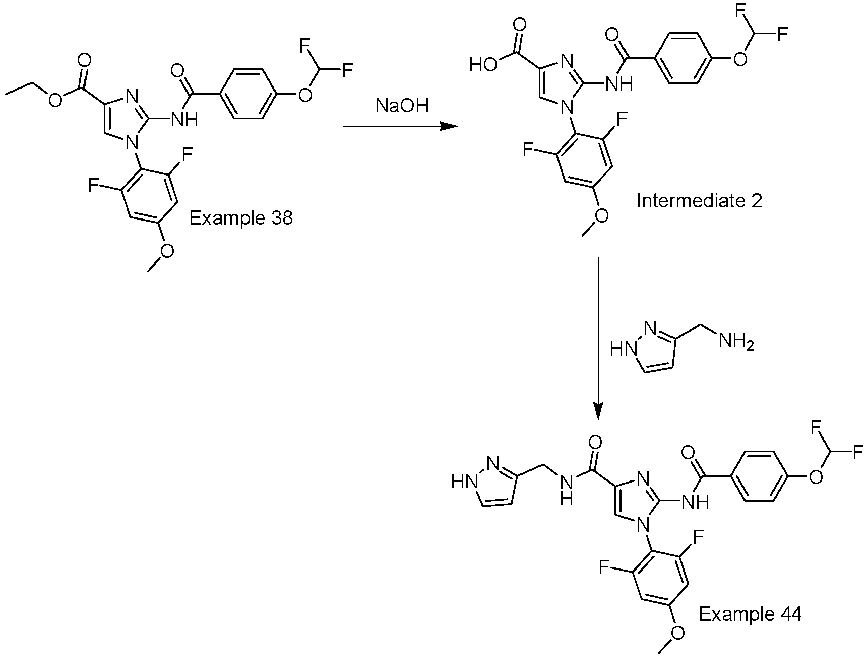

### Intermediate 2: 1-(2,6-Difluoro-4-methoxyphenyl)-2-(4-(difluoromethoxy)benzamido)-1H-imidazole-4-carboxylic acid

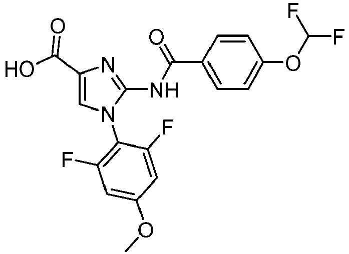

To a stirred solution of compound 38 (130 mg, 0.27 mmol) in THF (1 mL) and MeOH (0.25 mL) was added 1N NaOH solution (2.7 mL, 2.7 mmol). The resulting mixture was stirred at room temperature for 2 h. The reaction was adjusted to pH = 1 by using 1N HCl, and the mixture was extracted with EtOAc (3x). The combined extracts were dried (Na₂SO₄), filtered and evaporated under reduced pressure to afford Intermediate 2, which was used without further purification.

### Example 44: 1-(2,6-Difluoro-4-methoxyphenyl)-2-[4-(difluoromethoxy)benzamido]-N-[(1H-pyrazol-3-yl)methyl]-1H-imidazole-4-carboxamide

To a stirred solution of Intermediate 2 (10 mg, 0.023 mmol) in DMF (0.40 ml) was added BOP (15 mg, 0.034 mmol) followed by DIEA (0.020 mL, 0.11 mmol). The resulting solution was stirred at room temperature for 15 min. To the resulting mixture was added (1*H*-pyrazol-3-yl)methanamine (4 mg, 0.046 mmol) and the reaction was stirred at room temperature for 16 h. The crude material was purified by reverse phase HPLC to afford the title compound (5 mg, 0.009 mmol, 40 % yield). MS(ESI) m/z: 519.1 (M+H)⁺. ¹H NMR (500 MHz, DMSO-d₆) *δ* 8.33 (s, 1H), 7.94 (s, 1H), 7.86 (br d, *J* = 8.5 Hz, 2H), 7.58 (br s, 1H), 7.34 (t, *J* = 73.6 Hz, 1H), 7.26 (br d, *J* = 8.5 Hz, 2H), 6.94 (br d, *J* = 10.4 Hz, 2H), 6.17 (s, 1H), 4.46 (br d, *J* = 5.8 Hz, 2H), 3.79 (s, 3H)
The following Examples in Table 3 were prepared as described for Example 44.

**Table 3**

| | | | | |
|---|---|---|---|---|
| 45 | 4-(Difluoromethoxy)-*N*-[1-(4-methoxyphenyl)-4-(piperazine-1-carbonyl)-1*H-*imidazol-2-yl]benzamide | 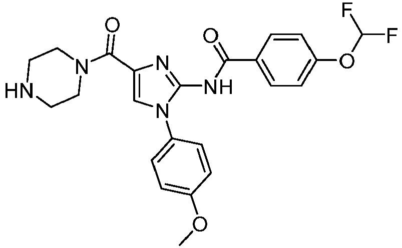 | 472.2 | Method B, Rt = 1.18 min, 98.2% |
| 46 | 2-[4-(Difluoromethoxy)ben zamido]-*N*-(3-hydroxypropyl)-1-(4-methoxyphenyl)-1*H-*imidazole-4-carboxamide | 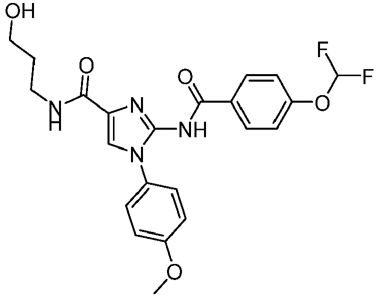 | 461.1 | Method A, Rt = 1.3 min, 96.1% |
| 47 | 1-(2,6-Difluoro-4-methoxyphenyl)-2-[4-(difluoromethoxy)ben zamido]-*N*-[(pyridin-3-yl)methyl]-1*H-*imidazole-4-carboxamide | 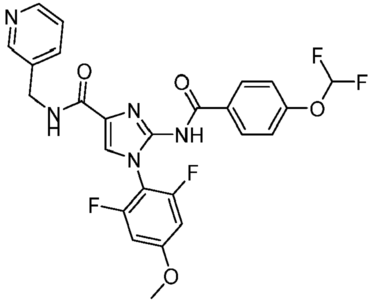 | 530.1 | Method A, Rt = 1.39 min, 97.9% |
| 48 | 1-(2,6-Difluoro-4-methoxyphenyl)-2-[4-(difluoromethoxy)ben zamido]-*N*-[(pyridin-4-yl)methyl]-1*H-*imidazole-4-carboxamide | 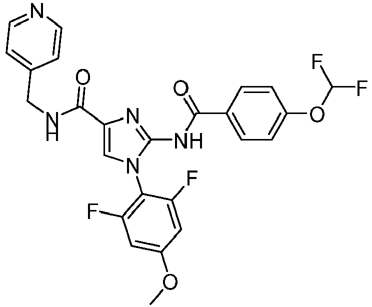 | 530.3 | Method A, Rt = 1.31 min, 97.8% |
| 49 | 1-(2,6-Difluoro-4-methoxyphenyl)-2-[4-(difluoromethoxy)ben zamido]-*N*-(4-hydroxycyclohexyl)-1*H*-imidazole-4-carboxamide | 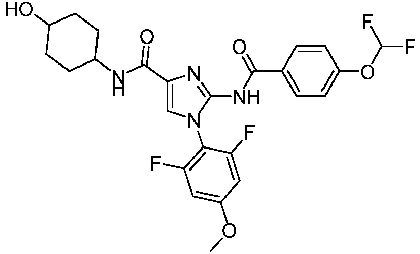 | 537.2 | Method B, Rt = 1.53 min, 100% |
| 50 | *N-*[1-(2,6-Difluoro-4-methoxyphenyl)-4-[(2S)-2-(hydroxymethyl)pyrrol idine-1-carbonyl]-1*H-*imidazol-2-yl]-4-(difluoromethoxy)ben zamide | 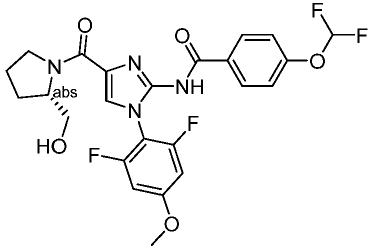 | 523 | Method B, Rt = 1.51 min, 97.6% |
| 51 | *N*-[1-(2,6-Difluoro-4-methoxyphenyl)-4-(3 - hydroxypiperidine-1-carbonyl)-1*H-*imidazol-2-yl]-4-(difluoromethoxy)ben zamide | 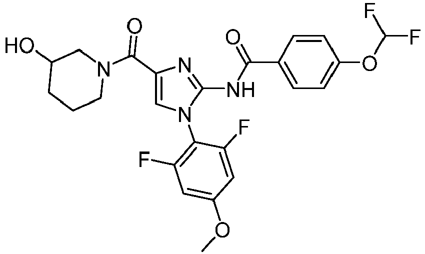 | 523.1 | Method B, Rt = 1.43 min, 97.7% |
| 52 | *N-*[1-(2,6-Difluoro-4-methoxyphenyl)-4-(4-hydroxypiperidine-1-carbonyl)-1*H-*imidazol-2-yl]-4-(difluoromethoxy)ben zamide | 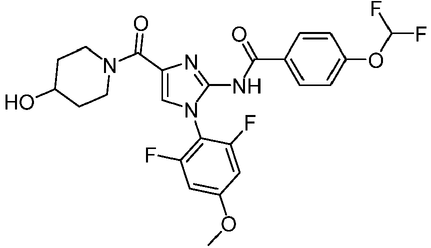 | 522.9 | Method B, Rt = 1.38 min, 95.7% |
| 53 | 1-(2,6-Difluoro-4-methoxyphenyl)-2-[4-(difluoromethoxy)ben zamido]-*N*-(2-hydroxyethyl)-1*H-*imidazole-4-carboxamide | 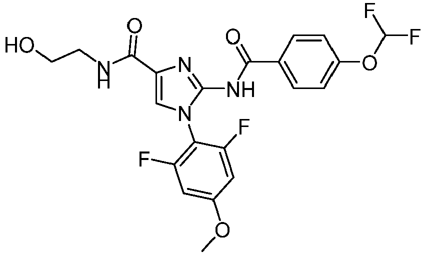 | 483 | Method A, Rt = 1.33 min, 99% |
| 54 | *N-*[1-(2,6-Difluoro-4-methoxyphenyl)-4-(morpholine-4-carbonyl)-1*H-*imidazol-2-yl]-4-(difluoromethoxy)ben zamide | 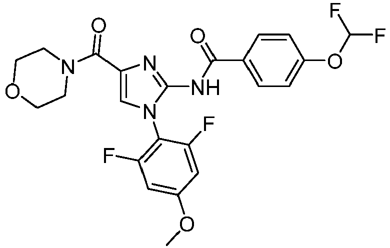 | 509 | Method A, Rt = 1.46 min, 95% |
| 55 | *N-*[1-(2,6-Difluoro-4-methoxyphenyl)-4-(4-methylpiperazine-1-carbonyl)-1*H-*imidazol-2-yl]-4-(difluoromethoxy)ben zamide | 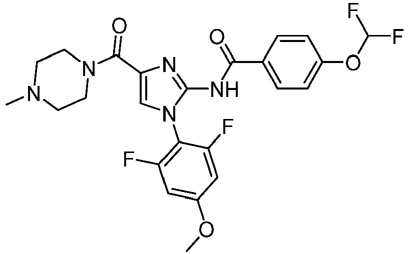 | 522.4 | Method B, Rt = 1.32 min, 96.8% |
| 56 | 1-(2,6-Difluoro-4-methoxyphenyl)-2-[4-(difluoromethoxy)ben zamido]-*N*-[(5-methylpyrazin-2-yl)methyl]-1*H-*imidazole-4-carboxamide | 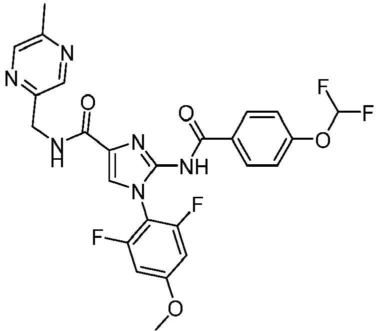 | 545.4 | Method B, Rt = 1.3 min, 95.5% |
| 57 | *N-*[1-(2,6-Difluoro-4-methoxyphenyl)-4-[(3S)-3-hydroxypyrrolidine-1-carbonyl]-1*H-*imidazol-2-yl]-4-(difluoromethoxy)ben zamide | 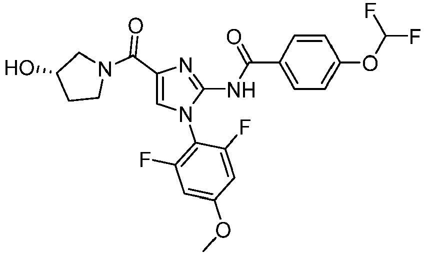 | 509.3 | Method B, Rt = 1.44 min, 97.7% |
| 58 | *N-*[1-(2,6-Difluoro-4-methoxyphenyl)-4-(3 - oxopiperazine-1-carbonyl)-1*H-*imidazol-2-yl]-4-(difluoromethoxy)ben zamide | 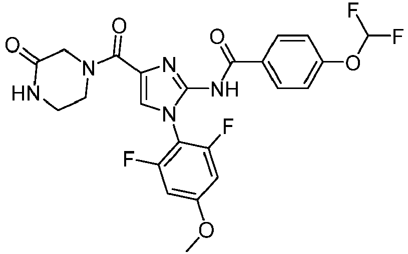 | 522.2 | Method A, Rt = 1.29 min, 98.91% |
| 59 | *N-*[1-(2,6-Difluoro-4-methoxyphenyl)-4-[3-(hydroxymethyl)pyrrol idine-1-carbonyl]-1*H-*imidazol-2-yl]-4-(difluoromethoxy)ben zamide | 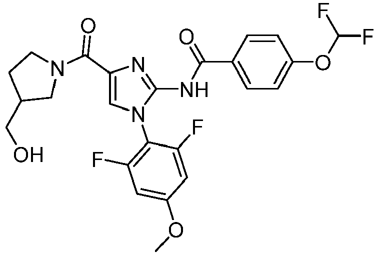 | 523 | Method B, Rt = 1.38 min, 100% |
| 60 | 1-(2,6-Difluoro-4-methoxyphenyl)-2-[4-(difluoromethoxy)ben zamido]-*N*-(3-hydroxypropyl)-1*H-*imidazole-4-carboxamide | 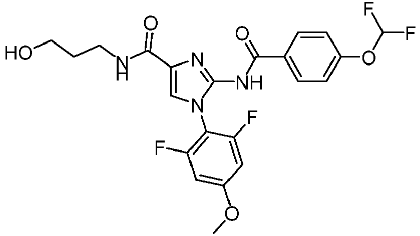 | 497.2 | Method A, Rt = 1.33 min, 99.1% |
| 61 | *N-*[1-(2,6-Difluoro-4-methoxyphenyl)-4-(piperazine-1-carbonyl)-1*H-*imidazol-2-yl]-4-(difluoromethoxy)ben zamide | 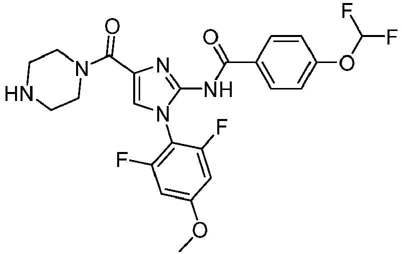 | 508.1 | Method B, Rt = 1.2 min, 96.7% |
| 62 | 1-(2,6-Difluoro-4-methoxyphenyl)-2-[4-(difluoromethoxy)ben zamido]-*N*-(1*H-*pyrazol-4-yl)-1H-imidazole-4-carboxamide | 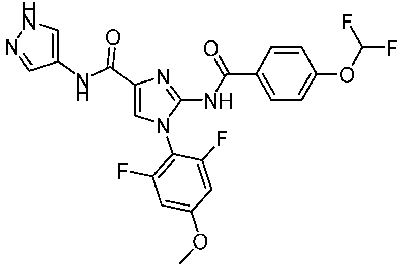 | 505.2 | Method A, Rt = 1.39 min, 95.1% |

### Example 63: N-[1-(2,6-Difluoro-4-methoxyphenyl)-4-(piperidin-4-yl)-1H-imidazol-2-yl]-4-(difluoromethoxy)benzamide

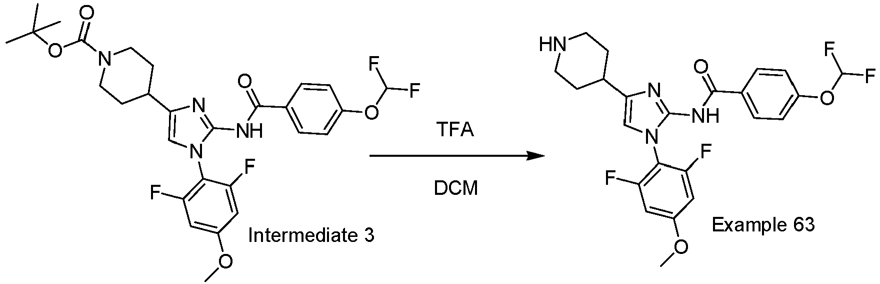

### Intermediate 3: tert-Butyl 4-(1-(2,6-difluoro-4-methoxyphenyl)-2-(4-difluoromethoxy)benzamido)-1H-imidazol-4-yl)piperidine-1-carboxylate

To a solution of Intermediate 3 (prepared as described for Example 1) (29 mg, 0.50 mmol) in DCM (0.5 mL) was added TFA (0.5 mL). The resulting mixture was stirred at room temperature for 16 h. The material was purified by reverse phase HPLC to afford Example 63 (14 mg, 0.29 mmol, 57% yield). MS(ESI) m/z: 479.2 (M+H)⁺. ¹H NMR (500 MHz, DMSO-d₆) *δ* 7.89 (br d, *J* = 8.5 Hz, 2H), 7.29 (t, *J* = 73.9 Hz, 1H), 7.19 (br d, *J* = 8.5 Hz, 2H), 7.00 (s, 1H), 6.95 (br d, *J* = 10.1 Hz, 2H), 3.82 (s, 3H), 3.30 - 3.24 (m, 1H), 3.20 - 3.14 (m, 1H), 3.09 (br d, *J* = 11.9 Hz, 2H), 2.79 - 2.65 (m, 3H), 2.56 (br s, 4H), 2.52 (br s, 6H), 1.97 (br d, *J* = 11.6 Hz, 2H), 1.83 (s, 3H), 1.54 (br d, *J* = 10.4 Hz, 2H), 1.01 (d, *J* = 6.4 Hz, 2H).

### Example 64: 4-(Difluoromethoxy)-N-[1-(4-methoxyphenyl)-4-(piperidin-3-yl)-1H-imidazol-2-yl]benzamide was prepared as described for example 63.

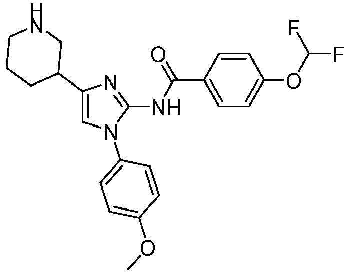

The material was purified by reverse phase HPLC to afford Example 64 (21.3 mg, 0.05 mmol, 87% yield). MS(ESI) m/z: 443.1 (M+H)⁺. ¹H NMR (500 MHz, DMSO-d₆) *δ* 7.95 (br d, *J* = 7.3 Hz, 2H), 7.46 (br s, 2H), 7.31 (t, *J* = 73.8 Hz, 1H), 7.22 (br d, *J* = 8.2 Hz, 2H), 7.17 (br s, 1H), 7.15 (br s, 1H), 7.02 (br d, *J* = 8.2 Hz, 2H), 3.77 (s, 3H), 3.30 (br s, 1H), 3.05 (br d, *J* = 8.9 Hz, 1H), 2.84 (br s, 1H), 2.78 - 2.63 (m, 2H), 2.07 - 2.01 (m, 1H), 1.74 (br s, 1H), 1.58 (br s, 2H).

### Example 65: N-[1-(2,6-Difluoro-4-methoxyphenyl)-4-[1-(ethanesulfonyl)piperidin-4-yl]-1H-imidazol-2-yl]-4-(difluoromethoxy)benzamide

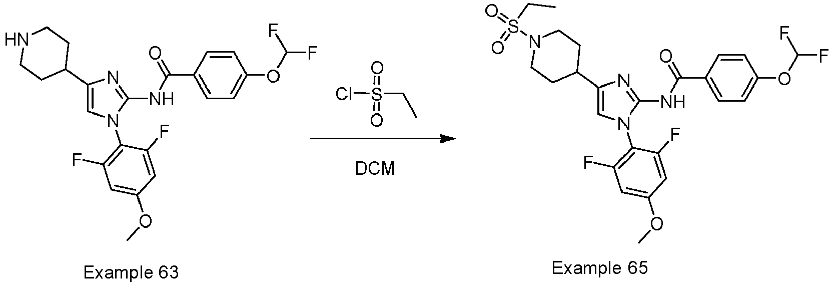

To a solution of Compound 63 (10 mg, 0.021 mmol) in DCM (0.4 ml) was added ethanesulfonyl chloride (16 mg, 0.12 mmol) followed by DIEA (0.027 mL, 0.16 mmol). The resulting solution was stirred at room temperature over night. The crude material was purified by reverse phase HPLC to afford Example 65 (2 mg, 0.003 mmol, 13% yield). MS(ESI) m/z: 571.2 (M+H)+. ¹H NMR (500 MHz, DMSO-d₆) *δ* 7.89 (br s, 2H), 7.30 (s, 2H), 7.20 (s, 2H), 7.10 (s, 1H), 3.81 (br s, 1H), 3.68 (br d, *J* = 11.9 Hz, 2H), 3.08 (q, *J* = 7.3 Hz, 2H), 3.00 (s, 1H), 2.97 - 2.89 (m, 3H), 2.55 (s, 6H), 2.51 (br s, 8H), 2.10 - 2.05 (m, 2H), 1.80 (br d, *J* = 6.4 Hz, 1H), 1.24 (t, *J* = 7.3 Hz, 4H), 1.18 (s, 1H).

### Example 66: 4-[1-(2,6-Difluoro-4-methoxyphenyl)-2-[4-(difluoromethoxy)benzamido]-1H-imidazol-4-yl]-N-ethylpiperidine-1-carboxamide

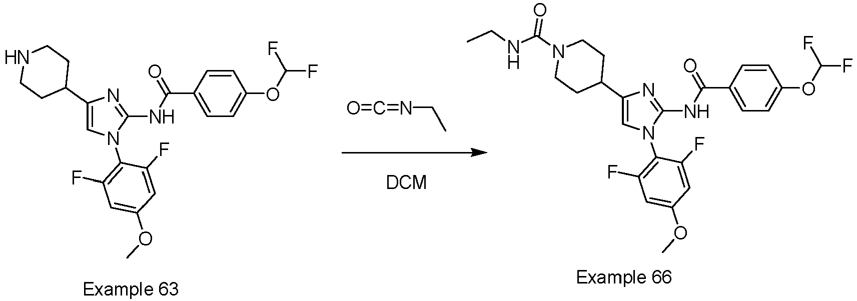

To a solution of Compound 63 (10 mg, 0.021 mmol) in DCM (0.4 mL) was added ethyl isocyanate (13 mg, 0.13 mmol) followed by DIEA (0.027 mL, 0.16 mmol). The resulting solution was stirred at room temperature ON. The crude material was purified by reverse phase HPLC to afford Example 66 (3.0 mg, 0.004 mmol, 15% yield). MS(ESI) m/z: 550.22 (M+H)⁺. ¹H NMR (500 MHz, DMSO-d₆) *δ* 7.90 (br d, *J* = 7.7 Hz, 2H), 7.39 (s, 1H), 7.24 (s, 1H), 7.17 (br d, *J* = 8.0 Hz, 2H), 7.09 (s, 1H), 6.98 - 6.89 (m, 3H), 4.01 (br d, *J* = 13.2 Hz, 2H), 3.89 (s, 1H), 3.83 (br s, 2H), 3.11 - 3.03 (m, 2H), 2.94 (q, *J* = 7.3 Hz, 1H), 2.78 (br t, *J* = 11.9 Hz, 2H), 1.98 - 1.91 (m, 2H), 1.50 - 1.39 (m, 2H), 1.19 (t, *J* = 7.3 Hz, 1H), 1.03 (t, *J* = 7.1 Hz, 3H).

### Example 67: N-{4-[1-(3-Carbamoylpropanoyl)piperidin-4-yl]-1-(2,6-difluoro-4-methoxyphenyl)-1H-imidazol-2-yl}-4-(difluoromethoxy)benzamide

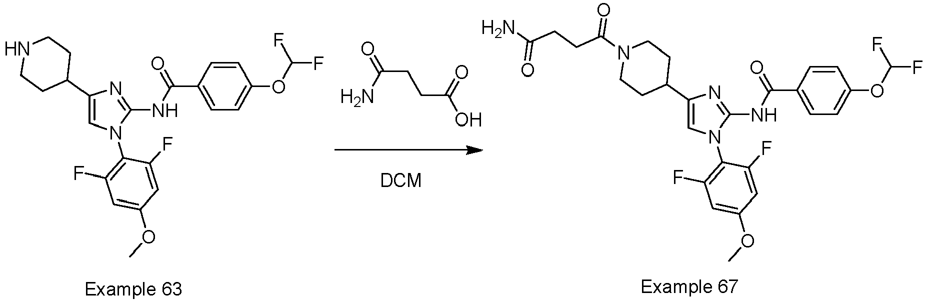

To a solution of 4-amino-4-oxobutanoic acid (5 mg, 0.042 mmol) in DMF (0.2 mL) was added BOP (12 mg, 0.026 mmol) followed by DIEA (0.018 mL, 0.11 mmol). The resulting solution was stirred at room temperature for 10 min. To this mixture was added a solution of Example 63 (10 mg, 0.021 mmol) in DMF (0.2 mL). The reaction was stirred at room temperatureover night. The crude material was purified by reverse phase HPLC to afford Example 67 (1 mg, 0.002 mmol, 9 % yield). MS(ESI) m/z: 578.14 (M+H)⁺. ¹H NMR (500 MHz, DMSO-d₆) *δ* 7.90 (br d, *J* = 8.5 Hz, 2H), 7.45 (s, 1H), 7.29 (br d, *J* = 13.4 Hz, 1H), 7.19 (br d, *J* = 8.5 Hz, 2H), 7.16 (s, 1H), 7.02 (s, 1H), 6.96 (br d, *J* = 10.4 Hz, 2H), 6.71 (br s, 1H), 4.44 (br d, *J*=13.1 Hz, 1H), 3.96 (br d, *J* = 13.1 Hz, 1H), 3.82 (s, 3H), 3.12 (br t, *J* = 12.7 Hz, 1H), 2.92 - 2.83 (m, 1H), 2.67 (br t, *J* = 12.5 Hz, 1H), 2.36 - 2.28 (m, 2H), 2.04 (br d, *J* = 13.1 Hz, 1H), 1.97 (br d, *J* = 11.9 Hz, 1H), 1.90 (s, 2H), 1.58 - 1.48 (m, 1H), 1.45 - 1.35 (m, 1H).
Examples 68 and 69 (Table 3) were prepared as described for example 67.

### Example 70: N-(1-(2,6-Difluoro-4-methoxyphenyl)-4-isopropyl-1H-imidazol-2-yl)-4-(difluoromethoxy)benzamide

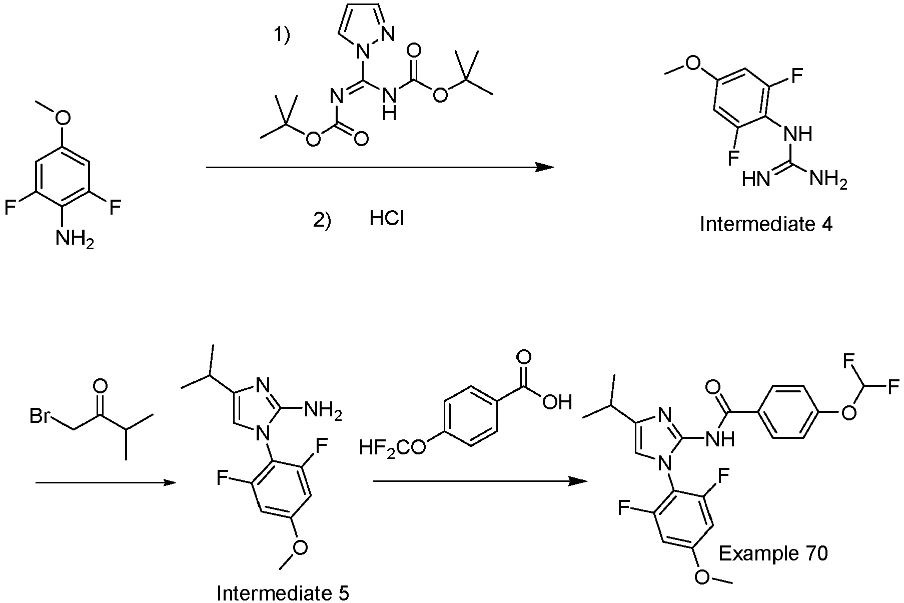

### Intermediate 4: 1-(2,6-Difluoro-4-methoxyphenyl)guanidine

To a solution of *N*,*N*'-di-Boc-1*H*-pyrazole-1-carboxamidine (780 mg, 2.5 mmol) in chloroform (5 mL) was added 2,6-difluoro-4-methoxyaniline (400 mg, 2.5 mmol) and the mixture was stirred at 55 °C for 16 h. Additional *N*,*N*'-di-Boc-1*H*-pyrazole-1-carboxamidine (200 mg) was added and heating continued for an additional 24 h. The mixture was allowed to cool to room temperature and then purified by silica gel chromatography eluting with 2 to 20% EtOAc/hexanes to give di-tert-butyl [(2,6-difluoro-4-methoxyphenyl)carbonimidoyl]biscarbamate (370 mg, 37% yield) as a white solid.

The white solid was then treated with 4N HCl in dioxane (2.3 mL, 9.1 mmol) and the mixture aged for 48 h. The mixture was evaporated, then co-evaporated from ACN followed by diethyl ether to give 1-(2,6-difluoro-4-methoxyphenyl)guanidine, HCl (230 mg, 100 % yield) as a white solid. MS(ESI) m/z: 202.1 (M+H)⁺. ¹H NMR (500 MHz, DMSO-d6) *δ* 9.29 (br s, 1H), 7.57 (br s, 4H), 6.92 (d, *J* = 9.6 Hz, 2H), 3.81 (s, 3H). Intermediate 5: 1-(2,6-Difluoro-4-methoxyphenyl)-4-isopropyl-1*H*-imidazol-2-amine: To a mixture of Intermediate 4 (24 mg, 0.10 mmol) and potassium carbonate (42 mg, 0.30 mmol) in EtOH (0.33 mL) was added 1-bromo-3-methylbutan-2-one (12 µl, 0.10 mmol) and the mixture heated at reflux for 10 min. The mixture was allowed to cool to room temperature and solvent removed under vacuum. The residue was purified by silica gel chromatography eluting with 0.5 to 15% MeOH/DCM to give the title compound (15 mg, 0.06 mmol, 56 % yield) as a white solid. MS(ESI) m/z: 268.1 (M+H)⁺. ¹H NMR (500 MHz, CHLOROFORM-d) *δ* 6.69 - 6.55 (m, 2H), 6.27 (s, 1H), 4.31 (br s, 2H), 3.85 (s, 3H), 2.79 (spt, *J* = 6.7 Hz, 1H), 1.25 (d, *J* = 6.9 Hz, 6H).

### Example 70: N-(1-(2,6-Difluoro-4-methoxyphenyl)-4-isopropyl-1H-imidazol-2-yl)-4-(difluoromethoxy)benzamide)

To a solution of Intermediate 5 (15 mg, 0.06 mmol) and 4-(difluoromethoxy)benzoic acid (16 mg, 0.084 mmol) in DMF (0.5 mL) was added DIPEA (0.035 mL, 0.20 mmol) followed by HATU (38 mg, 0.10 mmol) and the mixture stirred for 16 h. The mixture was diluted with 70% EtOAc/hexanes and washed with saturated NH₄Cl, 1.5M K₂HPO₄ then dried (Na₂SO₄) filtered and concentrated. The residue was purified by silica gel chromatography eluting with 5 to 40% EtOAc/hexanes followed by reverse phase HPLC to afford the title compound (10 mg, 0.022 mmol, 40% yield). MS(ESI) m/z: 438.2 (M+H)⁺. ¹H NMR (500 MHz, DMSO-d₆) *δ* 7.91 (br d, *J* = 8.5 Hz, 2H), 7.30 (t, *J* = 73.6 Hz, 1H), 7.20 (br d, *J* = 8.2 Hz, 2H), 7.05 (s, 1H), 6.98 (br d, *J=* 10.1 Hz, 2H), 3.84 (s, 3H), 2.95 (dt, *J* = 13.8, 6.7 Hz, 1H), 1.25 (d, *J* = 7.0 Hz, 6H)

Examples 71 and 72 (Table 3) was prepared as described for example 70.

### Example 73: N-(1-(2,6-Difluoro-4-methoxyphenyl)-4-((5-methyl-1,3,4-oxadiazol-2-yl)methyl)-1H-imidazol-2-yl)-4-(difluoromethoxy)benzamide

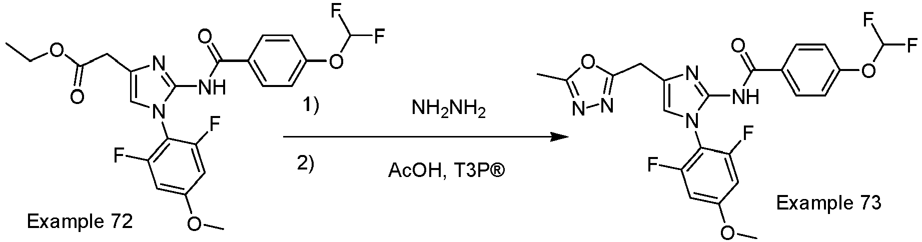

To a solution of compound 72 (prepared as described for Example 70) (30 mg, 0.062 mmol) in DCM (0.6 mL) was added hydrazine (0.020 mL, 0.62 mmol) and the mixture stirred for 16 h. The mixture was evaporated twice from DCM and place under vacuum to give N-(1-(2,6-difluoro-4-methoxyphenyl)-4-(2-hydrazinyl-2-oxoethyl)-1H-imidazol-2-yl)-4-(difluoromethoxy)benzamide (29 mg, 0.062 mmol, 99 % yield) as a white solid. To a solution of the solid (14 mg, 0.030 mmol) and acetic acid (2.1 µl, 0.036 mmol) in dioxane (0.25 mL) was added 1-propanephosphonic acid cyclic anhydride (50% in EtOAc) (0.045 mL, 0.075 mmol) and the reaction heated at 70 °C for 1.5 h. Additional 1-propanephosphonic acid cyclic anhydride (50% in EtOAc) (0.045 mL, 0.075 mmol) was added and the reaction heated at 105 °C for 1.5 h. The reaction was allowed to cool to room temperature, evaporated under a stream of nitrogen and then filtered. The crude material was purified by reverse phase HPLC to afford the title compound (4 mg, 7.4 µmol, 25% yield). MS(ESI) m/z: 492.12 (M+H)+. ¹H NMR (500 MHz, DMSO-d₆) δ 7.82 (br d, *J*=7.6 Hz, 2H), 7.31 (t, *J*=72.9 Hz, 1H), 7.23 (br d, *J*=7.3 Hz, 2H), 7.16 (s, 1H), 6.88 (br d, *J*=11.0 Hz, 2H), 4.16 (br s, 2H), 3.76 (s, 3H), 2.48 (s, 3H).

### Example 74: N-(1-(2,6-Difluoro-4-methoxyphenyl)-4-(3-hydroxycyclobutyl)-1H-imidazol-2-yl)-4-(difluoromethoxy)benzamide.

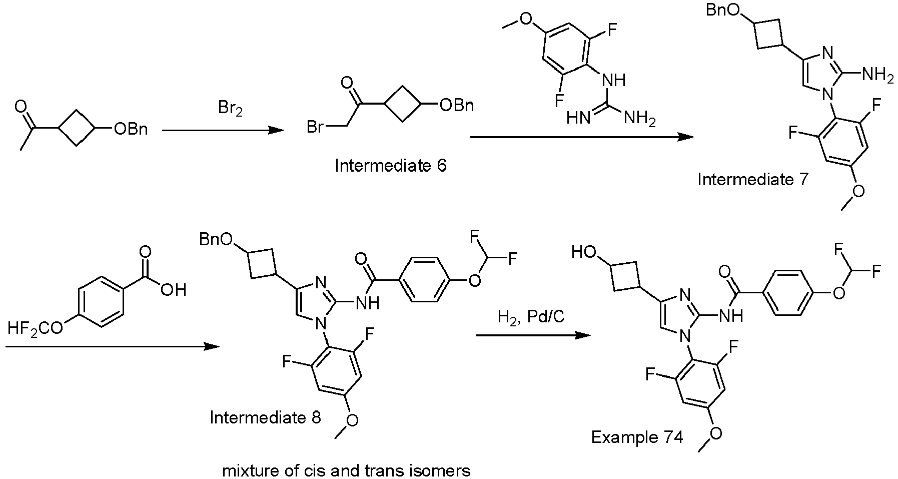

### Intermediate 6: 1-(3-(Benzyloxy)cyclobutyl)-2-bromoethan-1-one

To a solution of 1-(3-(benzyloxy)cyclobutyl)ethan-1-one (430 mg, 2.1 mmol) in EtOH (5.3 mL) at 0 °C was added a solution of bromine (110 µl, 2.1 mmol) in EtOH (1 mL) dropwise. The mixture was stirred at 0 °C for 2 h, warmed to room temperature and stirred for 16 h. The mixture was poured into saturated NaHCO₃ solution and extracted with DCM. The combined organic extracts were washed with brine, dried over Na₂SO₄ and concentrated under reduced pressure. The residue was purified by silica gel flash chromatography eluting with 0 to 100 % EtOAc/hexane to afford intermediate 6 (280 mg, 1.0 mmol, 47% yield) as a mixture of cis and trans isomers which was used directly in the next step.

### Intermediate 7: 4-(3-(Benzyloxy)cyclobutyl)-1-(2,6-difluoro-4-methoxyphenyl)-1H-imidazol-2-amine

To a mixture of Intermediate 4 (210 mg, 0.77 mmol) and potassium carbonate (320 mg, 2.30 mmol) in EtOH (2.6 mL) was added Intermediate 6 (280 mg, 1.0 mmol) and the mixture was stirred at 80 °C for 2.5 h. The mixture was cooled to room temperature, diluted with DCM and filtered. The filtrate was concentrated under reduced pressure, and the residue was purified by silica gel flash chromatography eluting with 0 to 20 %MeOH/DCM to afford Intermediate 7 (270 mg, 0.7 mmol, 91% yield). LCMS (Method B) Rt = 0.77 min, (M+H)⁺ = 386.1.

### Intermediate 8: N-(4-(3-(benzyloxy)cyclobutyl)-1-(2,6-difluoro-4-methoxyphenyl)-1H-imidazol-2-yl)-4-(difluoromethoxy)benzamide

To a solution of Intermediate 7 (270 mg, 0.7 mmol) and 4-(difluoromethoxy)benzoic acid (160 mg, 0.84 mmol) in DMF (2 mL) was added DIPEA (0.37 mL, 2.1 mmol) followed by HATU (400 mg, 1.05 mmol) and the mixture stirred at 80 °C for 3 days. The mixture was diluted with EtOAc, washed with water, 1N HCl and brine, dried over sodium sulfate, filtered and concentrated under reduced pressure. The residue was purified by silica gel flash chromatography eluting with 0 to 100% EtOAc/hexane to afford Intermediate 8 (185 mg, 0.33 mmol, 47 % yield) as a mixture of cis and trans isomers. LCMS (Method B) Rt = 0.93 min, (M+H)⁺ = 556.0.

### Example 74: N-(1-(2,6-difluoro-4-methoxyphenyl)-4-(3-hydroxycyclobutyl)-1H-imidazol-2-yl)-4-(difluoromethoxy)benzamide

To a solution of Intermediate 8 (180 mg, 0.33 mmol) in EtOH (5 mL) was added 10% Pd-C (35 mg, 0.033 mmol). The reaction was stirred under hydrogen atomsphere at room temperature overnight. The mixture was filtered through a pad of Celite and concentrated under reduced pressure. The material was purified by reverse phase HPLC to afford the title compound (7.7 mg, 0.016 mmol, 5% yield). MS(ESI) m/z: 466.1 (M+H)⁺. ¹H NMR (500 MHz, DMSO-d6) δ 8.00 - 7.81 (m, 2H), 7.09 (br s, 5H), 6.95 - 6.82 (m, 1H), 4.51 - 4.32 (m, 1H), 4.31 - 4.20 (m, 1H), 3.94 - 3.86 (m, 3H), 2.45 - 2.28 (m, 2H), 2.29 - 2.14 (m, 2H).

### Example 75: N-(1-(2,6-Difluoro-4-methoxyphenyl)-4-(2-azaspiro[3.3]heptan-6-yl)-1H-imidazol-2-yl)-4-(difluoromethoxy)benzamide

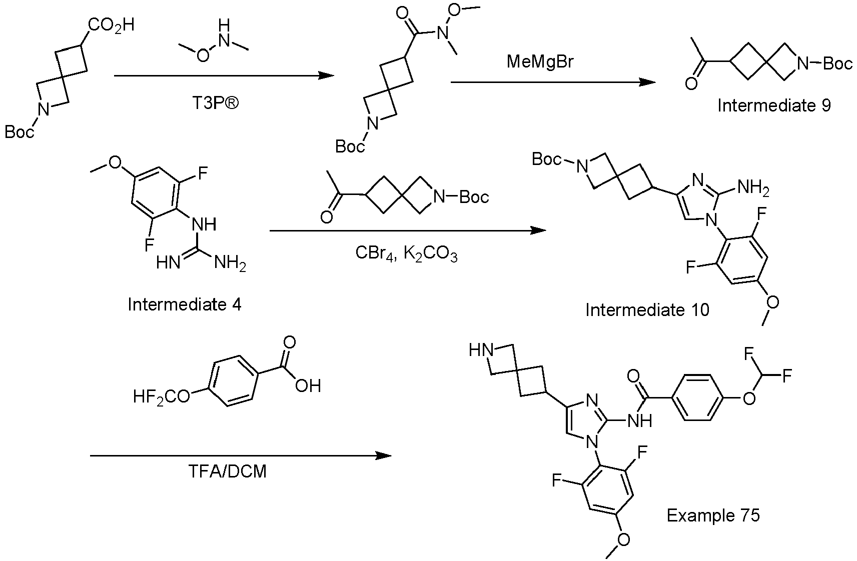

### Intermediate 9: Tert-butyl 6-acetyl-2-azaspiro[3.3]heptane-2-carboxylate

To a stirred solution of N,O-Dimethylhydroxylamine hydrochloride (200 mg, 2.1 mmol) and 2-(tert-butoxycarbonyl)-2-azaspiro[3.3]heptane-6-carboxylic acid (250 mg, 1.04 mmol) and TEA (0.7 mL, 5.18 mmol) in DCM (10 mL) at 0° C was added T3P ( 50% in EtOAc, 1.2 mL, 2.1 mmol) dropwise under argon atmosphere. The reaction mixture was allowed to stir at room temperature for 3 h. The mixture was diluted with EtOAc, washed with water, 1N HCl solution and brine, dried over sodium sulfate and concentrated. The crude product was purified by silica gel column chromatography eluting with 0-100% EtAOc/hexane which was then treated with Methylmagnesiumbromide (3M in THF) (1.1 mL, 3.2 mmol) to afford the title compound (227 mg, 0.95 mmol, 90 % yield)

### Intermediate 10: Tert-butyl 6-(2-amino-1-(2,6-difluoro-4-methoxyphenyl)-1H-imidazol-4-yl)-2-azaspiro[3.3]heptane-2-carboxylate

To a mixture of Intermediate 4 (210 mg, 0.77 mmol) and potassium carbonate (212 mg, 1.532 mmol) in ACN (7.6 mL) was added Intermediate 9 (220 mg, 0.92 mmol) and carbon tetrabromide (300 mg, 0.92 mmol). The mixture was heated at 70 °C overnight. The mixture was diluted with DCM and filtered through Celite. The filtrate was concentrated under reduced pressure, and the residue was purified by reverse phase preparative HPLC to afford Intermediate 10 (67 mg, 0.16 mmol, 21% yield). MS(ESI) m/z: 421.1 (M+H)⁺.

### Example 75: N-(1-(2,6-Difluoro-4-methoxyphenyl)-4-(2-azaspiro[3.3]heptan-6-yl)-1H-imidazol-2-yl)-4-(difluoromethoxy)benzamide

To a solution of Intermediate 10 (67 mg, 0.16 mmol) and 4-(difluoromethoxy)benzoic acid (45 mg, 0.24 mmol) in DMF (2 mL) was added DIPEA (0.083 mL, 0.47 mmol) followed by HATU (91 mg, 0.24 mmol) and the mixture was stirred at 80 °C for 6 h. The reaction mixture was concentrated under reduced pressure. The crude residue was treated with 50% TFA/DCM (1.0 mL) for 30 min. The mixture was concentrated reduced pressure, and the residue was purified reverse phase HPLC to afford the title compound (18 mg, 0.036 mmol, 23% yield). MS(ESI) m/z: 491.2 (M+H)⁺. ¹H NMR (500 MHz, METHANOL-d₄) δ 7.88 (br d, J=8.7 Hz, 2H), 7.23 - 7.15 (m, 2H), 7.04 - 6.72 (m, 4H), 3.85 (s, 3H), 3.51 - 3.41 (m, 1H), 2.79 - 2.70 (m, 2H), 2.58 - 2.50 (m, 2H), 1.50 - 1.26 (m, 4H).

### Example 76: N-(1-(2,6-Difluoro-4-methoxyphenyl)-4-(4-hydroxycyclohexyl)-1H-imidazol-2-yl)-4-(difluoromethoxy)benzamide

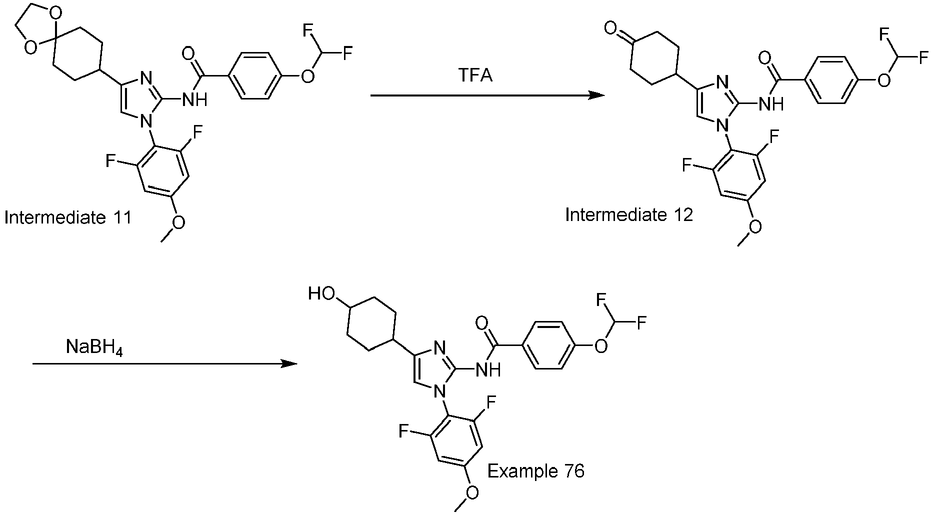

### Intermediate 11: N-(1-(2,6-Difluoro-4-methoxyphenyl)-4-(1,4-dioxaspiro[4.5]decan-8-yl)-1H-imidazol-2-yl)-4-(difluoromethoxy)benzamide

Intermediate 11 was prepared as described in Example 74 from Intermediate 4 and 1-(1,4-dioxaspiro[4.5]decan-8-yl)ethan-1-one to afford Intermediate 11 (390 mg, 0.72 mmol, 63 % yield). LCMS (Method A, Rt = 0.83 min), MS(ESI) m/z: 536.0 (M+H)⁺. ¹H NMR (500 MHz, DMSO-d₆) δ 8.04 - 7.79 (m, 2H), 7.38 - 6.82 (m, 6H), 3.97 - 3.70 (m, 7H), 2.82 - 2.66 (m, 1H), 1.79 - 1.44 (m, 8H).

### Intermediate 12: N-(1-(2,6-Difluoro-4-methoxyphenyl)-4-(4-oxocyclohexyl)-1H-imidazol-2-yl)-4-(difluoromethoxy)benzamide

A mixture of Intermediate 11 (390 mg, 0.73 mmol) and TFA (3 mL) was stirred at room temperature for 3 days. The reaction mixture was concentrated under reduced pressure to give Intermediate 12 (310 mg, 0.64 mmol, 87 % yield). LCMS (Method A, Rt = 0.80 min), MS(ESI) m/z: 492.0 (M+H)⁺.

### Example 76: N-(1-(2,6-Difluoro-4-methoxyphenyl)-4-(4-hydroxycyclohexyl)-1H-imidazol-2-yl)-4-(difluoromethoxy)benzamide

To a solution of Intermediate 12 (40 mg, 0.081 mmol) in MeOH (2 mL) at room temperature was added NaBH₄ (5 mg, 0.13 mmol). The reaction was stirred at room temperature for 2 h. The reaction mixture was filtered through Celite, and the filtrate was concentrated under reduced pressure. The residue was purified by reverse phase HPLC to afford Example 76 (6 mg, 0.011 mmol, 14 % yield). MS(ESI) m/z: 494.03 (M+H)+. ¹H NMR (500 MHz, DMSO-d₆) δ 7.90 (br d, J=8.6 Hz, 2H), 7.39 - 7.03 (m, 3H), 6.95 - 6.84 (m, 3H), 3.83 (s, 3H), 3.71 - 3.59 (m, 1H), 2.71 - 2.64 (m, 1H), 1.85 - 1.76 (m, 2H), 1.74 - 1.63 (m, 4H), 1.62 - 1.52 (m, 2H).

### Example 77: N-(1-(2,6-Difluoro-4-methoxyphenyl)-4-((6-fluoro-1H-imidazo[4,5-b]pyridin-2-yl)methyl)-1H-imidazol-2-yl)-4-(difluoromethoxy)benzamide

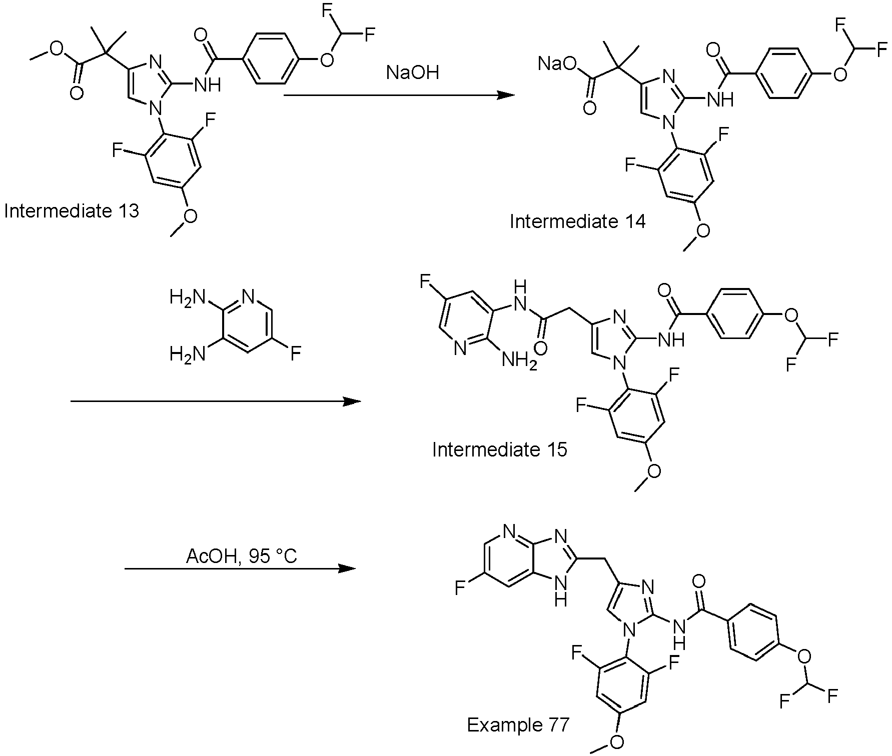

### Intermediate 13: Methyl 2-(1-(2,6-difluoro-4-methoxyphenyl)-2-(4-(difluoromethoxy)benzamido) -1H-imidazol-4-yl)-2-methylpropanoate

Intermediate 13 was prepared as described for Example 74.

### Intermediate 14: 2-(1-(2,6-Difluoro-4-methoxyphenyl)-2-(4-(difluoromethoxy)benzamido) -1H-imidazol-4-yl)-2-methylpropanoate, Na salt

To a stirred solution of Intermediate 13 (210 mg, 0.42 mmol) in THF/MeOH (3:1, 4 mL) was added 1N NaOH (0.85 mL, 0.85 mmol). The mixture was stirred at room temperature for 3 d. The mixture was concentrated and used directly in next step.

### Intermediate 15: N-(4-(2-((2-Amino-5-fluoropyridin-3-yl)amino)-2-oxoethyl)-1-(2,6-difluoro-4-methoxyphenyl)-1H-imidazol-2-yl)-4-(difluoromethoxy)benzamide

A mixture of Intermediate 14 (47 mg, 0.1 mmol), 5-fluoropyridine-2,3-diamine (16 mg, 0.13 mmol), HATU (49 mg, 0.13 mmol) and DIPEA (0.052 mL, 0.30 mmol) in ACN (2 mL) was stirred at room temperature overnight. The crude material was diluted with DCM, washed with brine, dried and concentrated. The residue was purified by flash chromatography eluting with 0 to 100% EtOAc to give Intermediate 14 (12 mg, 0.021 mmol, 22% yield).

### Example 77: N-(1-(2,6-Difluoro-4-methoxyphenyl)-4-((6-fluoro-1H-imidazo[4,5-b]pyridin-2-yl)methyl)-1H-imidazol-2-yl)-4-(difluoromethoxy)benzamide

A mixture of Intermediate 15 (12 mg, 0.021 mmol) and AcOH (1 mL) was heated at 95 °C for 3 h. The mixture was cooled to room temperature, diluted with DMF and purified by reverse phase HPLC to afford the title compound (3 mg, 0.005 mmol, 5% yield). MS(ESI) m/z: 545.2 (M+H)⁺. ¹H NMR (500 MHz, CD₃OD) δ 8.23 (br s, 1H), 7.84 (br d, *J*=7.8 Hz, 2H), 7.70 (br d, *J*=8.5 Hz, 1H), 7.33 - 6.63 (m, 6H), 4.31 (s, 2H), 3.82 (s, 3H).

Examples 78-81 (Table 3) were prepared as described in Example 75.

### Example 82: N-(1-(2,6-Difluoro-4-methoxyphenyl)-4-(3-((2,2,2-trifluoroethyl)amino)cyclobutyl)-1H-imidazol-2-yl)-4-(difluoromethoxy)benzamide

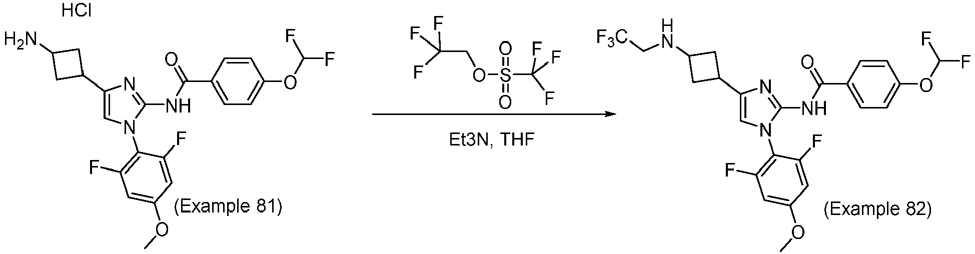

To a solution of Example 81 (20 mg, 0.040 mmol) in THF (0.4 mL) and TEA (56 µl, 0.40 mmol) was added 2,2,2-trifluoroethyl trifluoromethanesulfonate (23 mg, 0.10 mmol). The resulting solution was stirred at room temperature for 4 h. The reaction mixture was diluted with DMF and purified by reverse phase HPLC to afford Example 82 (16 mg, 0.028 mmol, 70% yield). MS(ESI) m/z: 547.3 (M+H)⁺. ¹H NMR (500 MHz, CD₃OD) δ 8.05 - 7.76 (m, 2H), 7.21 - 7.08 (m, 2H), 7.05 - 6.63 (m, 4H), 4.24 - 4.03 (m, 1H), 3.88 - 3.81 (m, 3H), 3.52 - 3.43 (m, 1H), 2.91 (s, 3H), 2.63 - 2.54 (m, 2H), 2.49 (br s, 2H).

### Example 83: N-[1-(2,6-Ddifluoro-4-methoxyphenyl)-4-(3-methanesulfonamidocyclobutyl)-1H-imidazol-2-yl]-4-(difluoromethoxy)benzamide (Table 3) was prepared as described in Example 82.

The following Examples in Table 4 were synthesized according to the procedures described above.

**Table 4**

| Ex. No. | Name | Structure | Obs Mass | Method Rt Purity |
|---|---|---|---|---|
| 68 | N-[1-(2,6-difluoro-4-methoxyphenyl)-4-[1-(3-hydroxypropanoy l)piperidin-4-yl]-1H-imidazol-2-yl]-4-(difluoromethoxy )benzamide | 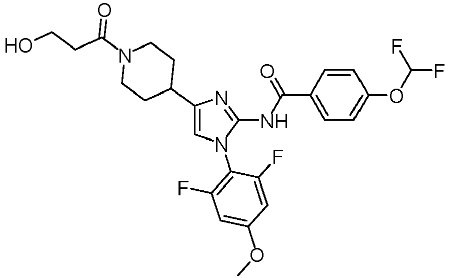 | 551.3 | Method A, Rt = 1.3 min, 100% |
| 69 | N-[1-(2,6-difluoro-4-methoxyphenyl)-4-[4-(hydroxymethyl)p iperidine-1-carbonyl]-1H-imidazol-2-yl]-4-(difluoromethoxy )benzamide | 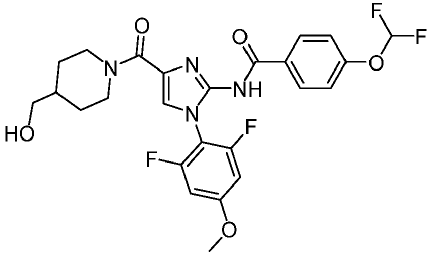 | 537 | Method A, Rt = 1.4 min, 98 % |
| 71 | N-[4-benzyl-1-(2,6-difluoro-4-methoxyphenyl)-1H-imidazol-2-yl]-4-(difluoromethoxy )benzamide | 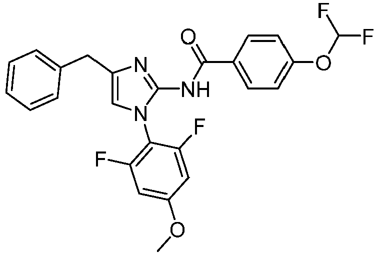 | 486 | Method B, Rt = 2.1 min, 97% |
| 72 | ethyl 2-[1-(2,6-difluoro-4-methoxyphenyl)-2-[4-(difluoromethoxy )benzamido]-1H-imidazol-4-yl] acetate | 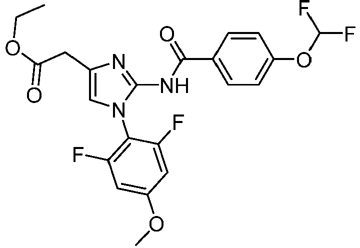 | 482 | Method A, Rt = 0.84 min, 100% |
| 78 | N-[1-(2,6-difluoro-4-methoxyphenyl)-4-[(pyridin-2-yl)methyl]-1H-imidazol-2-yl]-4-(difluoromethoxy )benzamide | 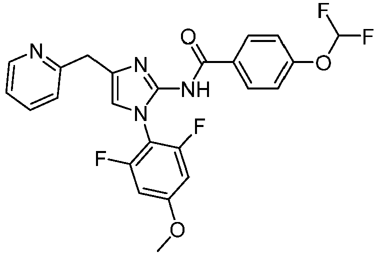 | 487.2 | Method A, Rt = 1.6 min, 97% |
| 79 | N-[4-(azetidin-3-yl)-1-(2,6-difluoro-4-methoxyphenyl)-1H-imidazol-2-yl]-4-(difluoromethoxy )benzamide | 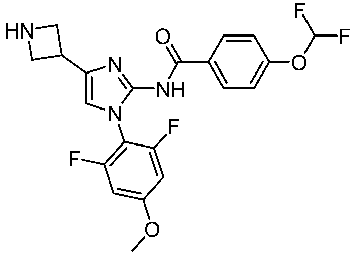 | 451 | Method A, Rt = 1.4 min, 99% |
| 80 | N-[1-(2,6-difluoro-4-methoxyphenyl)-4-(1H-pyrazol-5-yl)-1H-imidazol-2-yl]-4-(difluoromethoxy )benzamide | 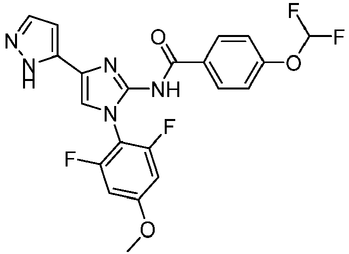 | 462.1 | Method A, Rt = 0.81 min, 100% |
| 81 | N-[4-(3-aminocyclobutyl) -1-(2,6-difluoro-4-methoxyphenyl)-1H-imidazol-2-yl]-4-(difluoromethoxy )benzamide | 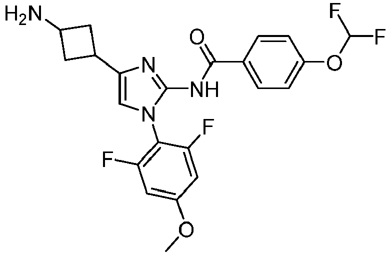 | 465.1 | Method A, Rt = 1.3 min, 100% |
| 83 | N-[1-(2,6-difluoro-4-methoxyphenyl)-4-(3-methanesulfonam idocyclobutyl)-1H-imidazol-2-yl]-4-(difluoromethoxy )benzamide | 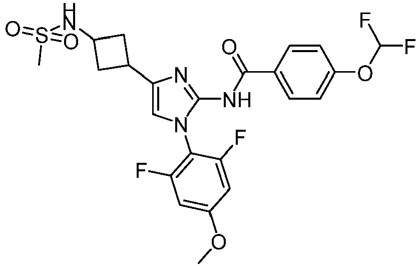 | 543.3 | Method A, Rt = 1.6 min, 97% |

### NMR data for Examples in Tables:

Example 2: ¹H NMR (500 MHz, DMSO-d₆) δ 7.95 - 7.87 (m, 3H), 7.81 (br d, *J*=7.2 Hz, 2H), 7.39 (br d, *J*=7.4 Hz, 4H), 7.32 (t, *J*=73.4 Hz, 1H), 7.29 - 7.20 (m, 3H), 7.00 (br d, *J*=8.1 Hz, 2H), 3.74 (br s, 3H).
Example 3: ¹H NMR (500 MHz, DMSO-d₆) δ 7.89 (s, 1H), 7.84 (br d, *J*=8.0 Hz, 2H), 7.39 - 7.34 (m, 2H), 7.33 - 7.27 (m, 2H), 7.24 (br d, *J*=8.2 Hz, 2H), 6.90 (br d, *J*=10.4 Hz, 2H), 6.82 (br d, *J*=7.2 Hz, 1H), 3.70 - 3.67 (m, 6H)
Example 4: ¹H NMR (500 MHz, DMSO-d₆) δ 7.88 - 7.78 (m, 5H), 7.27 (t, *J*=73.5 Hz, 1H), 7.25 - 7.19 (m, 4H), 6.89 (br d, *J*=10.4 Hz, 2H), 3.77 (s, 3H).
Example 5: ¹H NMR (500 MHz, DMSO-d₆) δ 10.91 (br s, 1H), 7.91 - 7.81 (m, 3H), 7.77 (s, 1H), 7.62 (br d, *J*=8.3 Hz, 1H), 7.41 (br d, *J*=8.3 Hz, 1H), 7.30 (t, *J*=73.5 Hz, 1H), 7.24 (br d, *J*=8.3 Hz, 2H), 6.90 (br d, *J*=10.3 Hz, 2H), 3.75 (br s, 3H), 2.35 (s, 3H).
Example 6: ¹H NMR (500 MHz, DMSO-d₆) δ 8.07 (br s, 1H), 8.00 (br d, *J*=8.0 Hz, 2H), 7.85 (br d, *J*=8.2 Hz, 2H), 7.75 (br d, *J*=8.0 Hz, 2H), 7.30 (t, *J*=73.5 Hz, 1H), 7.24 (br d, *J*=8.5 Hz, 2H), 6.92 (br d, *J*=10.4 Hz, 2H), 3.76 (s, 3H).
Example 7: ¹H NMR (500 MHz, DMSO-d₆) δ 7.92 - 7.78 (m, 5H), 7.36 - 7.06 (m, 1H), 7.30 (t, *J*=73.5 Hz, 1H), 7.22 (br dd, *J*=16.1, 8.3 Hz, 4H), 6.90 (br d, *J*=10.2 Hz, 2H), 3.76 (br s, 3H).
Example 8: ¹H NMR (500 MHz, DMSO-d₆) δ 7.82 (br d, *J*=7.5 Hz, 2H), 7.68 (br d, *J*=7.1 Hz, 2H), 7.49 - 7.39 (m, 2H), 7.32 - 7.11 (m, 4H), 6.93 (br d, *J*=9.9 Hz, 2H), 3.76 (br s, 3H), 2.21 (br s, 3H).
Example 9: ¹H NMR (500 MHz, DMSO-d₆) δ 8.32 (s, 1H), 8.01 (br s, 1H), 7.97 - 7.82 (m, 6H), 7.58 - 7.40 (m, 2H), 7.31 (t, *J*=73.4 Hz, 1H), 7.25 (br d, *J*=8.2 Hz, 2H), 6.93 (br d, *J*=10.1 Hz, 2H), 3.77 (br s, 3H).
Example 10: ¹H NMR (500 MHz, DMSO-d₆) δ 7.95 (s, 1H), 7.83 (br d, *J*=8.1 Hz, 2H), 7.63 (br d, *J*=7.7 Hz, 1H), 7.56 (br d, *J*=10.3 Hz, 1H), 7.47 - 7.39 (m, 1H), 7.27 (t, *J*=73.2 Hz, 1H), 7.23 (br d, *J*=8.4 Hz, 2H), 7.07 (br t, *J*=7.4 Hz, 1H), 6.89 (br d, *J*=10.4 Hz, 2H), 3.77 (s, 3H).
Example 11: ¹H NMR (500 MHz, DMSO-d₆) δ 7.89 (br s, 1H), 7.81 (br dd, *J*=14.7, 8.4 Hz, 4H), 7.48 - 7.40 (m, 2H), 7.27 (t, *J*=73.5 Hz, 1H), 7.23 (br d, *J*=8.3 Hz, 2H), 6.89 (br d, *J*=10.3 Hz, 2H), 3.77 (br s, 3H).
Example 12: ¹H NMR (500 MHz, DMSO-d₆) δ 7.99 (br s, 1H), 7.87 - 7.80 (m, 3H), 7.75 (br d, *J*=7.4 Hz, 1H), 7.43 (br t, *J*=7.7 Hz, 1H), 7.34 - 7.09 (m, 4H), 6.91 (br d, *J=10.4 Hz, 2H), 3.76 (br s, 3H).*
Example 13: ¹H NMR (500 MHz, DMSO-d₆) δ 7.82 (br d, *J*=7.7 Hz, 2H), 7.71 (br d, *J*=8.0 Hz, 2H), 7.48 (br d, *J*=8.0 Hz, 2H), 7.29 (t, *J*=73.2 Hz, 1H), 7.22 (br d, *J*=8.1 Hz, 2H), 6.93 (br d, *J*=9.9 Hz, 2H), 3.76 (br s, 3H), 2.21 (br s, 3H).
Example 14: ¹H NMR (500 MHz, DMSO-d₆) δ 7.87 (br d, *J*=7.6 Hz, 2H), 7.75 - 7.71 (m, 4H), 7.33 (t, *J*=73.6 Hz, 1H), 7.25 (br d, *J*=7.9 Hz, 2H), 6.98 (br d, *J*=8.2 Hz, 2H), 6.92 (br d, *J*=9.8 Hz, 2H), 3.78 (s, 6H).
Example 15: ¹H NMR (500 MHz, DMSO-d₆) δ 7.91 - 7.78 (m, 3H), 7.69 (br d, *J*=7.7 Hz, 2H), 7.33 (t, *J*=73.3 Hz, 1H), 7.23 (br dd, *J*=18.6, 8.0 Hz, 4H), 6.92 (br d, *J*=10.3 Hz, 2H), 3.77 (s, 3H), 2.31 (s, 3H).
Example 16: ¹H NMR (500 MHz, DMSO-d₆) δ 8.10 (br s, 1H), 7.97 (br d, *J*=7.9 Hz, 2H), 7.83 (br d, *J*=7.3 Hz, 4H), 7.28 (t, *J*=73.5 Hz, 1H), 7.23 (br d, *J*=6.4 Hz, 2H), 6.91 (br d, *J*=10.4 Hz, 2H), 3.76 (br s, 3H).
Example 17: ¹H NMR (500 MHz, DMSO-d₆) δ 8.19 (s, 1H), 8.13 (br d, *J*=8.2 Hz, 1H), 8.06 (s, 1H), 7.85 (br d, *J*=8.5 Hz, 2H), 7.70 (br d, *J*=7.6 Hz, 1H), 7.65 - 7.59 (m, 1H), 7.30 (t, *J*=73.6 Hz, 1H), 7.24 (br d, *J*=8.5 Hz, 2H), 6.92 (br d, *J*=10.1 Hz, 2H), 3.77 (s, 3H).
Example 18: ¹H NMR (500 MHz, DMSO-d₆) δ 8.02 (br s, 2H), 7.85 (br d, *J*=7.4 Hz, 2H), 7.77 (br d, *J*=6.6 Hz, 1H), 7.65 (br d, *J*=7.9 Hz, 1H), 7.30 (t, *J*=73.5 Hz, 1H), 7.23 (br d, *J*=6.7 Hz, 2H), 6.92 (br d, *J*=10.1 Hz, 2H), 3.76 (br s, 3H).
Example 19: ¹H NMR (500 MHz, DMSO-d₆) δ 10.53 (s, 1H), 7.90 (br d, *J*=7.0 Hz, 2H), 7.75 (s, 1H), 7.68 (br s, 1H), 7.55 (br d, *J*=7.6 Hz, 1H), 7.43 - 7.42 (m, 1H), 7.38 (br d, *J*=7.6 Hz, 1H), 7.35 (t, *J*=73.4 Hz, 1H), 7.27 (br d, *J*=7.6 Hz, 2H), 7.00 (br d, *J*=8.2 Hz, 2H), 6.77 (br d, *J*=8.2 Hz, 1H), 4.54 (br t, *J*=8.2 Hz, 2H), 3.75 (br s, 3H), 3.20 (br t, *J*=8.2 Hz, 2H).
Example 20: ¹H NMR (500 MHz, DMSO-d₆) δ 8.38 (d, *J*=5.2 Hz, 1H), 8.34 (s, 1H), 7.90 (br d, *J*=8.5 Hz, 2H), 7.84 (s, 1H), 7.78 (br d, *J*=4.6 Hz, 1H), 7.41 (br d, *J*=8.5 Hz, 2H), 7.34 (t, *J*=73.4 Hz, 1H), 7.27 (br d, *J*=8.5 Hz, 2H), 7.02 (br d, *J*=8.5 Hz, 2H), 3.75 (s, 3H).
Example 21: ¹H NMR (500 MHz, DMSO-d₆) δ 10.64 (s, 1H), 8.13 - 7.99 (m, 1H), 7.91 (br d, *J*=8.5 Hz, 2H), 7.68 (d, *J*=3.4 Hz, 1H), 7.41 (br d, *J*=8.9 Hz, 1H), 7.36 (t, *J*=73.6 Hz, 1H), 7.33 (br s, 1H), 7.32 (br s, 1H), 7.28 (br d, *J*=8.5 Hz, 2H), 7.17 (br t, *J*=7.6 Hz, 1H), 7.01 (br d, *J*=8.5 Hz, 2H), 3.75 (s, 3H).
Example 22: ¹H NMR (500 MHz, DMSO-d₆) δ 8.34 (s, 1H), 8.05 - 7.97 (m, 2H), 7.97 - 7.91 (m, 2H), 7.85 (br d, *J*=7.9 Hz, 1H), 7.76 (br d, *J*=5.2 Hz, 1H), 7.58 - 7.40 (m, 3H), 7.36 - 7.12 (m, 3H), 7.14 (s, 1H), 7.07 - 7.00 (m, 2H), 3.77 (s, 3H).
Example 23: ¹H NMR (500 MHz, DMSO-d₆) δ 8.22 (s, 1H), 8.10 (d, *J*=7.6 Hz, 1H), 7.96 (br d, *J*=7.9 Hz, 1H), 7.91 (br d, *J*=8.5 Hz, 2H), 7.54 - 7.39 (m, 5H), 7.34 (t, *J*=73.6 Hz, 1H), 7.28 (br d, *J*=8.5 Hz, 2H), 7.02 (br d, *J*=8.5 Hz, 2H), 3.75 (s, 3H).
Example 24: ¹H NMR (500 MHz, DMSO-d₆) δ 7.96 (s, 1H), 7.90 (br d, *J*=8.5 Hz, 2H), 7.83 (d, *J*=3.1 Hz, 1H), 7.64 (d, *J*=3.1 Hz, 1H), 7.42 (br d, *J*=8.5 Hz, 2H), 7.35 (t, *J*=73.6 Hz, 1H), 7.28 (br d, *J*=8.2 Hz, 2H), 7.01 (br d, *J*=8.9 Hz, 2H), 3.75 (s, 3H).
Example 25: ¹H NMR (500 MHz, DMSO-d₆) δ 10.64 (s, 1H), 8.05 (br t, *J*=6.7 Hz, 1H), 7.91 (br d, J=8.2 Hz, 2H), 7.69 (br d, *J*=3.1 Hz, 1H), 7.42 (br d, J=8.9 Hz, 2H), 7.35 (t, *J*=73.5 Hz, 1H), 7.28 (br d, *J*=7.9 Hz, 5H), 7.01 (br d, *J*=8.9 Hz, 2H), 3.75 (s, 3H).
Example 26: ¹H NMR (500 MHz, DMSO-d₆) δ 7.97 (s, 1H), 7.90 (br d, *J*=8.5 Hz, 2H), 7.71 (d, *J*=1.5 Hz, 1H), 7.60 (d, *J*=8.9 Hz, 1H), 7.43 (br d, *J*=8.5 Hz, 2H), 7.34 (t, *J*=73.6 Hz, 1H), 7.32 - 7.25 (m, 3H), 7.07 (s, 1H), 7.02 (br d, *J*=8.5 Hz, 2H), 3.75 (s, 3H).
Example 27: ¹H NMR (500 MHz, DMSO-d₆) δ 8.13 - 7.85 (m, 3H), 7.65 (br s, 1H), 7.31 - 6.99 (m, 6H), 3.85 (s, 3H), 2.72 - 2.61 (m, 1H), 2.00 (br s, 2H), 1.77 (br s, 2H), 1.69 (br d, *J*=1.3 Hz, 1H), 1.36 (br t, *J*=9.6 Hz, 4H), 1.23 (br s, 1H).
Example 29: ¹H NMR (500 MHz, DMSO-d₆) δ 8.21 (s, 1H), 7.89 (br d, *J*=8.5 Hz, 2H), 7.41 (br d, *J*=8.5 Hz, 2H), 7.33 (t, *J*=73.6 Hz, 1H), 7.27 (br d, *J*=8.5 Hz, 2H), 7.02 (br d, *J*=8.5 Hz, 3H), 4.39 (q, *J*=6.9 Hz, 2H), 3.75 (s, 3H), 1.33 (t, *J*=7.2 Hz, 3H).
Example 30: ¹H NMR (500 MHz, DMSO-d₆) δ 10.67 (s, 1H), 8.10 (s, 1H), 7.88 (br d, J=8.5 Hz, 2H), 7.39 (br d, *J*=8.9 Hz, 2H), 7.35 (t, *J*=73.6 Hz, 1H), 7.27 (br d, J=8.5 Hz, 2H), 7.00 (br d, *J*=8.9 Hz, 2H), 4.26 (q, *J*=7.0 Hz, 2H), 3.74 (s, 3H), 1.29 (t, *J*=7.0 Hz, 3H).
Example 32: ¹H NMR (500 MHz, DMSO-d₆) δ 8.05 (br d, *J*=11.1 Hz, 1H), 7.88 (br d, *J*=4.5 Hz, 1H), 7.74 - 7.61 (m, 1H), 7.49 - 6.91 (m, 7H), 3.90 - 3.67 (m, 3H), 1.33 - 1.23 (m, 9H).
Example 33: ¹H NMR (500 MHz, DMSO-d₆) δ 10.82 (s, 1H), 8.05 (br t, J=6.9 Hz, 1H), 7.87 (br d, J=8.6 Hz, 2H), 7.65 (br d, *J* = 3.3 Hz, 1H), 7.36 - 7.20 (m, 5H), 7.29 (t, *J* = 73.6 Hz, 1H), 6.89 (br d, *J* = 10.1 Hz, 2H), 3.79 (s, 3H).
Example 34: ¹H NMR (500 MHz, DMSO-d₆) δ 8.39 (d, *J* = 5.2 Hz, 1H), 8.24 (s, 1H), 7.86 (br d, *J* = 8.7 Hz, 2H), 7.82 (s, 1H), 7.76 (d, *J* = 5.1 Hz, 1H), 7.28 (t, *J* = 73.5 Hz, 1H), 7.24 (d, *J* = 8.6 Hz, 2H), 6.91 (br d, *J* = 10.0 Hz, 2H), 3.79 (s, 3H).
Example 36: ¹H NMR (500 MHz, DMSO-d₆) δ 7.92 - 7.84 (m, 3H), 7.82 - 7.74 (m, 1H), 7.65 (br s, 1H), 7.50 - 7.41 (m, 1H), 7.29 (t, *J* = 73.5 Hz, 1H), 7.25 (br d, *J* = 8.6 Hz, 2H), 6.91 (br d, *J* = 10.1 Hz, 2H), 3.80 (s, 3H).
Example 37: ¹H NMR (500 MHz, DMSO-d₆) δ 7.96 - 7.78 (m, 2H), 7.24 - 7.10 (m, 3H), 7.10 - 6.99 (m, 2H), 6.87 (br s, 1H), 3.92 - 3.70 (m, 3H), 2.04 - 1.77 (m, 1H), 0.82 (br s, 2H), 0.69 (br s, 2H).
Example 38: ¹H NMR (500 MHz, DMSO-d₆) δ 8.04 (s, 1H), 7.84 (d, *J* = 8.7 Hz, 2H), 7.26 (t, *J* = 73.6 Hz, 1H), 7.22 (br d, *J* = 8.6 Hz, 2H), 6.88 (br d, *J* = 10.2 Hz, 2H), 4.28 (q, *J* = 7.1 Hz, 2H), 3.79 (s, 3H), 1.30 (t, *J* =7.1 Hz, 3H).
Example 39: ¹H NMR (500 MHz, DMSO-d₆) δ 8.03 - 7.80 (m, 2H), 7.36 (br s, 1H), 7.27 - 7.06 (m, 3H), 7.04 - 6.80 (m, 3H), 3.89 (br d, *J* = 4.5 Hz, 3H), 2.61 (br s, 2H), 1.22 (t, *J* = 7.5 Hz, 3H).
Example 40: ¹H NMR (500 MHz, DMSO-d₆) δ 7.98 (s, 1H), 7.85 (br d, *J* = 8.2 Hz, 3H), 7.66 (br s, 1H), 7.31 (t, *J* = 73.5 Hz, 1H), 7.25 (br d, *J* = 8.5 Hz, 2H), 6.92 (br d, *J =* 10.1 Hz, 2H), 3.77 (s, 3H).
Example 42: ¹H NMR (500 MHz, DMSO-d₆) δ 8.11 (s, 1H), 7.88 (br d, *J* = 8.7 Hz, 2H), 7.40 (br d, *J* = 8.8 Hz, 2H), 7.31 (t, *J* = 73.5 Hz, 1H), 7.26 (br d, *J* = 8.7 Hz, 2H), 7.01 (br d, *J* = 8.9 Hz, 2H), 3.76 (s, 3H).
Example 43: ¹H NMR (500 MHz, DMSO-d₆) δ 8.13 (s, 1H), 7.87 (br d, *J* = 8.5 Hz, 2H), 7.39 (d, *J* = 8.9 Hz, 2H), 7.34 (t, *J* = 73.4 Hz,1H),7.27 (br d, *J* = 8.5 Hz, 2H), 7.01 (d, *J=* 8.9 Hz, 2H), 3.74 (s, 3H).
Example 45: ¹H NMR (500 MHz, DMSO-d₆) δ 7.89 (br d, *J* = 7.9 Hz, 2H), 7.82 (s, 1H), 7.39 (br d, *J* = 8.9 Hz, 2H), 7.35 (t, *J*=73.4 Hz, 1H), 7.26 (br d, *J* = 8.5 Hz, 2H), 7.01 (s, 2H), 3.89 (s, 1H), 3.74 (s, 3H), 3.17 (s, 1H), 2.89 (s, 1H), 2.73 (br d, *J* = 6.4 Hz, 5H).
Example 46: ¹H NMR (500 MHz, DMSO-d₆) δ 8.11 (br s, 1H), 7.89 (br d, *J* = 8.2 Hz, 2H), 7.85 (s, 1H), 7.39 (br d, *J* = 8.5 Hz, 2H), 7.35 (t, *J* = 73.4 Hz, 1H), 7.27 (br d, *J* = 8.5 Hz, 2H), 7.01 (br d, *J* = 8.9 Hz, 2H), 3.75 (s, 3H), 3.46 (br t, *J* = 6.1 Hz, 1H), 1.65 (br t, *J* = 6.4 Hz, 2H). Three alkyl protons obscured by DMSO solvent peak.
Example 47: ¹H NMR (500 MHz, DMSO-d₆) δ 8.79 (br s, 1H), 8.54 (s, 1H), 8.44 (br d, *J=* 4.3 Hz, 1H), 7.92 (s, 1H), 7.83 (br d, *J* = 8.5 Hz, 2H), 7.73 (br d, *J* = 7.6 Hz, 1H), 7.35 (dd, *J* = 7.3, 4.9 Hz, 1H), 7.31 (t, *J* = 73.5 Hz, 1H), 7.24 (br d, *J* = 8.5 Hz, 2H), 6.92 (br d, *J =* 10.1 Hz, 2H), 4.46 (br d, *J =* 6.1 Hz, 2H), 3.77 (s, 3H).
Example 49:¹HNMR (500 MHz, DMSO-d₆) δ 7.87 (s, 1H), 7.83 (br d, *J=* 8.2 Hz, 2H), 7.67 (br d, *J* = 7.3 Hz, 1H), 7.32 (t, J = 73.4 Hz,1H), 7.24 (br d, *J* = 8.2 Hz, 2H), 6.91 (br d, *J =* 10.1 Hz, 2H), 3.89 (s, 1H), 3.77 (s, 3H), 3.74 - 3.68 (m, 1H), 1.86 - 1.76 (m, 4H), 1.47 - 1.36 (m, 2H), 1.29 - 1.20 (m, 2H).
Example 50: ¹H NMR (500 MHz, DMSO-d₆) δ 7.90 - 7.82 (m, 3H), 7.32 (t, *J* = 73.4 Hz,1H), 7.24 (br d, *J* =8.2 Hz, 2H), 6.90 (br d, *J=* 10.4 Hz, 2H), 4.18 (br s, 1H), 3.89 (s, 1H), 3.76 (s, 3H), 3.66 - 3.54 (m, 1H), 3.45 (s, 1H), 3.16 (s, 1H), 2.91 (br d, *J* = 7.0 Hz, 2H), 1.97 - 1.82 (m, 4H).
Example 51: ¹H NMR (500 MHz, DMSO-d₆) δ 7.86 - 7.80 (m, 4H), 7.31 (t, *J* = 73.6 Hz,1H), 7.24 (br d, *J* = 8.2 Hz, 2H), 6.90 (br d, *J* = 10.4 Hz, 2H), 3.76 (s, 3H), 3.46 (br s, 1H), 3.16 (br s, 1H), 2.91 (q, *J=* 7.0 Hz, 1H), 1.90 (br d, *J=* 4.6 Hz, 1H), 1.73 (br s, 1H), 1.41 (br s, 2H), 1.27 - 1.21 (m, 1H), 1.15 (t, *J* = 7.3 Hz, 2H).
Example 52: ¹H NMR (500 MHz, DMSO-d₆) δ 7.86 - 7.81 (m, 3H), 7.32 (t, *J=* 73.4 Hz,1H) , 7.24 (br d, *J* = 8.5 Hz, 2H), 6.90 (br d, *J* = 10.4 Hz, 2H), 4.12 - 3.92 (m, 1H), 3.76 (s, 3H), 3.49 - 3.42 (m, 1H), 3.16 (br d, *J* = 4.3 Hz, 1H), 2.95 - 2.87 (m, 1H), 1.79 (br d, *J* = 9.2 Hz, 2H), 1.36 (br d, J=8.5 Hz, 2H), 1.15 (t, *J* = 7.2 Hz, 1H).
Example 53: ¹H NMR (500 MHz, DMSO-d₆) δ 7.94 - 7.88 (m, 2H), 7.84 (br d, *J=* 8.2 Hz, 2H), 7.33(t, *J*= 73.6 Hz,1H), 7.25 (br d, *J=* 8.5 Hz, 2H), 6.93 (br d, *J*=10.4 Hz, 2H), 4.78 (br t, *J =* 5.2 Hz, 1H), 3.77 (s, 3H), 3.50 (q, *J*=5.8 Hz, 1H), 3.34 (br d, *J* = 8.9 Hz, 1H), 3.17 (d, *J* = 5.2 Hz, 1H), 2.92 (q, *J=* 7.2 Hz, 1H).
Example 54: ¹H NMR (500 MHz, DMSO-d₆) δ 7.91 (s, 1H), 7.84 (br d, *J* = 8.2 Hz, 2H), 7.33(t, *J=* 73.4 Hz,1H) , 7.24 (br d, *J=* 8.2 Hz, 2H), 6.91 (br d, *J=* 10.1 Hz, 2H), 3.77 (s, 3H), 3.64 (br s, 2H), 3.43 - 3.34 (m, 1H), 3.17 (br d, *J* = 4.6 Hz, 1H), 2.97 - 2.87 (m, 1H). Four alkyl protons not observed (water suppression).
Example 55: ¹H NMR (500 MHz, DMSO-d₆) δ 7.99 (s, 1H), 7.84 (br d, *J* = 8.2 Hz, 2H), 7.33(t, *J* = 73.6 Hz,1H), 7.25 (br d, *J* = 8.5 Hz, 2H), 6.92 (br d, *J* = 10.4 Hz, 2H), 3.77 (s, 3H), 3.48 - 3.40 (m, 1H), 3.16 (s, 1H), 2.92 (br d, *J* = 6.1 Hz, 1H), 2.74 - 2.62 (m, 3H). Four alkyl protons obscured by DMSO solvent peak.
Example 56: ¹H NMR (500 MHz, DMSO-d₆) δ 8.69 (br s, 1H), 8.47 (s, 2H), 7.94 (s, 1H), 7.84 (br d, *J=* 8.5 Hz, 2H), 7.33 (t, *J=* 73.6 Hz,1H), 7.25 (br d, *J=* 8.5 Hz, 2H), 6.93 (br d, *J* = 10.1 Hz, 2H), 4.57 (br d, *J* = 5.8 Hz, 2H), 3.77 (s, 3H), 2.47 (s, 3H).
Example 57: ¹H NMR (500 MHz, DMSO-d₆) δ 7.84 (br d, *J* = 10.4 Hz, 3H), 7.27 (t, *J=* 73.6 Hz,1H) ,7.23 (br d, *J=* 7.8 Hz, 2H), 6.89 (br d, *J=* 6.4 Hz, 2H), 4.33 (br d, *J* =19.5 Hz, 1H), 3.91 (br s, 1H), 3.79 (br s, 3H), 3.63 - 3.52 (m, 1H), 3.50 - 3.38 (m, 1H), 2.98 (s, 1H), 2.02 - 1.76 (m, 3H).
Example 58:¹H NMR (500 MHz, DMSO-d₆) δ 8.09 (br s, 1H), 7.97 (s, 1H), 7.85 (br d, *J=* 8.5 Hz, 2H), 7.33 (t, *J=* 73.6 Hz,1H) , 7.24 (br d, *J=* 8.5 Hz, 2H), 6.92 (br d, *J=* 10.1 Hz, 2H), 3.77 (s, 3H), 1.90 (s, 1H), 1.23 (s, 1H). Alkyl protons obscured by DMSO peak and water suppression.
Example 59: ¹H NMR (500 MHz, DMSO-d₆) δ 7.92 - 7.83 (m, 3H), 7.34 (t, *J=* 73.2 Hz,1H) , 7.24 (br d, *J* = 7.9 Hz, 2H), 6.92 (br d, *J* = 9.2 Hz, 2H), 4.13 - 4.05 (m, 1H), 3.78 (br s, 3H), 3.65 - 3.55 (m, 1H), 3.51 - 3.39 (m, 1H), 2.38 (br dd, *J=* 12.1, 7.5 Hz, 1H), 2.33 - 2.22 (m, 1H), 1.99 (br d, *J=* 7.3 Hz, 1H), 1.94 - 1.86 (m, 1H), 1.71 (br s, 1H), 1.66 - 1.55 (m, 1H), 1.23 (s, 1H).
Example 60: ¹H NMR (500 MHz, DMSO-d₆) δ 8.08 (br s, 1H), 7.87 (s, 1H), 7.83 (br d, *J* = 8.2 Hz, 2H), 7.32 (t, *J* = 73.5 Hz,1H) 7.24 (br d, *J* = 8.2 Hz, 2H), 6.91 (br d, *J* = 10.4 Hz, 2H), 4.58 (t, *J =* 5.2 Hz, 1H), 3.77 (s, 3H), 3.31 (q, *J* = 6.7 Hz, 1H), 3.16 (d, *J =* 5.2 Hz, 1H), 1.66 (quin, *J* = 6.5 Hz, 3H).
Example 61: ¹H NMR (500 MHz, DMSO-d₆) δ 7.88 (s, 1H), 7.85 (br d, *J* = 8.5 Hz, 2H), 7.34 (t, *J=* 73.6 Hz, 1H), 7.25 (d, *J* = 8.5 Hz, 2H), 6.93 (br d, *J* = 10.1 Hz, 2H), 3.78 (s, 3H), 2.85 - 2.78 (m, 4H). Four protons obscured by DMSO solvent peak.
Example 64: ¹H NMR (500 MHz, DMSO-d₆) δ 7.95 (br d, *J* = 7.3 Hz, 2H), 7.46 (br s, 2H), 7.31 (t, J=73.8 Hz, 1H), 7.22 (br d, *J* = 8.2 Hz, 2H), 7.17 (br s, 1H), 7.15 (br s, 1H), 7.02 (br d, *J* = 8.2 Hz, 2H), 3.77 (s, 3H), 3.30 (br s, 1H), 3.05 (br d, *J* = 8.9 Hz, 1H), 2.84 (br s, 1H), 2.78 - 2.63 (m, 2H), 2.07 - 2.01 (m, 1H), 1.74 (br s, 1H), 1.58 (br s, 2H).
Example 68: ¹H NMR (500 MHz, DMSO-d₆) δ 7.97 - 7.81 (m, 2H), 7.35 - 6.80 (m, 7H), 4.53 (br t, *J* = 5.5 Hz, 1H), 4.45 (br d, *J*=3.1 Hz, 1H), 3.97 (br d, *J* = 11.6 Hz, 1H), 3.91 - 3.74 (m, 3H), 3.65 (q, *J* = 6.4 Hz, 2H), 3.19 - 3.08 (m, 1H), 2.99 - 2.90 (m, 1H), 2.80 (br s, 1H), 2.67 (br d, *J* = 19.8 Hz, 1H), 2.08 (s, 1H), 2.06 - 1.94 (m, 2H), 1.92 (s, 1H), 1.52 (br s, 1H), 1.41 (br d, *J* = 10.7 Hz, 1H), 1.24 (s, 1H).
Example 69: ¹H NMR (500 MHz, DMSO-d₆) δ 7.87 - 7.81 (m, 3H), 7.34 (t, *J=* 73.5 Hz,1H), 7.24 (br d, *J* = 8.5 Hz, 2H), 6.91 (br d, *J =* 10.4 Hz, 2H), 5.16 - 5.03 (m, 1H), 4.50 (br d, *J* = 5.5 Hz, 1H), 3.90 (s, 1H), 3.77 (s, 3H), 3.31 - 3.24 (m, 1H), 3.17 (d, *J* = 4.9 Hz, 1H), 1.91 (s, 1H), 1.78 - 1.63 (m, 4H), 1.11 (br s, 2H).
Example 71: ¹H NMR (500 MHz, DMSO-d₆) δ 8.00 - 7.76 (m, 2H), 7.38 - 7.27 (m, 4H), 7.29 - 6.78 (m, 8H), 4.03 - 3.72 (m, 5H).
Example 72: ¹H NMR (500 MHz, CDCl₃) δ 8.10 (br d, *J* =8.3 Hz, 2H), 7.06 (br d, *J=* 8.3 Hz, 2H), 6.66 (s, 1H), 6.64 (s, 2H), 6.54 (t, *J* = 73.7 Hz, 1H), 4.25 (q, *J* = 7.0 Hz, 2H), 3.87 (s, 3H), 3.65 (s, 2H), 1.32 (t, *J* = 7.2 Hz, 3H).
Example 78: ¹H NMR (500 MHz, DMSO-d₆) δ 8.40 (br s, 1H), 8.03 - 7.74 (m, 4H), 7.38 (br d, *J* = 7.6 Hz, 1H), 7.30 - 7.07 (m, 4H), 6.92 - 6.62 (m, 2H), 3.83 (br s, 2H), 3.73 (br s, 3H).
Example 79: ¹H NMR (500 MHz, DMSO-d₆) δ 7.91 - 7.86 (m, 2H), 7.31 (s, 1H), 7.24 - 7.17 (m, 3H), 6.98 - 6.92 (m, 2H), 3.98 - 3.84 (m, 4H), 3.81 (s, 3H), 3.29 - 3.21 (m, 1H).
Example 80: ¹H NMR (500 MHz, CD₃OD) δ 8.01 - 7.80 (m, 3H), 7.31 - 7.20 (m, 3H), 7.13 - 6.61 (m, 4H), 3.87 (s, 3H).
Example 81: ¹H NMR (500 MHz, DMSO-d₆) δ 7.96 - 7.73 (m, 2H), 7.41 - 7.08 (m, 3H), 7.07 - 7.03 (m, 1H), 6.91 (br d, *J=* 10.3 Hz, 2H), 3.82 (s, 3H), 3.70 (br s, 1H), 3.41 - 2.92 (m, 1H), 2.67 - 2.59 (m, 2H), 2.36 - 2.26 (m, 2H).
Example 83: ¹H NMR (500 MHz, CD₃OD) δ 8.05 - 7.76 (m, 2H), 7.21 - 7.08 (m, 2H), 7.05 - 6.63 (m, 4H), 4.24 - 4.03 (m, 1H), 3.88 - 3.81 (m, 3H), 3.52 - 3.43 (m, 1H), 2.91 (s, 3H), 2.63 - 2.54 (m, 2H), 2.49 (br s, 2H).

## Claims

1. A compound of Formula (I): 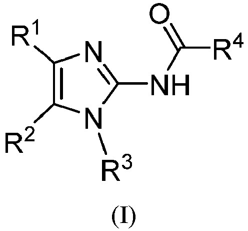 wherein
R¹ is alkyl, haloalkyl, hydroxyalkyl, alkoxyalkyl, (alkoxycarbonyl)alkyl, alkoxycarbonyl, (NR⁶R⁷)carbonyl, Ar¹, or (Ar¹)alkyl;
Ar¹ is cycloalkyl, aryl, heteroaryl comprising carbon atoms and 1-5 heteroatoms selected from N, NR^{5a}, O, and S, heterocyclyl comprising carbon atoms and 1-5 heteroatoms selected from N, NR^{5a}, O, and S, or spiroheterocyclyl comprising carbon atoms and 1-5 heteroatoms selected from N, NR^{5a}, O, and S, each substituted with 1-5 R⁵;
R² is hydrogen, alkyl, or haloalkyl;
R³ is phenyl or pyridinyl substituted with 1 R^{3a} and 1-2 R^{3b};
R^{3a} is halo, haloalkyl, alkoxy, or haloalkoxy;
R^{3b} is hydrogen, halo, or haloalkyl;
R⁴ is phenyl or pyridinyl substituted with 1-2 R^{4a};
R^{4a} is halo, alkoxy, or haloalkoxy;
R⁵ is hydrogen, hydroxyl, cyano, halo, alkyl, haloalkyl, amino, haloalkylamino, alkoxyalkyl, hydroxyalkyl, alkoxy, haloalkoxy, carboxamide, alkoxycarbonyl, alkylsulfonylamino, or hydroxyalkylcarbonyl;
R^{5a} is hydrogen, alkyl, haloalkyl, alkoxyalkyl, hydroxyalkyl, hydroalkylcarbonyl, carboxamide, alkylaminocarbonyl, aminocarbonylalkylcarbonyl, alkylsulfonyl, or alkoxycarbonyl;
R⁶ and R⁷ are independently hydrogen, alkyl, haloalkyl, hydroxyalkyl, cycloalkyl, aryl, heteroaryl comprising carbon atoms and 1-4 heteroatoms selected from N, NR^{8a}, O, and S, heterocyclyl comprising carbon atoms and 1-4 heteroatoms selected from N, NR^{8a}, O, and S, arylalkyl, or heteroarylalkyl comprising carbon atoms and 1-4 heteroatoms selected from N, NR^{8a}, O, and S; wherein said cycloalkyl, aryl, heteroaryl, or heterocyclyl is substituted with 1-5 R⁸;
or R⁶ and R⁷, together with the nitrogen to which they are attached, form a heterocyclyl or heteroaryl comprising carbon atoms and 0-3 additional heteroatoms selected from N, NR^{8a}, O, S, wherein said heteroaryl or heterocyclyl is substituted with 1-5 R⁸;
R⁸ is hydrogen, halo, hydroxy, hydroxyalkyl, alkyl, alkoxy, or oxo;
R^{8a} is hydrogen, hydroxyalkyl, or alkyl;
or a pharmaceutically acceptable salt thereof.

2. The compound of claim 1 wherein
R³ is phenyl substituted with 1 R^{3a} and 1-2 R^{3b};
R^{3a} is halo, haloalkyl, or alkoxy substituent in the para-position with respect to the imidazole moiety; and
R^{3b} is hydrogen, halo, or haloalkyl.

3. The compound of claim 1 wherein
R⁴ is phenyl substituted with 1 R^{4a} in the para-position with respect to the amide moiety.

4. The compound of claim 1, having Formula (II): 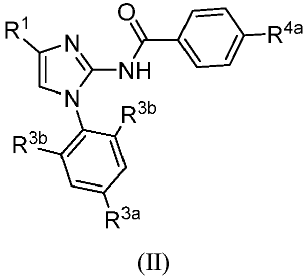 wherein
R¹ is alkyl, haloalkyl, hydroxyalkyl, alkoxyalkyl, (alkoxycarbonyl)alkyl, alkoxycarbonyl, (NR⁶R⁷)carbonyl, Ar¹, or (Ar¹)alkyl;
Ar¹ is cycloalkyl, aryl, heteroaryl comprising carbon atoms and 1-4 heteroatoms selected from N, NR^{5a}, O, and S, heterocyclyl comprising carbon atoms and 1-4 heteroatoms selected from N, NR^{5a}, O, and S, spiroheterocyclyl comprising carbon atoms and 1-4 heteroatoms selected from N, NR^{5a}, O, and S, each substituted with 1-4 R⁵;
R^{3a} is alkoxy;
R^{3b} is hydrogen, halo or haloalkyl;
R^{4a} is halo or haloalkoxy;
R⁵ is hydrogen, hydroxyl, cyano, halo, alkyl, haloalkyl, amino, haloalkylamino, alkoxyalkyl, hydroxyalkyl, alkoxy, haloalkoxy, alkoxycarbonyl, or alkylsulfonylamino;
R^{5a} is hydrogen, alkyl, haloalkyl, alkoxyalkyl, hydroxyalkyl, hydroalkylcarbonyl, carboxamide, alkylaminocarbonyl, aminocarbonylalkylcarbonyl, alkylsulfonyl, or alkoxycarbonyl;
R⁶ and R⁷ are independently hydrogen, alkyl, haloalkyl, hydroxyalkyl, cycloalkyl, heteroaryl comprising carbon atoms and 1-4 heteroatoms selected from N, NR^{8a}, O, and S, arylalkyl, or heteroarylalkyl comprising carbon atoms and 1-4 heteroatoms selected from N, NR^{8a}, O, and S, wherein said cycloalkyl, heteroaryl, or heteroarylalkyl is substituted with 1-4 R⁸;
or R⁶ and R⁷, together with the nitrogen to which they are attached, form a heterocyclyl or heteroaryl with 0-3 additional heteroatoms selected from N, NR^{8a}, O, and S, wherein said heterocyclyl or heteroaryl is substituted with 1-4 R⁸;
R⁸ is hydrogen, halo, hydroxy, hydroxyalkyl, alkyl, alkoxy, or oxo;
R^{8a} is hydrogen, hydroxyalkyl, or alkyl;
or a pharmaceutically acceptable salt thereof.

5. The compound of claim 4, wherein
R¹ is Ar¹ substituted with 1-3 R⁵;
Ar¹ is cycloalkyl, aryl, heteroaryl comprising carbon atoms and 1-3 heteroatoms selected from N, NR^{5a}, O, and S, heterocyclyl comprising carbon atoms and 1-3 heteroatoms selected from N, NR^{5a}, O, and S, spiroheterocyclyl comprising carbon atoms and 1-3 heteroatoms selected from N, NR^{5a}, O, and S, each substituted with 1-3 R⁵;
R^{3a} is alkoxy;
R^{3b} is hydrogen or halo;
R^{4a} is haloalkoxy;
R⁵ is hydrogen, hydroxyl, cyano, halo, alkyl, haloalkyl, amino, haloalkylamino, alkoxyalkyl, hydroxyalkyl, alkoxy, haloalkoxy, alkoxycarbonyl, or alkylsulfonylamino; and
R^{5a} is hydrogen, alkyl, haloalkyl, alkoxyalkyl, hydroxyalkyl, hydroalkylcarbonyl, alkylaminocarbonyl, aminocarbonylalkylcarbonyl, alkylsulfonyl, or alkoxycarbonyl.

6. The compound of claim 5, wherein
Ar¹ is 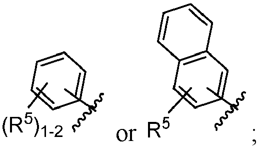 and
R⁵ is hydrogen, cyano, halo, alkyl, haloalkyl, alkoxyalkyl, hydroxyalkyl, alkoxy, or haloalkoxy;
or wherein
Ar¹ is 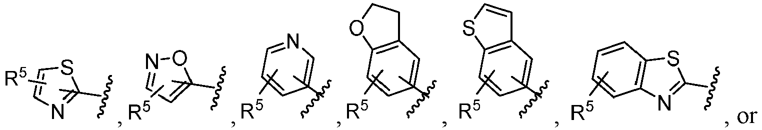 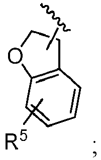 and
R⁵ is hydrogen, halo, alkyl, haloalkyl, alkoxyalkyl, hydroxyalkyl, alkoxy, alkoxycarbonyl, or haloalkoxy.

7. The compound of claim 5, wherein
Ar¹ is 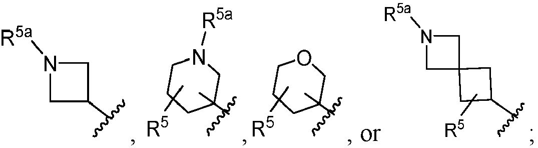
R⁵ is hydrogen, alkyl, or hydroxyalkyl; and
R^{5a} is hydrogen, alkyl, hydroalkylcarbonyl, alkylaminocarbonyl, aminocarbonylalkylcarbonyl, alkylsulfonyl, or alkoxycarbonyl.

8. The compound of claim 5, wherein
Ar¹ is 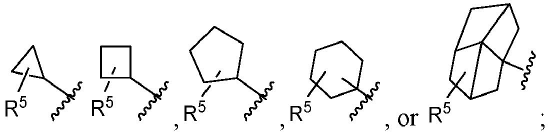 ; and
R⁵ is hydrogen, hydroxyl, hydroxyalkyl, amino, haloalkylamino, or alkylsulfonylamino.

9. The compound of claim 4, wherein
R¹ is (Ar¹)alkyl;
R^{3a} is alkoxy;
R^{3b} is hydrogen or halo; and
R^{4a} is haloalkoxy.

10. The compound of claim 9, wherein
Ar¹ is 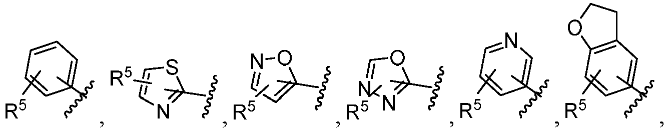 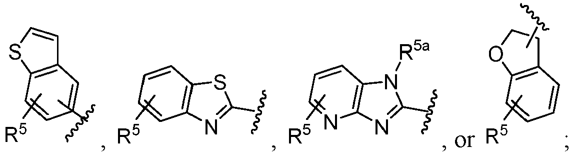
R⁵ is hydrogen, cyano, halo, alkyl, haloalkyl, alkoxyalkyl, hydroxyalkyl, alkoxy, or haloalkoxy; and
R^{5a} is hydrogen or alkyl.

11. The compound of claim 4, wherein
R¹ is alkyl or haloalkyl;
R^{3a} is alkoxy;
R^{3b} is hydrogen or halo; and
R^{4a} is haloalkoxy;
or wherein
R¹ is alkoxycarbonyl or (alkoxycarbonyl)alkyl;
R^{3a} is alkoxy;
R^{3b} is hydrogen or halo; and
R^{4a} is haloalkoxy.

12. The compound of claim 4, wherein
R¹ is (NR⁶R⁷)carbonyl;
R⁶ and R⁷ are independently hydrogen, alkyl, haloalkyl, hydroxyalkyl, cycloalkyl, heteroaryl comprising carbon atoms and 1-3 heteroatoms selected from N, NR^{8a}, O, and S, or heteroarylalkyl comprising carbon atoms and 1-3 heteroatoms selected from N, NR^{8a}, O, and S, wherein said cycloalkyl, heteroaryl, or heteroarylalkyl is substituted with 1-3 R⁸;
or R⁶ and R⁷, together with the nitrogen to which they are attached, form 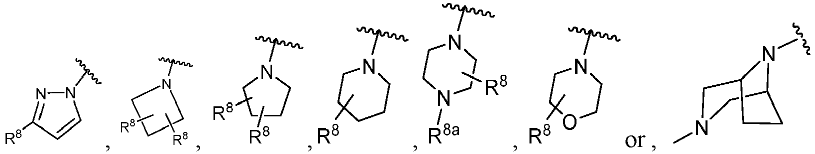
R⁸ is hydrogen, halo, hydroxy, hydroxyalkyl, alkyl, alkoxy, or oxo; and
R^{8a} is hydrogen, hydroxyalkyl, or alkyl;
preferably wherein
R⁶ is hydrogen;
R⁷ is 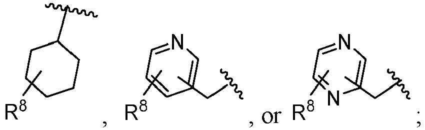 and
R⁸ is hydrogen, halo, hydroxy, hydroxyalkyl, alkyl, or alkoxy.

13. A pharmaceutical composition comprising a compound of any of claims 1 to 12 or a pharmaceutically acceptable salt thereof and a pharmaceutically acceptable carrier, diluent, or excipient.

14. A compound of any of claims 1 to 12 or a pharmaceutically acceptable salt thereof, or a pharmaceutical composition of claim 13, for use in therapy.

15. A compound of any of claims 1 to 12 or a pharmaceutically acceptable salt thereof, or a pharmaceutical composition of claim 13, for use in a method for treating a heart disease, preferably wherein the heart disease is selected from the group consisting of angina pectoris, unstable angina, myocardial infarction, heart failure, acute coronary disease, acute heart failure, chronic heart failure, and cardiac iatrogenic damage.

## Patentansprüche

1. Verbindung der Formel (I): 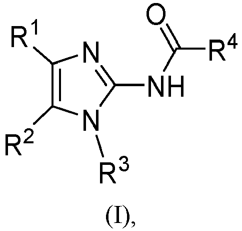 wobei
R¹ Alkyl, Haloalkyl, Hydroxyalkyl, Alkoxyalkyl, (Alkoxycarbonyl)alkyl, Alkoxycarbonyl, (NR⁶R⁷)-Carbonyl, Ar¹ oder (Ar¹)-Alkyl ist;
Ar¹ Cycloalkyl, Aryl, Heteroaryl, umfassend Kohlenstoffatome und 1-5 Heteroatome, die aus N, NR^{5a}, O und S ausgewählt sind, Heterocyclyl, umfassend Kohlenstoffatome und 1-5 Heteroatome, die aus N, NR^{5a}, O und S ausgewählt sind, oder Spiroheterocyclyl ist, umfassend Kohlenstoffatome und 1-5 Heteroatome, die aus N, NR^{5a}, O und S ausgewählt sind, die jeweils mit 1-5 R⁵ substituiert sind;
R² Wasserstoff, Alkyl oder Haloalkyl ist;
R³ Phenyl oder Pyridinyl ist, das mit 1 R^{3a} und 1-2 R^{3b} substituiert ist;
R^{3a} Halo, Haloalkyl, Alkoxy oder Haloalkoxy ist;
R^{3b} Wasserstoff, Halo oder Haloalkyl ist;
R⁴ Phenyl oder Pyridinyl ist, das mit 1-2 R^{4a} substituiert ist;
R^{4a} Halo, Alkoxy oder Haloalkoxy ist;
R⁵ Wasserstoff, Hydroxyl, Cyano, Halo, Alkyl, Haloalkyl, Amino, Haloalkylamino, Alkoxyalkyl, Hydroxyalkyl, Alkoxy, Haloalkoxy, Carboxamid, Alkoxycarbonyl, Alkylsulfonylamino oder Hydroxyalkylcarbonyl ist;
R^{5a} Wasserstoff, Alkyl, Haloalkyl, Alkoxyalkyl, Hydroxyalkyl, Hydroalkylcarbonyl, Carboxamid, Alkylaminocarbonyl, Aminocarbonylalkylcarbonyl, Alkylsulfonyl oder Alkoxycarbonyl ist;
R⁶ und R⁷ unabhängig Wasserstoff, Alkyl, Haloalkyl, Hydroxyalkyl, Cycloalkyl, Aryl, Heteroaryl, umfassend Kohlenstoffatome und 1-4 Heteroatome, die aus N, NR^{8a}, O und S ausgewählt sind, Heterocyclyl, umfassend Kohlenstoffatome und 1-4 Heteroatome, die aus N, NR^{8a}, O und S ausgewählt sind, Arylalkyl, oder Heteroarylalkyl, umfassend Kohlenstoffatome und 1-4 Heteroatome, die aus N, NR^{8a}, O und S ausgewählt sind, sind; wobei das Cycloalkyl, Aryl, Heteroaryl oder Heterocyclyl mit 1-5 R⁸ substituiert ist;
oder R⁶ und R⁷ zusammen mit dem Stickstoff, an den sie angelagert sind, ein Heterocyclyl oder Heteroaryl, umfassend Kohlenstoffatome und 0-3 zusätzliche Heteroatome, die aus N, NR^{8a}, O und S ausgewählt sind, bilden, wobei das Heteroaryl oder Heterocyclyl mit 1-5 R⁸ substituiert ist;
R⁸ Wasserstoff, Halo, Hydroxy, Hydroxyalkyl, Alkyl, Alkoxy oder Oxo ist;
R^{8a} Wasserstoff, Hydroxyalkyl oder Alkyl ist;
oder ein pharmazeutisch verträgliches Salz davon.

2. Verbindung nach Anspruch 1, wobei
R³ Phenyl ist, das mit 1 R^{3a} und 1-2 R^{3b} substituiert ist;
R^{3a} ein Halo-, Haloalkyl- oder Alkoxysubstituent in der para-Stellung in Bezug auf den Imidazol-Anteil ist und
R^{3b} Wasserstoff, Halo oder Haloalkyl ist.

3. Verbindung nach Anspruch 1, wobei
R⁴ Phenyl, das mit 1 R^{4a} substituiert ist, in der para-Stellung in Bezug auf den Amid-Anteil ist.

4. Verbindung nach Anspruch 1 mit der Formel (II): 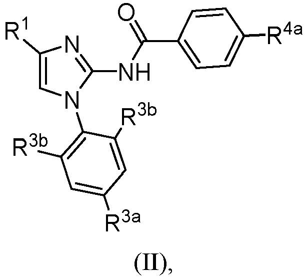 wobei
R¹ Alkyl, Haloalkyl, Hydroxyalkyl, Alkoxyalkyl, (Alkoxycarbonyl)alkyl, Alkoxycarbonyl, (NR⁶R⁷)Carbonyl, Ar¹ oder (Ar¹)Alkyl ist;
Ar¹ Cycloalkyl, Aryl, Heteroaryl, umfassend Kohlenstoffatome und 1-4 Heteroatome, die aus N, NR^{5a}, O und S ausgewählt sind, Heterocyclyl, umfassend Kohlenstoffatome und 1-4 Heteroatome, die aus N, NR^{5a}, O und S ausgewählt sind, Spiroheterocyclyl, umfassend Kohlenstoffatome und 1-4 Heteroatome, die aus N, NR^{5a}, O und S ausgewählt sind, die jeweils mit 1-4 R⁵ substituiert sind, ist;
R^{3a} Alkoxy ist;
R^{3b} Wasserstoff, Halo oder Haloalkyl ist;
R^{4a} Halo oder Haloalkoxy ist;
R⁵ Wasserstoff, Hydroxyl, Cyano, Halo, Alkyl, Haloalkyl, Amino, Haloalkylamino, Alkoxyalkyl, Hydroxyalkyl, Alkoxy, Haloalkoxy, Alkoxycarbonyl oder Alkylsulfonylamino ist;
R^{5a} Wasserstoff, Alkyl, Haloalkyl, Alkoxyalkyl, Hydroxyalkyl, Hydroalkylcarbonyl, Carboxamid, Alkylaminocarbonyl, Aminocarbonylalkylcarbonyl, Alkylsulfonyl oder Alkoxycarbonyl ist;
R⁶ und R⁷ unabhängig Wasserstoff, Alkyl, Haloalkyl, Hydroxyalkyl, Cycloalkyl, Heteroaryl, umfassend Kohlenstoffatome und 1-4 Heteroatome, die aus N, NR^{8a}, O und S ausgewählt sind, Arylalkyl oder Heteroarylalkyl, umfassend Kohlenstoffatome und 1-4 Heteroatome, die aus N, NR^{8a}, O und S ausgewählt sind, sind; wobei das Cycloalkyl, Heteroaryl oder Heteroarylalkyl mit 1-4 R⁸ substituiert ist;
oder R⁶ und R⁷ zusammen mit dem Stickstoff, an den sie angelagert sind, ein Heterocyclyl oder Heteroaryl mit 0-3 zusätzlichen Heteroatomen, die aus N, NR^{8a}, O und S ausgewählt sind, bilden, wobei das Heterocyclyl oder Heteroaryl mit 1-4 R⁸ substituiert ist;
R⁸ Wasserstoff, Halo, Hydroxy, Hydroxyalkyl, Alkyl, Alkoxy oder Oxo ist;
R^{8a} Wasserstoff, Hydroxyalkyl oder Alkyl ist;
oder ein pharmazeutisch verträgliches Salz davon.

5. Verbindung nach Anspruch 4, wobei
R¹ Ar¹ ist, das mit 1-3 R⁵ substituiert ist;
Ar¹ Cycloalkyl, Aryl, Heteroaryl, umfassend Kohlenstoffatome und 1-3 Heteroatome, die aus N, NR^{5a}, O und S ausgewählt sind, Heterocyclyl, umfassend Kohlenstoffatome und 1-3 Heteroatome, die aus N, NR^{5a}, O und S ausgewählt sind, Spiroheterocyclyl, umfassend Kohlenstoffatome und 1-3 Heteroatome, die aus N, NR^{5a}, O und S ausgewählt sind, die jeweils mit 1-3 R⁵ substituiert sind, ist;
R^{3a} Alkoxy ist;
R^{3b} Wasserstoff oder Halo ist;
R^{4a} Haloalkoxy ist;
R⁵ Wasserstoff, Hydroxyl, Cyano, Halo, Alkyl, Haloalkyl, Amino, Haloalkylamino, Alkoxyalkyl, Hydroxyalkyl, Alkoxy, Haloalkoxy, Alkoxycarbonyl oder Alkylsulfonylamino ist und
R^{5a} Wasserstoff, Alkyl, Haloalkyl, Alkoxyalkyl, Hydroxyalkyl, Hydroalkylcarbonyl, Alkylaminocarbonyl, Aminocarbonylalkylcarbonyl, Alkylsulfonyl oder Alkoxycarbonyl ist.

6. Verbindung nach Anspruch 5, wobei
Ar¹ 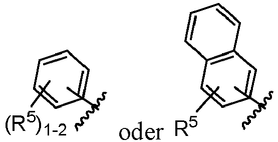
ist; und
R⁵ Wasserstoff, Cyano, Halo, Alkyl, Haloalkyl, Alkoxyalkyl, Hydroxyalkyl, Alkoxy oder Haloalkoxy ist;
oder wobei 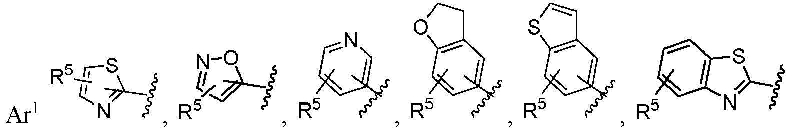 oder 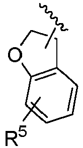 ist; und
R⁵ Wasserstoff, Halo, Alkyl, Haloalkyl, Alkoxyalkyl, Hydroxyalkyl, Alkoxy, Alkoxycarbonyl oder Haloalkoxy ist.

7. Verbindung nach Anspruch 5, wobei
Ar¹ 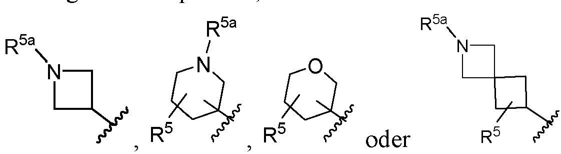 ist;
R⁵ Wasserstoff, Alkyl oder Hydroxyalkyl ist und
R^{5a} Wasserstoff, Alkyl, Hydroalkylcarbonyl, Alkylaminocarbonyl, Aminocarbonylalkylcarbonyl, Alkylsulfonyl oder Alkoxycarbonyl ist.

8. Verbindung nach Anspruch 5, wobei 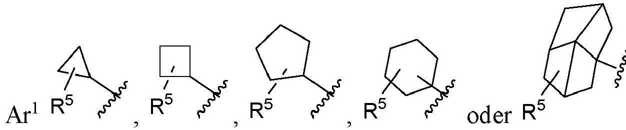 ist; und
R⁵ Wasserstoff, Hydroxyl, Hydroxyalkyl, Amino, Haloalkylamino oder Alkylsulfonylamino ist.

9. Verbindung nach Anspruch 4, wobei
R¹ (Ar¹)Alkyl ist;
R^{3a} Alkoxy ist;
R^{3b} Wasserstoff oder Halo ist und
R^{4a} Haloalkoxy ist.

10. Verbindung nach Anspruch 9, wobei 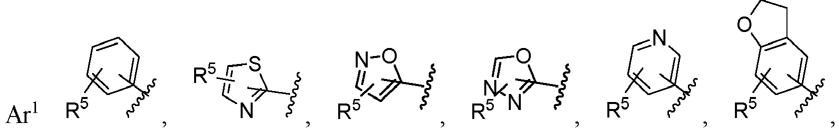 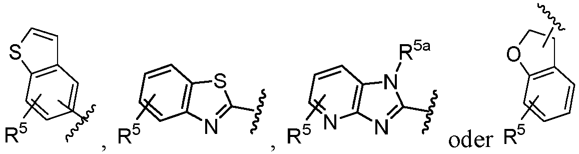 ist;
R⁵ Wasserstoff, Cyano, Halo, Alkyl, Haloalkyl, Alkoxyalkyl, Hydroxyalkyl, Alkoxy oder Haloalkoxy ist und
R^{5a} Wasserstoff oder Alkyl ist.

11. Verbindung nach Anspruch 4, wobei
R¹ Alkyl oder Haloalkyl ist;
R^{3a} Alkoxy ist;
R^{3b} Wasserstoff oder Halo ist; und
R^{4a} Haloalkoxy ist;
oder wobei
R¹ Alkoxycarbonyl oder (Alkoxycarbonyl)alkyl ist;
R^{3a} Alkoxy ist;
R^{3b} Wasserstoff oder Halo ist und
R^{4a} Haloalkoxy ist.

12. Verbindung nach Anspruch 4, wobei
R¹ (NR⁶R⁷)Carbonyl ist;
R⁶ und R⁷ unabhängig Wasserstoff, Alkyl, Haloalkyl, Hydroxyalkyl, Cycloalkyl, Heteroaryl, umfassend Kohlenstoffatome und 1-3 Heteroatome, die aus N, NR^{8a}, O und S ausgewählt sind, oder Heteroarylalkyl, umfassend Kohlenstoffatome und 1-3 Heteroatome, die aus N, NR^{8a}, O und S ausgewählt sind, sind, wobei das Cycloalkyl, Heteroaryl oder Heteroarylalkyl mit 1-3 R⁸ substituiert ist:
oder R⁶ und R⁷ zusammen mit dem Stickstoff, an den sie angelagert sind, 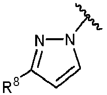 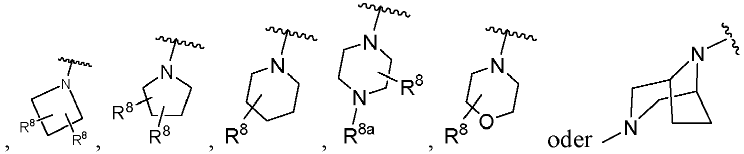 bilden,
R⁸ Wasserstoff, Halo, Hydroxy, Hydroxyalkyl, Alkyl, Alkoxy oder Oxo ist und
R^{8a} Wasserstoff, Hydroxyalkyl oder Alkyl ist;
vorzugsweise wobei
R⁶ Wasserstoff ist; 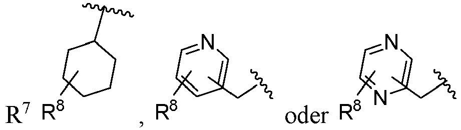 ist; und
R⁸ Wasserstoff, Halo, Hydroxy, Hydroxyalkyl, Alkyl oder Alkoxy ist.

13. Pharmazeutische Zusammensetzung, umfassend eine Verbindung nach einem der Ansprüche 1 bis 12 oder ein pharmazeutisch verträgliches Salz davon und einen pharmazeutisch verträglichen Trägerstoff, ein pharmazeutisch verträgliches Verdünnungsmittel oder einen pharmazeutisch verträglichen Hilfsstoff.

14. Verbindung nach einem der Ansprüche 1 bis 12 oder pharmazeutisch verträgliches Salz davon oder pharmazeutische Zusammensetzung nach Anspruch 13 zur Verwendung in einer Therapie.

15. Verbindung nach einem der Ansprüche 1 bis 12 oder pharmazeutisch verträgliches Salz davon oder pharmazeutische Zusammensetzung nach Anspruch 13 zur Verwendung in einem Verfahren zur Behandlung einer Herzkrankheit, vorzugsweise wobei die Herzkrankheit aus der Gruppe bestehend aus Angina pectoris, instabiler Angina, Myokardinfarkt, Herzinsuffizienz, akuter koronarer Herzkrankheit, akuter Herzinsuffizienz, chronischer Herzinsuffizienz und kardialer iatrogener Schädigung ausgewählt ist.

## Revendications

1. Composé de formule (I) : 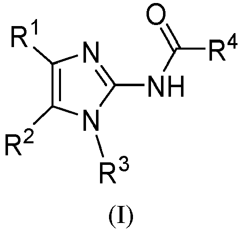 où
R¹ est un alkyle, un haloalkyle, un hydroxyalkyle, un alcoxyalkyle, un (alcoxycarbonyl)alkyle, un alcoxycarbonyle, un (NR⁶R⁷)carbonyle, Ar¹ ou un (Ar¹)alkyle ;
Ar¹ est un cycloalkyle, un aryle, un hétéroaryle comprenant des atomes de carbone et 1-5 hétéroatomes choisis parmi N, NR^{5a}, O et S, un hétérocyclyle comprenant des atomes de carbone et 1-5 hétéroatomes choisis parmi N, NR^{5a}, O et S, ou un spirohétérocyclyle comprenant des atomes de carbone et 1-5 hétéroatomes choisis parmi N, NR^{5a}, O et S, chacun étant substitué par 1-5 R⁵ ;
R² est un hydrogène, un alkyle ou un haloalkyle ;
R³ est un phényle ou un pyridinyle substitué par 1 R^{3a} et 1-2 R^{3b} ;
R^{3a} est un halo, un haloalkyle, un alcoxy ou un haloalcoxy ;
R^{3b} est un hydrogène, un halo ou un haloalkyle ;
R⁴ est un phényle ou un pyridinyle substitué par 1-2 R^{4a} ;
R^{4a} est un halo, un alcoxy ou un haloalcoxy ;
R⁵ est un hydrogène, un hydroxyle, un cyano, un halo, un alkyle, un haloalkyle, un amino, un haloalkylamino, un alcoxyalkyle, un hydroxyalkyle, un alcoxy, un haloalcoxy, un carboxamide, un alcoxycarbonyle, un alkylsulfonylamino ou un hydroxyalkylcarbonyle ;
R^{5a} est un hydrogène, un alkyle, un haloalkyle, un alcoxyalkyle, un hydroxyalkyle, un hydroalkylcarbonyle, un carboxamide, un alkylaminocarbonyle, un aminocarbonylalkylcarbonyle, un alkylsulfonyle ou un alcoxycarbonyle ;
R⁶ et R⁷ sont indépendamment un hydrogène, un alkyle, un haloalkyle, un hydroxyalkyle, un cycloalkyle, un aryle, un hétéroaryle comprenant des atomes de carbone et 1-4 hétéroatomes choisis parmi N, NR^{8a}, O et S, un hétérocyclyle comprenant des atomes de carbone et 1-4 hétéroatomes choisis parmi N, NR^{8a}, O et S, un arylalkyle ou un hétéroarylalkyle comprenant des atomes de carbone et 1-4 hétéroatomes choisis parmi N, NR^{8a}, O et S ; dans lesquels ledit cycloalkyle, aryle, hétéroaryle ou hétérocyclyle est substitué par 1-5 R⁸ ;
ou R⁶ et R⁷, ensemble avec l'azote auquel ils sont liés, forment un hétérocyclyle ou hétéroaryle comprenant des atomes de carbone et 0-3 hétéroatomes supplémentaires choisis parmi N, NR^{8a}, O et S, dans lequel ledit hétéroaryle ou hétérocyclyle est substitué par 1-5 R⁸;
R⁸ est un hydrogène, un halo, un hydroxy, un hydroxyalkyle, un alkyle, un alcoxy ou un oxo ;
R^{8a} est un hydrogène, un hydroxyalkyle ou un alkyle ;
ou un sel pharmaceutiquement acceptable de celui-ci.

2. Composé selon la revendication 1, dans lequel
R³ est un phényle substitué par 1 R^{3a} et 1-2 R^{3b} ;
R^{3a} est un substituant halo, haloalkyle ou alcoxy en position para par rapport à la fraction imidazole ; et
R^{3b} est un hydrogène, un halo ou un haloalkyle.

3. Composé selon la revendication 1, dans lequel
R⁴ est un phényle substitué par 1 R^{4a} en position para par rapport à la fraction amide.

4. Composé selon la revendication 1, ayant la formule (II) : 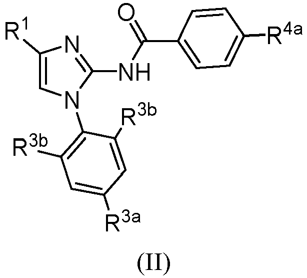 où
R¹ est un alkyle, un haloalkyle, un hydroxyalkyle, un alcoxyalkyle, un (alcoxycarbonyl)alkyle, un alcoxycarbonyle, un (NR⁶R⁷)carbonyle, Ar¹ ou un (Ar¹)alkyle ;
Ar¹ est un cycloalkyle, un aryle, un hétéroaryle comprenant des atomes de carbone et 1-4 hétéroatomes choisis parmi N, NR^{5a}, O et S, un hétérocyclyle comprenant des atomes de carbone et 1-4 hétéroatomes choisis parmi N, NR^{5a}, O et S, un spirohétérocyclyle comprenant des atomes de carbone et 1-4 hétéroatomes choisis parmi N, NR^{5a}, O et S, chacun étant substitué par 1-4 R⁵ ;
R^{3a} est un alcoxy ;
R^{3b} est un hydrogène, un halo ou un haloalkyle ;
R^{4a} est un halo ou un haloalcoxy ;
R⁵ est un hydrogène, un hydroxyle, un cyano, un halo, un alkyle, un haloalkyle, un amino, un haloalkylamino, un alcoxyalkyle, un hydroxyalkyle, un alcoxy, un haloalcoxy, un alcoxycarbonyle ou un alkylsulfonylamino ;
R^{5a} est un hydrogène, un alkyle, un haloalkyle, un alcoxyalkyle, un hydroxyalkyle, un hydroalkylcarbonyle, un carboxamide, un alkylaminocarbonyle, un aminocarbonylalkylcarbonyle, un alkylsulfonyle ou un alcoxycarbonyle ;
R⁶ et R⁷ sont indépendamment un hydrogène, un alkyle, un haloalkyle, un hydroxyalkyle, un cycloalkyle, un hétéroaryle comprenant des atomes de carbone et 1-4 hétéroatomes choisis parmi N, NR^{8a}, O, et S, un arylalkyle ou un hétéroarylalkyle comprenant des atomes de carbone et 1-4 hétéroatomes choisis parmi N, NR^{8a}, O et S ; dans lequel ledit cycloalkyle, hétéroaryle ou hétéroarylalkyle est substitué par 1-4 R⁸ ;
ou R⁶ et R⁷, ensemble avec l'azote auquel ils sont liés, forment un hétérocyclyle ou hétéroaryle avec 0-3 hétéroatomes supplémentaires choisis parmi N, NR^{8a}, O et S, dans lequel ledit hétérocyclyle ou hétéroaryle est substitué par 1-4 R⁸ ;
R⁸ est un hydrogène, un halo, un hydroxy, un hydroxyalkyle, un alkyle, un alcoxy ou un oxo ;
R^{8a} est un hydrogène, un hydroxyalkyle ou un alkyle ;
ou un sel pharmaceutiquement acceptable de celui-ci.

5. Composé selon la revendication 4, dans lequel
R¹ est Ar¹ substitué par 1-3 R⁵ ;
Ar¹ est un cycloalkyle, un aryle, un hétéroaryle comprenant des atomes de carbone et 1-3 hétéroatomes choisis parmi N, NR^{5a}, O et S, un hétérocyclyle comprenant des atomes de carbone et 1-3 hétéroatomes choisis parmi N, NR^{5a}, O et S, ou un spirohétérocyclyle comprenant des atomes de carbone et 1-3 hétéroatomes choisis parmi N, NR^{5a}, O et S, chacun étant substitué par 1-3 R⁵ ;
R^{3a} est un alcoxy ;
R^{3b} est un hydrogène ou un halo ;
R^{4a} est un haloalcoxy ;
R⁵ est un hydrogène, un hydroxyle, un cyano, un halo, un alkyle, un haloalkyle, un amino, un haloalkylamino, un alcoxyalkyle, un hydroxyalkyle, un alcoxy, un haloalcoxy, un alcoxycarbonyle ou un alkylsulfonylamino ; et
R^{5a} est un hydrogène, un alkyle, un haloalkyle, un alcoxyalkyle, un hydroxyalkyle, un hydroalkylcarbonyle, un alkylaminocarbonyle, un aminocarbonylalkylcarbonyle, un alkylsulfonyle ou un alcoxycarbonyle.

6. Composé selon la revendication 5, dans lequel
Ar¹ est 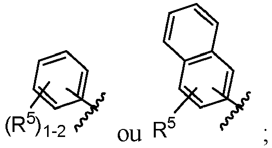 et
R⁵ est un hydrogène, un cyano, un halo, un alkyle, un haloalkyle, un alcoxyalkyle, un hydroxyalkyle, un alcoxy ou un haloalcoxy ;
ou dans lequel
Ar¹ est 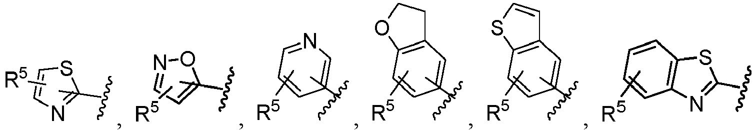 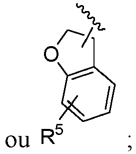 et
R⁵ est un hydrogène, un halo, un alkyle, un haloalkyle, un alcoxyalkyle, un hydroxyalkyle, un alcoxy, un alcoxycarbonyle ou un haloalcoxy.

7. Composé selon la revendication 5, dans lequel
Ar¹ est 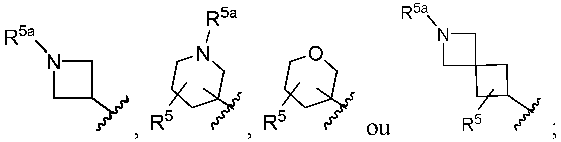
R⁵ est un hydrogène, un alkyle ou un hydroxyalkyle ; et
R^{5a} est un hydrogène, un alkyle, un hydroalkylcarbonyle, un alkylaminocarbonyle, un aminocarbonylalkylcarbonyle, un alkylsulfonyle ou un alcoxycarbonyle.

8. Composé selon la revendication 5, dans lequel
Ar¹ est 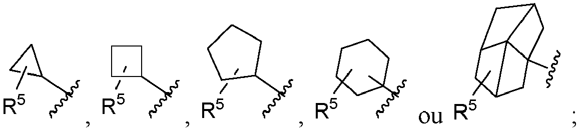 et
R⁵ est un hydrogène, un hydroxyle, un hydroxyalkyle, un amino, un haloalkylamino ou un alkylsulfonylamino.

9. Composé selon la revendication 4, dans lequel
R¹ est un (Ar¹)alkyle ;
R^{3a} est un alcoxy ;
R^{3b} est un hydrogène ou un halo ; et
R^{4a} est un haloalcoxy.

10. Composé selon la revendication 9, dans lequel
Ar¹ est 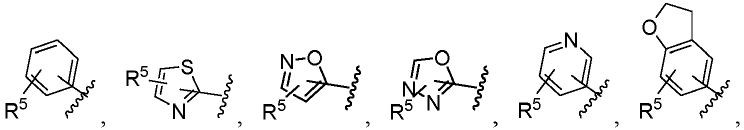 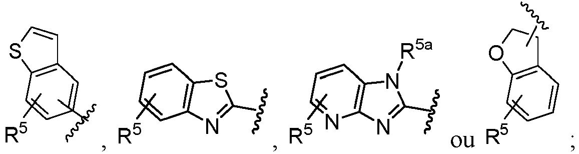
R⁵ est un hydrogène, un cyano, un halo, un alkyle, un haloalkyle, un alcoxyalkyle, un hydroxyalkyle, un alcoxy ou un haloalcoxy ; et
R^{5a} est un hydrogène ou un alkyle.

11. Composé selon la revendication 4, dans lequel
R¹ est un alkyle ou un haloalkyle ;
R^{3a} est un alcoxy ;
R^{3b} est un hydrogène ou un halo ; et
R^{4a} est un haloalcoxy ;
ou dans lequel
R¹ est un alcoxycarbonyle ou un (alcoxycarbonyle)alkyle ;
R^{3a} est un alcoxy ;
R^{3b} est un hydrogène ou un halo ; et
R^{4a} est un haloalcoxy.

12. Composé selon la revendication 4, dans lequel
R¹ est un (NR⁶R⁷)carbonyle ;
R⁶ et R⁷ sont indépendamment un hydrogène, un alkyle, un haloalkyle, un hydroxyalkyle, un cycloalkyle, un hétéroaryle comprenant des atomes de carbone et 1-3 hétéroatomes choisis parmi N, NR^{8a}, O et S, ou un hétéroarylalkyle comprenant des atomes de carbone et 1-3 hétéroatomes choisis parmi N, NR^{8a}, O et S, dans lequel ledit cycloalkyle, hétéroaryle ou hétéroarylalkyle est substitué par 1-3 R⁸ ;
ou R⁶ et R⁷, ensemble avec l'azote auquel ils sont liés, forment 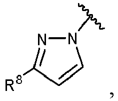 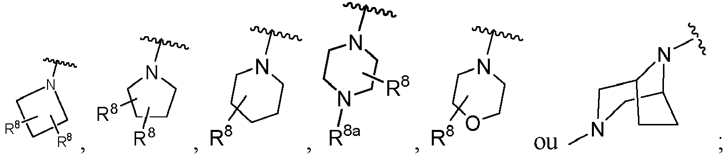
R⁸ est un hydrogène, un halo, un hydroxy, un hydroxyalkyle, un alkyle, un alcoxy ou un oxo ; et
R^{8a} est un hydrogène, un hydroxyalkyle ou un alkyle ;
de préférence dans lequel
R⁶ est un hydrogène ;
R⁷ est 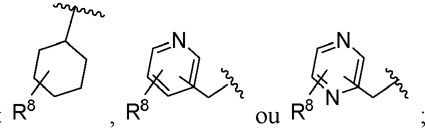 et
R⁸ est un hydrogène, un halo, un hydroxy, un hydroxyalkyle, un alkyle ou un alcoxy.

13. Composition pharmaceutique comprenant un composé selon l'une quelconque des revendications 1 à 12 ou un sel pharmaceutiquement acceptable de celui-ci et un véhicule, diluant ou excipient pharmaceutiquement acceptables.

14. Composé selon l'une quelconque des revendications 1 à 12 ou sel pharmaceutiquement acceptable de celui-ci, ou composition pharmaceutique selon la revendication 13, pour utilisation en thérapie.

15. Composé selon l'une quelconque des revendications 1 à 12 ou sel pharmaceutiquement acceptable de celui-ci, ou composition pharmaceutique selon la revendication 13, pour utilisation dans une méthode de traitement d'une cardiopathie, de préférence où la cardiopathie est choisie dans le groupe constitué par une angine de poitrine, une angine de poitrine instable, un infarctus du myocarde, une insuffisance cardiaque, une maladie coronarienne aiguë, une insuffisance cardiaque aiguë, une insuffisance cardiaque chronique et une lésion cardiaque iatrogène.
